# EUROPEAN PATENT APPLICATION

(11) **EP 3 006 439 A1**
(43) Date of publication of application: **13.04.2016**
(21) Application number: 14800740.4
(22) Date of filing: 23.05.2014
(51) Int. Cl.: C07D 403/04, C07D 401/04, A61K 31/506, A61P 1/04

(54) **BICYCLIC DERIVATIVE CONTAINING PYRIMIDINE RING, AND PREPARATION METHOD THEREFOR**

(30) Priority: 24.05.2013 KR 20130058843
(71) Applicant: Yuhan Corporation, Seoul 156-754 (KR)
(72) Inventor: SIM, Jae Young, Yongin-si Gyeonggi-do 446-749 (KR); CHA, Myung Hun, Hwaseong-si Gyeonggi-do 445-855 (KR); KIM, Tae Kyun, Hwaseong-si Gyeonggi-do 445-160 (KR); YOON, Young Ae, Seoul 150-959 (KR); KIM, Dong Hoon, Suwon-si Gyeonggi-do 441-470 (KR)
(74) Representative: D Young & Co LLP
(86) International application number: PCT/KR2014/004636
(87) International publication number: WO 2014/189331

(57) **Abstract**

The present invention provides: a bicyclic derivative comprising a pyrimidine ring, or a pharmaceutically acceptable salt thereof; a preparation method therefor, a pharmaceutical composition comprising the same; and a use therefor. According to the present invention, the bicyclic compound derivative comprising a pyrimidine ring, or a pharmaceutically acceptable salt thereof acts as a 5-HT₄ receptor agonist, and thus can be usefully applied to the prevention or treatment of dysfunction in gastrointestinal motility, for example, gastrointestinal diseases such as gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, diabetic gastric atony and the like.

## Description

### [Technical Field]

The present invention relates to a novel 5-HT₄ receptor agonist, more specifically, a novel bicyclic derivative which comprises pyrimidine ring or pharmaceutically acceptable salt thereof which acts as a 5-HT₄ receptor agonist, a method for preparing thereof, a pharmaceutical composition comprising the same, and a use thereof.

### [Background Art]

Serotonin (5-hydroxytryptamine, 5-HT), one of the neurotransmitters, is broadly distributed throughout human body including both the central nervous system and the peripheral nervous system. Approximately 95% of the human body's total serotonin is found in the gastrointestinal tract, while about 5% thereof is found in the brain. Serotonin receptors are located in intestinal nerves, enterochromaffin cells, intestinal smooth muscle, immune tissues, etc. Serotonin receptor subtypes include 5-HT₁, 5-HT₂, 5-HT₃, 5-HT₄, 5-HT₆, and 5-HT₇. Interactions between these various receptors and serotonin are linked to various physiological functions. Therefore, various researches have been performed for developing therapeutic agents that are capable of interacting with a specific serotonin subtype as a target. The researches include identification of 5-HT₄ receptors and active agents interacting therewith (Langlois and Fischmeister, J. Med. Chem. 2003, 46, 319-344).

It has been found by the previous literatures that 5-HT4 receptor agonists are useful for treating an abnormal gastrointestinal motility, i.e., dysfunction in gastrointestinal motility. The abnormal gastrointestinal motility may result in various disorders, for example irritable bowel syndrome (IBS), constipation, dyspepsia, delayed gastric emptying, gastroesophageal reflux disease (GERD), gastroparesis, post-operative ileus, intestinal pseudo-obstruction, drug-induced delayed transit, etc.

Representative 5-HT₄ receptor agonists disclosed in prior arts include tegaserod (an aminoguanidine derivative, US 5,510,353), prucalopride (a benzofuran carboxamide derivative, EP0445862), cisapride (a benzamide derivative, US 4,962,115), mosapride (EP 0243959), etc. These compounds are known as an agent stimulating gastrointestinal motility.

### [Disclosure]

### [Technical Problem]

The present inventors found that various bicyclic derivatives comprising pyrimidine ring are useful for preventing or treating a dysfunction in gastrointestinal motility by acting as a 5-HT₄ receptor agonist.

Therefore, the present invention provides the bicyclic derivative comprising pyrimidine ring or pharmaceutically acceptable salt thereof, a method for preparing thereof, a pharmaceutical composition comprising the same, and a use thereof.

### [Technical Solution]

According to one embodiment of the present inventon, there is provided a bicyclic derivative comprising pyrimidine ring or pharmaceutically acceptable salt thereof.

According to another embodiment of the present invention, there is provided a method for preparing the bicyclic derivative comprising pyrimidine ring or pharmaceutically acceptable salt thereof.

According to still another embodiment of the present invention, there is provided a pharmaceutical composition for preventing or treating a dysfunction in gastrointestinal motility, which comprise the bicyclic derivative comprising pyrimidine ring or pharmaceutically acceptable salt thereof as an active ingredient.

According to still another embodiment of the present invention, there is provided a use of the bicyclic derivative comprising pyrimidine ring or pharmaceutically acceptable salt thereof for use in the preparation of a medicament for preventing or treating a dysfunction in gastrointestinal motility.

As used herein, the term, "alkyl" refers to a straight or branched hydrocarbon radical. For example, C₁-C₆ alkyl is an aliphatic hydrocarbon having 1 to 6 carbon atoms, such as methyl, ethyl, propyl, *n*-butyl, *n*-pentyl, *n*-hexyl, isopropyl, isobutyl, *sec*-butyl, *tert*-butyl, neopentyl, isopentyl, etc.

In addition, the term "alkoxy or alkyloxy" refers to a radical formed by substituting the hydrogen atom of a hydroxy group with an alkyl group. For example, C₁-C₆ alkoxy includes methoxy, ethoxy, propoxy, *n*-butoxy, *n*-pentyloxy, isopropoxy, *sec*-butoxy, *tert*-butoxy, neopentyloxy, isopentyloxy, etc.

### Novel compounds

### Compounds of formula 1

The present invention provides a compound of formula 1 as below, i.e. a bicyclic derivative comprising pyrimidine ring, or a pharmaceutically acceptable salt thereof: 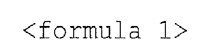 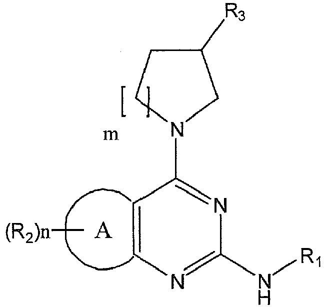 wherein,
R₁ is phenyl group; or pyridine group (wherein the phenyl group or pyridine group can be unsubstituted or substituted with one or more of substituents selected from the group consisting of halogen, amino, nitro, cyano, C₁₋₅ alkyl, C₁₋₅ alkyl substituted with halogen, C₁₋₅ alkoxy, C₁₋₅ alkoxy substitued with halogen, and hydroxy),
R₂ is each independently hydrogen; halogen; amino; mono- or di- C₁₋₅ alkyl amino; nitro; cyano; C₁₋₅ alkyl; C₁₋₅ alkyl substituted with halogen; C₁₋₅ alkoxy; C₁₋₅ alkoxy substituted with halogen; C₁₋₅ alkoxy carbonyl; hydroxy; or hydroxycarbonyl,
R₃ is a substituent selected from the group consisting of the below formulae I to III, 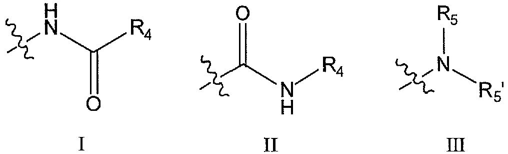
R₄ is C₁₋₅ alkyl; C₁₋₅ alkyl substituted with phenyl, thiophene (wherein the phenyl group or thiophene group can be unsubstituted or substituted with one or more of substituents selected from the group consisting of halogen, amino, C₁₋₅ alkyl, C₁₋₅ alkoxy, and hydroxy), or di C₁₋₅ alkyl amino group; or C₁₋₅ alkoxy,
R₅ and R₅' are each independently hydrogen; C₁₋₈ alkyl; C₁₋₈ alkyl substituted with phenyl or C₃₋₈ cycloalkyl (wherein the phenyl group can be unsubstituted or substituted with one or more of substituents selected from the group consisting of halogen, amino, C₁₋₅ alkyl, C₁₋₅ alkoxy and hydroxy); or C₃₋₈ cycloalkyl,
ring A is C₅₋₆ cycloalkyl; phenyl; or 5- to 6-membered heteroaryl comprising nitrogen atom,
m is 1 or 2,
n is integer of 0 to 2.

In addition, according to the preferable embodiment of the present invention, in said formulae,
R₁ is phenyl group; or pyridine group (wherein the phenyl group or pyridine group can be unsubstituted or substituted with one or more of substituents selected from the group consisting of halogen, amino, nitro, cyano, C₁₋₅ alkyl, C₁₋₅ alkyl substituted with halogen, and C₁₋₅ alkoxy),
R₂ is each independently hydrogen; halogen; C₁₋₅ alkyl; C₁₋₅ alkyl substituted with halogen; or C₁₋₅ alkoxy,
R₃ is a substituent selected from the group consisting of the below formulae I to III, 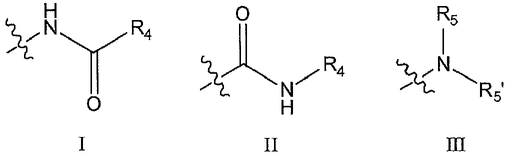 R₄ is C₁₋₅ alkyl; C₁₋₅ alkyl substituted with phenyl, thiophene, or di C₁₋₅ alkyl amino; or C₁₋₅ alkoxy,
R₅ and R₅' are each independently hydrogen; C₁₋₈ alkyl; C₁₋₈ alkyl substituted with phenyl or C₃₋₈ cycloalkyl; or C₃₋₈ cycloalkyl,
ring A is C₅₋₆ cycloalkyl; phenyl; or 5- to 6-membered heteroaryl comprising nitrogen atom,
m is 1 or 2,
n is integer of 0 to 2.

In addition, in said formulae, it is preferable that C₁₋₅ alkyl substituted with halogen of R₁ or R₂ is trifluoromethyl; it is preferable that C₁₋₅ alkoxy of R₂ is methoxy, and it is preferable that C₁₋₅ alkyl of R₄ is methyl.

The compound of formula 1 or pharmaceutically acceptable salt thereof may have substituents (for example, substituents of R₃) comprising chiral carbon, and in this case the compound of the formula 1 or salt thereof can be present as optical isomers such as (R), (S), racemate (RS), and the like. Therefore, unless otherwise indicated, the compound of formula 1 or pharmaceutically acceptable salt thereof include all of optical isomers such as (R), (S), racemate (RS), and the like. In addition, the compound of formula 1 or salt thereof can be present geometrical isomers with a double bond cis- or trans-form according to substituents. Therefore, unless otherwise indicated, the compound of formula 1 or salt thereof includes geometrical isomers of cis- and trans-forms.

In addition, the compound of formula 1 or salt thereof can be present as a diastereomer, and unless otherwise indicated, they include all of diastereomers or the mixture thereof.

The compound of formula 1 of the present invention may be a form of the pharmaceutically acceptable salt. The salt may be conventional acid additional salts, for example, salts derived from an inorganic acid such as hydrochloric acid, hydrobromic acid, sulfuric acid, sulfonic acid, sulphamic acid, phosphoric acid or nitric acid and salts derived from organic acid such as acetic acid, propionic acid, succinic acid, glycolic acid, stearic acid, citric acid, maleic acid, malonic acid, methane sulfonic acid, tartaric acid, hydroxymaleic acid, phenylacetic acid, glutamic acid, benzoic acid, salicyclic acid, 2-acetoxybenzoic acid, fumaric acid, toluenesulfonic acid, oxalic acid or trifluoroacetic acid. The said salts can be prepared by reacting the compound of the formula 1 in the form of free base with a stoichiometric amount or excess amount of the desired salt-forming inorganic acid or organic acid in suitable solvents or various mixtures of solvents.

### Compounds of formula 7

The present invention provides a compound of formula 7 as below or a pharmaceutically acceptable salt thereof which can be used as an intermediate for preparing the compound of formula 1. 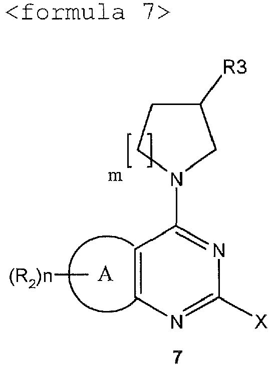 wherein, R₂, R₃, A ring, m and n are as defined in the above, and X is halogen.

The compound of formula 7 can be reacted with R₁-NH₂ to prepare the compound of formula 1.

### Methods for preparing the novel compounds

The present invention provides a method for preparing the compound of formula 1 or pharmaceutically acceptable salt thereof, which is bicyclic derivative comprising pyrimidine ring.

### Methods for preparing the compounds of formula 1

The method for preparing the compound of formula 1 or pharmaceutically acceptable salt thereof of the present invention may comprise,
performing a halogenation a compound of formula 4 as below to prepare a compound of formula 5 as below;
reacting the compound of formula 5 with a compound of formula 6 as below to prepare a compound of formula 7 as below; and
reacting the compound of formula 7 with R₁-NH₂ to prepare the compound of formula 1: 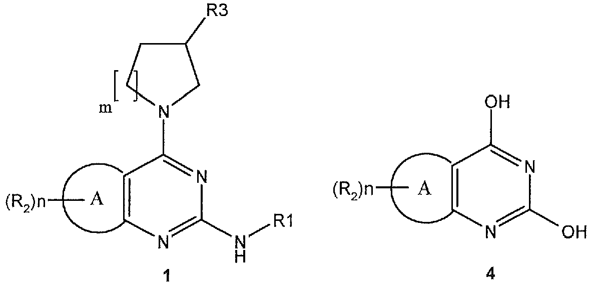 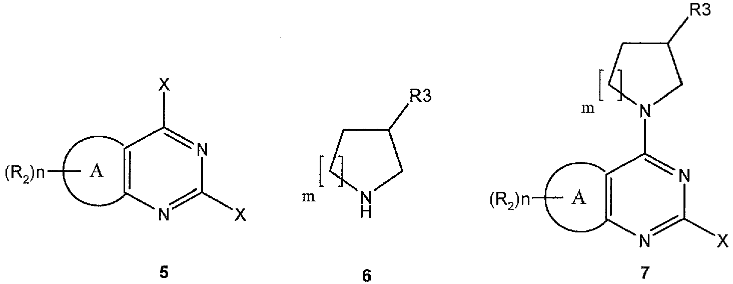 wherein, R₁, R₂, R₃, ring A, m and n are as defined in the above, and X is halogen.

The halogenation of the compound of formula 4 may be performed by using a halogenating agent such as phosphorous oxychloride, etc. The halogenation may be preferably performed by stirring at a temperature of between 100 °C and 120 °C overnight. And also, for improving reaction rate and/or yield, the halogenation may be performed in the presence of *N*,*N*-dimethylaniline, *N*,*N*-dimethylformamide, or diisopropylethylamine, etc. in a catalytic amount.

The reaction between the compound of formula 5 and the compound of formula 6 may be performed under an organic solvent such as tetrahydrofuran, alcohol, chloroform or *N,N-*dimethylformamide, and the like. The reaction may be performed in a condition of room temperature or elevated temperature (20 °C, to 60 °C). And also, for improving reaction rate and/or yield, the reaction may be performed in the presence of a base such as triethylamine or diisopropylethylamine, etc.

The reaction between the compound of formula 7 and R₁-NH₂ may be performed under an organic solvent such as alcohol, toluene, 1,4-dioxane, *N,N*-dimethylformamide, etc. or without the solvent. The reaction may be preferably performed by stirring overnight under a condition of elevated temperature (120 °C to 140 °C). And also, for improving reaction rate and/or yield, the reaction may be performed in the presence of a metallic catalyst such as palladium, etc., a ligand and a base such as cesium carbonate, etc., or performed under a microwave (300 W to 600 W).

In addition, the compound of formula 4 may be prepared by reacting a compound of formula 2 as below and a compound of formula 3 as below. 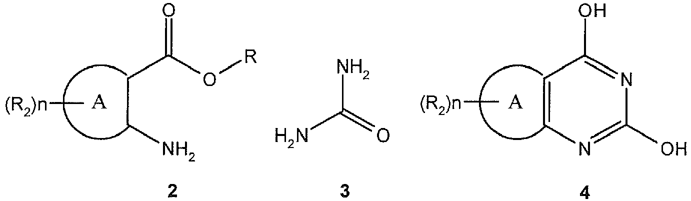 wherein, R₂, ring A and n are as defined in the above, and R is hydrogen or C₁₋₅alkyl.

The cyclization between the compound of formula 2 and the compound of formula 3 may be preferably performed at a temperature of 150 °C to 220 C.

In addition, the compound of formula 4 may be prepared by reacting a compound of formula 8 as below with a compound of formula 9 as below to prepare a compound of formula 10 as below, and then reacting the compound of formula 10 with an acid. 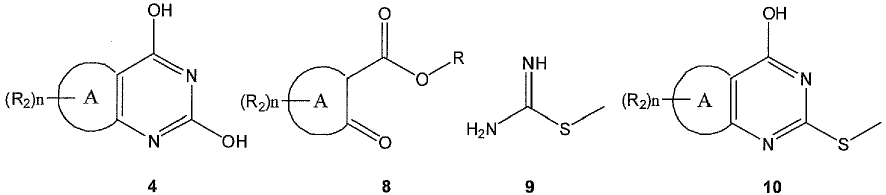 wherein, R₂, ring A and n are as defined in the above, and R is hydrogen or C₁₋₅alkyl.

The reaction between the compound of formula 8 and the compound of formula 9 may be performed in the presence of a base and a solvent. The base may be potassium carbonate, sodium carbonate, etc., and the solvent can be an aqueous solvent such as water, etc. And also, the reaction may be performed at room temperature.

The reaction between the compound of formula 10 and the acid may be performed by using an organic or an inorganic acid, such as acetic acid, hydrochloric acid, etc. The reaction may be preferably performed in an aqueous solvent such as water in a condition of elevated temperature (110 °C to 120 °C).

### Methods for preparing compounds of formula 1b

According to one embodiment of the present invention, the present invention provides a method for preparing a compound of formula 1b as below or pharmaceutically acceptable salt thereof, which comprises reacting a compound of formula 1a with an organic acid or an acyl halide. 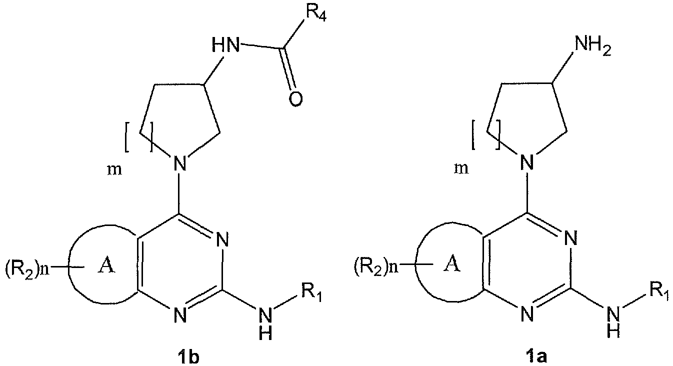 wherein, R₁, R₂, R₄, ring A, m and n are as defined in the above.

The reaction between the compound of formula 1a and the organic acid may be performed through an amide condensation by using a binding agent such as (benzotriazole-1-yloxy)-tris-(dimethylamino)phosphonium hexafluorophosphate, *N*-(3-dimethylaminopropyl)-*N'*-ethylcarbodiimide hydrochloride or 1-hydroxybenzotriazole hydrate, etc. and a base such as diisopropylethylamine or triethylamine, etc. The condensation may be performed in an organic solvent such as dichloromethane, *N*,*N*-dimethylformamide, *N,N*-dimethylacetamide, etc. And also, the condensation may be preferably performed at room temperature.

Meanwhile, the reaction between the compound of formula 1a and the acyl halide may be performed through an amide condensation using an organic base such as diisopropylethylamine, triethylamine, etc., or an inorganic base such as sodium hydroxide, etc. The condensation can be performed by using an organic solvent such as dichloromethane, etc. or a mixed solvent of the organic solvent and water. And also, the condensation may be preferably performed at room temperature.

In addition, the compound of formula 1b may be prepared by reacting a compound of formula 7a as below with an organic acid or an acyl halide to prepare a compound of formula 7b, and then reacting the compound of formula 7b with R₁-NH₂. 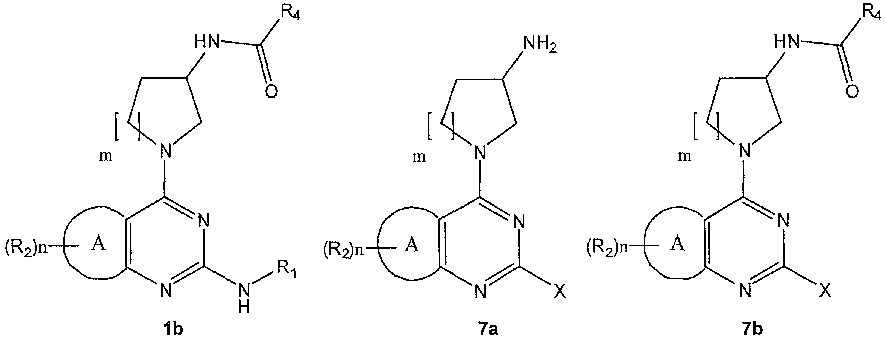 wherein, R₁, R₂, R₄, ring A, X, m and n are as defined in the above.

The reaction between the compound of formula 7a and the organic acid may be performed through an amide condensation by using a binding agent such as (benzotriazole-1-yloxy)-tris-(dimethylamino)phosphonium hexafluorophosphate, *N*-(3-dimethylaminopropyl)-*N*'-ethylcarbodiimide hydrochloride or 1-hydroxybenzotriazole hydrate, etc. and a base such as diisopropylethylamine, triethylamine, etc. The condensation may be performed in an organic solvent such as dichloromethane, *N,N*-dimethylformamide, *N,N*-dimethylacetamide, etc. In addition, the condensation may be preferably performed at room temperature.

Meanwhile, the reaction between the compound of formula 7a and the acyl halide may be performed through an amide condensation using an organic base such as diisopropylethylamine, triethylamine, etc. or an inorganic base such as sodium hydroxide, etc. The condensation may be performed by using an organic solvent such as dichloromethane, etc., or a mixed solvent of the organic solvent and water. And also, the condensation may be preferably performed at room temperature.

The reaction between the compound of formula 7b and R₁-NH₂ may be performed in an organic solvent such as alcohol, toluene, 1,4-dioxane, *N*,*N*-dimethylformamide, etc. or without the solvent. The reaction may be preferably performed by stirring overnight in a condition of elevated temperature (120 °C to 140 °C). And also, for improving reaction rate and/or yield, the reaction may be performed in the presence of a metallic catalyst such as palladium, etc., a ligand and a base such as cesium carbonate, etc, or performed under the microwave (300 W to 600 W).

### Methods for preparing compounds of formula 1c

According to another embodiment of the present invention, the present invention provides a method for preparing a compound of formula 1c or pharmaceutically acceptable salt thereof, which comprises performing a reductive amination of the compound of formula 1a with an aldehyde or a ketone compound. 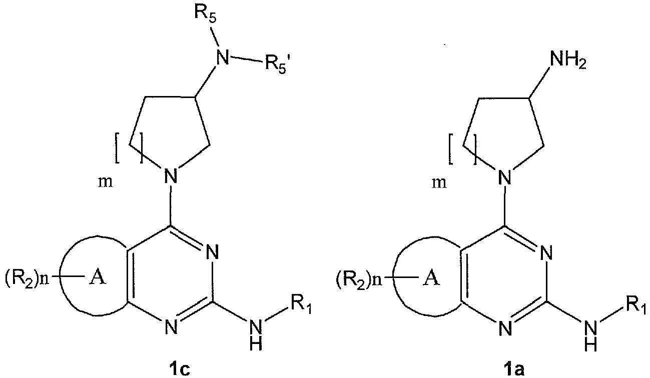 wherein, R₁, R₂, R₅, R₅', ring A, m and n are as defined in the above.

The reductive amination may be performed by using a reducing agent such as sodium borohydride, sodium triacetoxy borohydride, sodium cyanoborohydride, etc. The reductive amination may be performed in an organic solvent such as alcohol, etc, and may be performed at room temperature or at a low temperature of 0 °C or below. And also, for improving reaction rate and/or yield, acetic acid, etc. may be added.

### Methods for preparing compounds of formula 1d

According to one embodiment of the present invention, a compound of formula 1d may be prepared through introducing an amine-protecting group into a compound of formula 7a to prepare a compound of formula 7c; performing alkylation of the compound of formula 7c to prepare a compound of formula 7d; reacting the compound of formula 7d with R₁-NH₂, followed by removing the amine-protecting group. 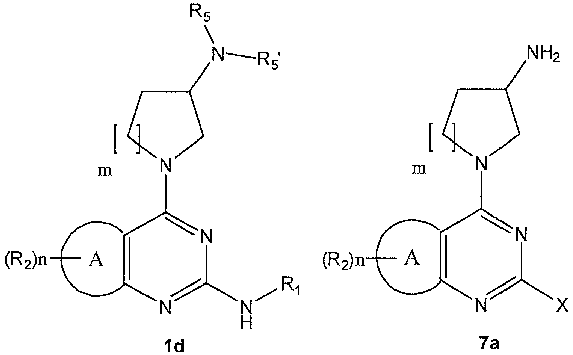 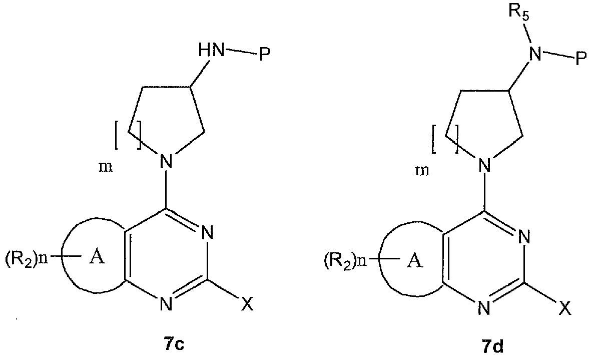 wherein, R₁, R₂, R₅, ring A, X, m and n are as defined in the above, R₅' is hydrogen, and P is an amine-protecting group.

The preferable amine-protecting agent is *tert*-butoxy carbonyl.

The reaction introducing the amine-protecting group into the compound of formula 7a may be performed in an organic solvent such as dichloromethane, chloroform, 1,4-dioxane, etc. and may be performed at room temperature or at 0 °C or below. And also, triethylamine, diisopropylethylamine, 4-dimethylaminopyridine, etc. may be added.

The alkylation of the compound of formula 7c may be performed by using an alkyl halide. The alkylation may be performed by using a base such as sodium hydride, potassium *t*-butoxide, etc. in an organic solvent such as *N,N-*dimethylformamide, etc. The alkylation may be performed at room temperature.

The reaction of the compound of formula 7d with R₁-NH₂ may be performed in an organic solvent such as alcohol, toluene, 1,4-dioxane, *N*,*N*-dimethylformamide, etc. or without the solvent. The reaction may be preferably performed by stirring overnight at the warming temperature condition (120 °C to 140 °C). And also, for improving reaction rate and/or yield, the reaction may be made in the presence of a metallic catalyst such as palladium, etc., a ligand and a base such as cesium carbonate, etc., or performed in the microwave (300 W to 600 W).

In addition, the reaction for removing the amine-protecting group may be performed by using an inorganic acid or an organic acid such as hydrochloric acid, trifluoroacetic acid, etc. in an organic solvent such as ethyl acetate, methanol, etc., and may be preferably performed at room temperature or at 0 °C or below.

In addition, the compound of formula 7d may be prepared through performing an reductive amination a compound of formula 7a as below to prepare a compound of formula 7e; and introducing an amine-protecting group into the compound of formula 7e. 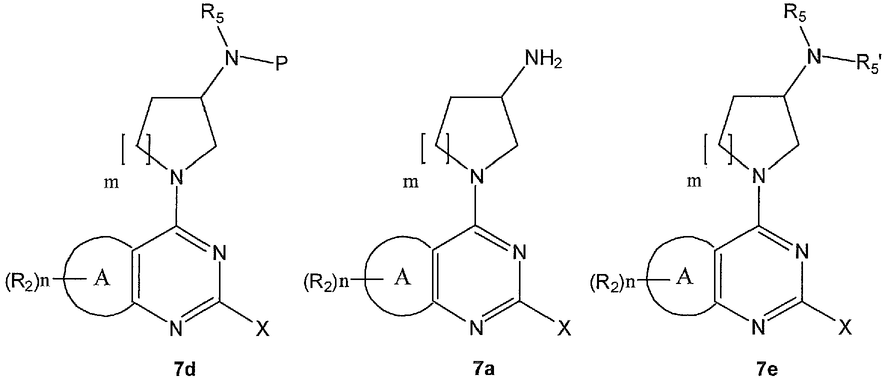 wherein, R₂, R₃, ring A, X, m and n are as defined in the above, R₅' is hydrogen, and P is an amine protecting group.

The preferable amine-protecting group is *tert-*butoxycarbonyl.

The reductive amination of the compound of formula 7a may be performed by using a reductive agent such as sodium borohydride, sodium triacetoxy borohydride, sodium cyanoborohydride, etc. The reductive amination may be performed in an organic solvent such as alcohol, etc., and may be performed at room temperature, or at 0 °C or below. And also, for improving reaction rate and yield, acetic acid and the like may be added.

The reaction introducing the amine-protecting group into the compound of formula 7e may be performed in an organic solvent such as dichloromethane, chloroform, 1,4-dioxane, etc. and may be performed at room temperature, or at 0 °C or below. In addition, triethylamine, diisopropylethylamine, 4-dimethylaminopyridine, and the like may be added.

### Methods for preparing compounds of formula 1e

According to one embodiment of the present invention, the present invention provides a method for preparing a compound of formula 1e or pharmaceutically acceptable salt thereof, which comprises reacting a compound of formula 7f as below with an organic amine to prepare a compound of formula 7g as below; and reacting the compound of formula 7g with R₁-NH₂: 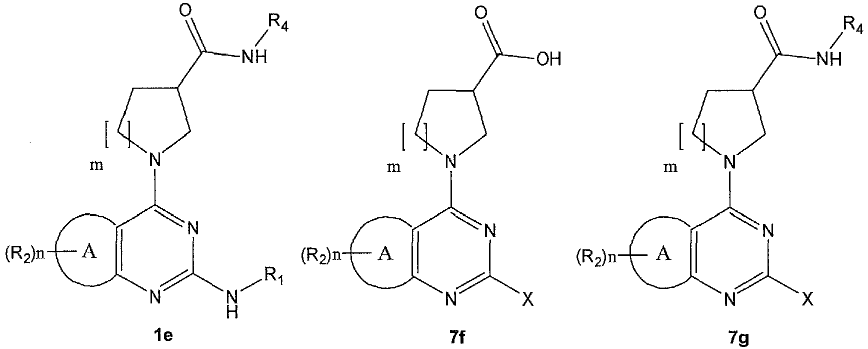 wherein, R₁, R₂, R₄, ring A, X, m and n are as defined in the above.

The reaction of the compound of formula 7f with the organic amine may be performed through amide condensation using a binding agent such as (benzotriazole-1-yloxy)-tris-(dimethylamino)phosphonium hexafluorophosphate, *N*-(3-dimethylaminopropyl)-*N*'-ethylcarbodiimide hydrochloride, 1-hydroxybenzotriazole hydrate, etc and a base such as diisopropylethylamine, triethylamine, etc. The condensation may be performed in an organic solvent such as dichloromethane, *N,N*-dimethylformamide, *N,N*-dimethylacetamide, etc. And also, the condensation may be preferably performed at room temperature.

The reaction of the compound of formula 7g with R₁-NH₂ may be performed in an organic solvent such as alcohol, toluene, 1,4-dioxane, *N*,*N*-dimethylformamide, etc. or without the solvent. The reaction may be preferably performed by stirring overnight in a condition of elevated temperature (120 °C to 140 °C). And also, for improving reaction rate and/or yield, the reaction may be performed in the presence of a metallic catalyst such as palladium, etc., a ligand and a base such as cesium carbonate, etc., or performed under the microwave (300 W to 600 W).

### Methods for preparing compounds of formula 7

According to one embodiment of the present invention, a compound of formula 7 may be prepared by reacting a compound of formula 5 as below with a compound of formula 6 to prepare a compound of formula 7: 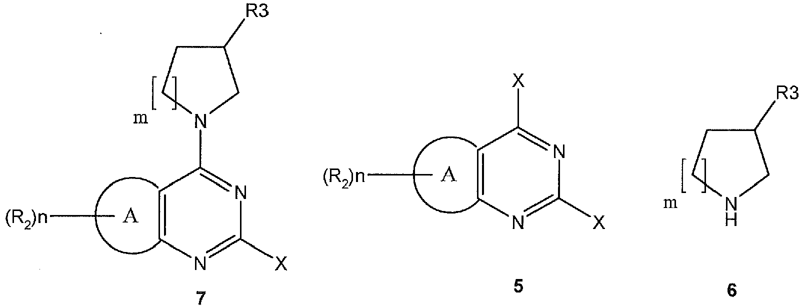 wherein, R₂, R₃, ring A, m and n are as defined in the above, and X is halogen.

The reaction between the compound of formula 5 and the compound of formula 6 may be performed in an organic solvent such as tetrahydrofuran, alcohol, chloroform or *N,N-*dimethylformamide, etc. In addition, the reaction may be performed at room temperature or elevated temperature (20 °C to 60 °C). And also, for improving the reaction rate and/or yield, the reaction may be performed in the presence of a base such as triethylamine or diisopropylethylamine, etc.

### Pharmaceutical compositions comprising the novel compounds

The present invention provides a pharmaceutical composition for preventing or treating a dysfunction in gastrointestinal motility, which comprise a therapeutically effective amount of the compound of formula 1 or pharmaceutically acceptable salt thereof and a pharmaceutically acceptable carrier.

The dysfunction in gastrointestinal motility includes, for example, gastrointestinal diseases such as gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, diabetic gastric atony and the like. The constipation includes chronic constipation, chronic idiopathic constipation (CIO), opioid-induced constipation (OIC), etc. And also, the dyspepsia includes non-ulcerative dyspepsia and functional dyspepsia.

The pharmaceutical composition may comprises pharmaceutically acceptable carriers such as diluents, disintegrants, sweeteners, lubricants, flavoring agents, etc. as commonly used. The pharmaceutical composition and may be prepared as an oral dosage form such as tablets, capsules, powders, granules and suspensions, emulsions or syrups; or a parenteral dosage form such as injection, according to the conventional methods. The dosage form may be prepared into the various forms, for example, dosage forms for single administration or dosage forms for multiple administrations.

The pharmaceutical composition of the present invention may comprise a diluent such as lactose, corn starch, etc, a lubricant such as magnesium stearate, etc., an emulsifying agent, a suspending agent, a stabilizer, an isotonic agent, etc. If necessary, the composition further comprises a sweetener and/or a flavoring agent.

The pharmaceutical composition of the present invention may be administered orally or parenterally including intravenous, intraperitoneal, subcutaneous, rectal and topical routes of administration. Therefore, the composition of the present invention may be prepared into various dosage forms such as tablets, capsules, aqueous solutions or suspensions, etc. In the case of tablets for oral administration, a carrier such as lactose, corn starch, etc., and a lubricant such as magnesium stearate are commonly used. In the case of the capsules for oral administration, lactose and/or dried corn starch can be as a diluent. When an aqueous suspension is required for oral administration, an active ingredient may be combined with an emulsifying agent and/or a suspending agent. If necessary, certain sweetening agent and/or flavoring agent may be added. For intramuscular, intraperitoneal, subcutaneous and intravenous administrations, sterile solutions of the active ingredient are usually prepared, and the pH of the solutions should be suitably adjusted and buffered. For intravenous administration, the total concentration of solutes should be controlled in order to render the preparation isotonic. The composition of the present invention may be in the form of an aqueous solution comprising a pharmaceutically acceptable carrier such as brine of pH 7.4. The solutions may be introduced into a patient's intramuscular blood-stream by local bolus injection.

The compound of formula 1 or pharmaceutically acceptable salt thereof may be administered in a therapeutically effective amount ranging from about 0.001 mg/kg to about 10 mg/kg per day to a subject patient. Of course, the dosage may be changed according to age, weight, susceptibility, and symptom of the patient or activity of the compound.

In addition, the present invention provides a use of the compound of formula 1 or pharmaceutically acceptable salt thereof for preparing a medicament for the prevention or treatment of dysfunction in gastrointestinal motility, for example, gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, or diabetic gastric atony.

### Method for preventing or treating gastrointestinal dysmotility

In addition, the present invention provides a method for preventing or treating dysfunction in gastrointestinal motility, for example, gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, or diabetic gastric atony, which comprises administering the composition comprising the compound of formula 1 or pharmaceutically acceptable salt thereof as an active ingredient to a subject in need of it.

The composition used in the prevention or treatment of the present invention includes the pharmaceutical composition disclosed in the present specification.

In addition, the subject needed the prevention or treatment method includes a mammal, in particular a human.

### [Advantageous Effects]

The compound according to the present invention, i.e., the bicyclic derivative comprising pyrimidine ring or pharmaceutically acceptable salt thereof act as a 5-HT₄ receptor agonist, and thus can be usefully applied for the prevention or treatment of gastrointestinal diseases such as dysfunction in gastrointestinal motility, for example, gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, diabetic gastric atony, and the like.

### [Best Mode for Invention]

Hereinafter, the present invention will be explained more specifically via reference examples, examples and experimental examples. However, such reference examples, examples and experimental examples merely exemplify the present invention, and are not intended to limit the present invention to them.

Nuclear Magnetic Resonance (NMR) spectrum analysis of the compounds prepared in the reference examples and examples was performed on Bruker 400 MHz spectrometer, a chemical shift was analyzed in ppm, a column chromatography was performed on silica gel (Merck, 70-230 mesh) (W.C. Still, J. Org. Chem., 43, 2923, 1978). And also, the starting materials in each example were synthesized from the known compounds according to the references, or were obtained from Sigma Aldrich.

### Reference Example

### Reference Example 1: (S)-tert-butyl{1-(2-chloro-6,7-dimethoxyquinazolin-4-yl)pyrrolidin-3-yl}carbamate

(3*S*)-(-)-3-(tert-butoxycarbonylamino)pyrrolidine (1.08 g, 5.79 mmol) was added into a mixed solution of 2,4-dichloro-6,7-dimethoxyquinazoline (1 g, 3.86 mmol) in ethanol (15 mL), and then the reaction mixture was stirred at room temperature overnight. The solvent was concentrated under reduced pressure, and the resulting residue was purified with silica gel column chromatography *(n-*hexane/ethyl acetate = 1/1) to give the titled compound (970 mg) as a pale yellow solid.
¹H-NMR (400MHz, CD₃OD) 5 7.37(s, 1H), 7.14(s, 1H), 4.76(m, 1H), 4.38(m, 1H), 4.19(m, 1H), 4.07(m, 1H), 3.97(s, 3H), 3.96(s, 3H), 3.80(m, 1H), 2.29(m, 1H), 2.05(m, 1H), 1.45(s, 9H)

### Reference Example 2: (S)-1-(2-chloro-6,7-dimethoxyquinazolin-4-yl)-N-methylpyrrolidin-3-amine

(3*S*)-(-)-3-(methylamino)pyrrolidine (580 mg, 5.79 mmol) was added into a mixed solution of 2,4-dichloro-6,7-dimethoxyquinazoline (1 g, 3.86 mmol) in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (950 mg) as a pale yellow solid.
¹H-NMR (400MHz, CD₃OD) δ 7.48(s, 1H), 6.93(s, 1H), 4.11(m, 2H), 3.99(m, 1H), 3.93(s, 3H), 3.92(s, 3H), 3.75(m, 1H), 3.40(m, 1H), 2.46(s, 3H), 2.28(m, 1H), 1.99(m, 1H)

### Reference Example 3: (S)-1-(2-chloro-6,7-dimethoxyquinazolin-4-yl)-N-ethylpyrrolidin-3-amine

(3*S*)-(-)-3-(ethylamino)pyrrolidine (580 mg, 5.79 mmol) was added into a mixed solution of 2,4-dichloro-6,7-dimethoxyquinazoline (1 g, 3.86 mmol) in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (980 mg) as a pale yellow solid.
¹H-NMR (400MHz, CD₃OD) δ 7.48(s, 1H), 6.92(s, 1H), 4.13(m, 2H), 3.93(m, 1H), 3.93(s, 3H), 3.92(s, 3H), 3.70(m, 1H), 3.48(m, 1H), 2.76(m, 2H), 2.30(m, 1H), 1.95(m 1H), 1.17(t, 3H)

### Reference Example 4: (S)-N-{1-(2-chloro-6,7-dimethoxyquinazolin-4-yl)pyrrolidin-3-yl}acetamide

(3*S*)-(-)-3-acetamidopyrrolidine (742 mg, 5.79 mmol) was added into a mixed solution of 2,4-dichloro-6,7-dimethoxyquinazoline (1 g, 3.86 mmol) in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (940 mg) as a pale yellow solid.
¹H-NMR (400MHz, DMSO-d₆) δ 8.17(d, 1H), 7.50(s, 1H), 7.11(s, 1H), 4.35(m, 1H), 4.11(m, 1H), 4.07(m, 2H), 3.98(s, 3H), 3.90(s, 3H), 3.70(m, 1H), 2.18(m, 1H), 2.11(m, 1H), 1.81(s, 3H)

### Reference Example 5: (R)-N-{1-(2-chloro-6,7-dimethoxyquinazolin-4-yl)piperidin-3-yl}acetamide

### <Step 1> (R)-1-(2-chloro-6,7-dimethoxyquinazolin-4-yl)piperidin-3-amine

*(R)*-3-aminopiperidine dihydrochloride (1 g, 5.79 mmol) and *N*,*N*-dimethylisopropylamine (2.7 mL, 15.44 mmol) were added into a mixed solution of 2,4-dichloro-6,7-dimethoxyquinazoline (1 g, 3.86 mmol) in ethanol (15 mL), and then the reaction mixture was stirred at room temperature overnight. The solvent was concentrated under reduced pressure, and the resulting residue was purified with silica gel column chromatography (dichloromethane/methanol = 20/1) to give the titled compound (1.1 g) as a pale yellow solid.
¹H-NMR (400MHz, DMSO) δ 7.22(s, 1H), 7.12(s, 1H), 4.21(m, 1H), 4.00(m, 1H), 3.98(s, 6H), 3.62(m, 3H), 2.21(m, 1H), 1.98(m, 1H), 1.82(m, 2H)

### <Step 2> (R)-N-{1-(2-chloro-6,7-dimethoxyquinazolin-4-yl)piperidin-3-yl}acetamide

Acetyl chloride (121 µL, 1.70 mmol) and triethylamine (544 µL, 3.86 mmol) were added into a mixed solution of (*R*)-1-(2-chloro-6,7-dimethoxyquinazolin-4-yl)piperidin-3-amine (500 mg, 1.55 mmol) prepared in Step 1 and dichloromethane (10 ml), and then they were stirred at room temperature overnight. The solvent was concentrated under reduced pressure, and the resulting residue was purified with silica gel column chromatography (*n*-hexane/ethyl acetate = 1/1) to give the titled compound (450 mg) as a pale yellow solid.

### Reference Example 6: (S)-N-{1-(2-chloro-6-methoxyquinazolin-4-yl)pyrrolidin-3-yl}acetamide

### <Step 1> 6-methoxyquinazolin-2,4(1H, 3H)-dione

Urea (5.4 g, 89.7 mmol) was added to 2-amino-5-methoxybenzoic acid (5 g, 29.9 mmol), and then the reaction mixture was stirred at 200 °C for 1 hour, cooled to room temperature and stirred for 1 hour. Water (30 ml) was added into the reaction mixture, and the reaction mixture was stirred at room temperature for 1 hour. The resulting precipitate was filtered and dried *in vacuo* to give the titled compound (4.8 g) as a pale yellow solid.

### <Step 2> 2,4-dichloro-6-methoxyquinazoline

*N*,*N*-dimethylamine (6.3 mL, 50 mmol) was added into a mixed solution of 6-methoxyquinazolin-2,4(1*H*, 3*H*)-dione (4.8 g, 25.0 mmol) prepared in Step 1 in phosphorus oxychloride (50 mL), and then they were stirred at 110 °C overnight. After cooling to room temperature, the reaction mixture was added into ice water and then basified to pH 9 with sodium hydroxide. The aqueous layer was extracted with ethyl acetate, and the organic layer was dried on anhydrous sodium sulfate and concentrated under reduced pressure. The resulting residue was purified with silica gel column chromatography (*n*-hexane/ethyl acetate = 5/1) to give the titled compound (3.6 g) as a yellow solid.

### <Step 3> (S)-N-{1-(2-chloro-6-methoxyquinazolin-4-yl)pyrrolidin-3-yl}acetamide

(3S)-(-)-3-acetamidopyrrolidine (419 mg, 3.27 mmol) was added into a reaction solution of 2,4-dichloro-6-methoxyquinazoline (500 mg, 2.18 mmol) prepared in Step 2 in ethanol (10 mL), and then the reaction mixture was stirred at room temperature overnight. The solvent was concentrated under reduced pressure, and the resulting residue was purified with silica gel column chromatography (*n-*hexane/ethyl acetate = 1/1) to give the titled compound (360 mg) as a yellow solid.

### Reference Example 7: (S)-1-(2-chloro-6-methoxyquinazolin-4-yl)-N-methylpyrrolidin-3-amine

(3*S*)-(-)-3-(methylamino)pyrrolidine (327 mg, 3.27 mmol) was added into a mixed solution of 2,4-dichloro-6-methoxyquinazoline (500 mg, 2.18 mmol) prepared in Step 2 of Reference Example 6 in ethanol (10 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (400 mg) as a pale yellow solid.

### Reference Example 8: (S)-1-(2-chloro-6-methoxyquinazolin-4-yl)-N-ethylpyrrolidin-3-amine

(3*S*)-(-)-3-(ethylamino)pyrrolidine (373 mg, 3.27 mmol) was added into a mixed solution of 2,4-dichloro-6-methoxyquinazoline (500 mg, 2.18 mmol) prepared in Step 2 of Reference Example 6 in ethanol (10 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (420 mg) as a pale yellow solid.

### Reference Example 9: (R)-N-{1-(2-chloro-6-methoxyquinazolin-4-yl)piperidin-3-yl}acetamide

### <Step 1> (R)-1-(2-chloro-6-methoxyquinazolin-4-yl)piperidin-3-amine

*(R)*-3-aminopiperidine dihydrochloride (567 mg, 3.27 mmol) and *N*,*N*-dimethylisopropylamine (570 µL, 3.27 mmol) were added into a mixed solution of 2,4-dichloro-6-methoxyquinazoline (500 mg, 2.18 mmol) prepared in Step 2 of Reference Example 6 in ethanol (10 mL), and then the reaction mixture was stirred at room temperature overnight. The solvent was concentrated under reduced pressure, and the resulting residue was purified with silica gel column chromatography (dichloromethane/methanol = 20/1) to give the titled compound (420 mg) as a pale yellow solid.

### <Step 2> (R)-N-{1-(2-chloro-6-methoxyquinazolin-4-yl)piperidin-3-yl}acetamide

Acetyl chloride (101 µL, 1.43 mmol) and triethylamine (602 µL, 4.29 mmol) were added into a mixed solution of *(R)*-1-(2-chloro-6-methoxyquinazolin-4-yl)piperidin-3-amine (420 mg, 1.43 mmol) prepared in Step 1 in dichloromethane (10 mL), and then they were stirred at room temperature overnight. The solvent was concentrated under reduced pressure, and the resulting residue was purified with silica gel column chromatography (*n*-hexane/ethyl acetate = 1/1) to give the titled compound (400 mg) as a pale yellow solid.

### Reference Example 10: (S)-1-{2-chloro-7-(trifluoromethyl)quinazolin-4-yl}-N-methylpyrrolidin-3-amine

### <Step 1> 7-(trifluoromethyl)quinazolin-2,4(1H, 3H)-dione

Urea (4.4 g, 73.1 mmol) was added to 2-amino-4-(trifluoromethyl)benzoic acid (5 g, 24.4 mmol), and then the reaction mixture was stirred at 200 °C for 1 hour. After cooling to room temperature, the reaction mixture was stirred for 1 hour. Water (100 ml) was added thereto, and the reaction mixture was stirred at room temperature for 1 hour. The resulting precipitate was filtered and dried *in vacuo* to give the titled compound (5 g) as a pale green solid.
¹H-NMR (400MHz, DMSO-d₆) δ 11.58(s, 1H), 11.41(s, 1H), 8.08(d, 1H), 7.48(d, 1H), 7.45(s, 1H)

### <Step 2> 2,4-dichloro-7-(trifluoromethyl)quinazoline

7-(trifluoromethyl)quinazolin-2,4(1*H*, 3*H*)-dione (5 g, 21.7 mmol) prepared in Step 1 was added into phosphorus oxychloride (30 mL), and then they were stirred at 110 °C overnight. After cooling to room temperature, the reaction mixture was added into ice water and then basified to pH 9 with sodium hydroxide. The aqueous layer was extracted with ethyl acetate, and the organic layer was dried on anhydrous sodium sulfate and concentrated under reduced pressure. The resulting residue was purified with silica gel column chromatography (*n*-hexane/ethyl acetate = 5/1) to give the titled compound (4.5 g) as a yellow solid.
¹H-NMR (400MHz, CDCl₃) δ 8.42(d, 1H), 8. 32 (s, 1H), 7.92(d, 1H)

### <Step 3> (S)-1-(2-chloro-7-(trifluoromethyl)quinazolin-4-yl)-N-methylpyrrolidin-3-amine

*(S)*-(-)-3-(methylamino)pyrrolidine (563 mg, 5.62 mmol) was added into a mixed solution of 2,4-dichloro-7-(trifluoromethyl)quinazoline (1 g, 3.74 mmol) prepared in Step 2 in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (560 mg) as a yellow solid.
¹H-NMR (400MHz, CDCl₃) δ 8.51(d, 1H), 7.89(s, 1H), 7.69(d, 1H), 4.17(m, 3H), 3.90(m, 1H), 3.52(m, 1H), 2.51(s, 3H), 2.35(m, 1H), 2.09(m, 1H)

### Reference Example 11: (S)-1-{2-chloro-7-(trifluoromethyl)quinazolin-4-yl}-N-ethylpyrrolidin-3-amine

*(S)*-(-)-3-(ethylamino)pyrrolidine (642 mg, 5.62 mmol) was added into a mixed solution of 2,4-dichloro-7-(trifluoromethyl)quinazoline (1 g, 3.74 mmol) in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (680 mg) as a yellow solid.
¹H-NMR (400MHz, CDCl₃) δ 8.50(m, 1H), 7.88(m, 1H), 7.69(m, 1H), 4.16(m, 3H), 3.83(m, 1H), 3.59(m, 1H), 2.32(m, 2H), 2.32(m, 1H), 2.04(m, 1H), 1.19(t, 3H)

### Reference Example 12: (S)-N-[1-{2-chloro-7-(trifluoromethyl)quinazolin-4-yl}pyrrolidin-3-yl]acetamide

*(S)*-(-)-3-acetamidopyrrolidine (720 mg, 5.62 mmol) was added into a mixed solution of 2,4-dichloro-7-(trifluoromethyl)quinazoline (1 g, 3.74 mmol) in ethanol (15 mL), and then the reaction mixture was stirred at room temperature overnight. The solvent was concentrated under reduced pressure, and the resulting residue was purified with silica gel column chromatography (*n*-hexane/ethyl acetate = 1/1) to give the titled compound (600 mg) as a yellow solid.
¹H-NMR (400MHz, CDCl₃) δ 8.48(m, 1H), 7.88(m, 1H), 7.69(m, 1H), 4.51(m, 1H), 4.22∼4.09(m, 3H), 3.88(m, 1H), 2.30(m, 1H), 2.11(m, 1H), 1.95(s, 3H)

### Reference Example 13. (S)-tert-butyl 1-(2-chloroquinazolin-4-yl)pyrrolidin-3-ylcarbamate

### <Step 1> quinazolin-2,4-diol

A mixture of 2-aminobenzoic acid (3 g, 21.5 mmol) and urea (3.9 g, 64.5 mmol) was stirred at 200 °C for 2 hours. After cooling the reaction solution, water was added thereto and the reaction solution was stirred for 1 hour. The resulting yellow solid was filtered, washed with water and dried *in vacuo* to give the titled compound (2.5 g). This compound was used in the subsequent reaction without further purification.

### <Step 2> 2,4-dichloroquinazoline

A mixture of quinazolin-2,4-diol (2.3 g, 14.2 mmol) prepared in Step 1 and phosphorus oxychloride (10 ml) was stirred at reflux overnight. After cooling the reaction mixture to room temperature, the reaction mixture was added into ice water and then basified to pH 7-8 with sodium hydroxide. The resulting yellow precipitate was filtered, washed with water and dried *in vacuo* to give the titled compound (2.5 g).
¹H NMR(400MHz, CDCl₃) δ 8.28(d, 1H), 8.05-8.00(m, 2H), 7.80-7.70(m, 1H)

### <Step 3> (S)-tert-butyl 1-(2-chloroquinazolin-4-yl)pyrrolidin-3-ylcarbamate

3-((*S*)-*tert*-butoxycarbonylamino)pyrrolidine (3.37 g, 18.1 mmol) was added into ethanol/chloroform (40/40 ml) solution of 2,4-dichloroquinazoline (3 g, 15.1 mmol) prepared in Step 2 and diisopropylethylamine (3.15 ml, 18.1 mmol), and then they were stirred at room temperature for 1 hour. The reaction mixture was concentrated, diluted in chloroform, and then washed with water, dried with anhydrous sodium sulfate and concentrated. The resulting residue was purified with silica gel column chromatography (*n*-hexane/ethyl acetate = 1/1) to give the titled compound (3.51 g) as a white solid.
¹H NMR(400MHz, CDCl₃) δ 8.09(d, 1H), 7.77(d, 1H), 7.71(t, 1H), 7.40(t, 1H), 4.70(m, 1H), 4.39(m, 1H), 4.22(m, 1H), 4.10-4.02(m, 2H), 3.86(m, 1H), 2.30(m, 1H), 2.04(m, 1H), 1.45(s, 9H)

### Reference Example 14. (S)-tert-butyl 1-(2-chloroquinazolin-4-yl)pyrrolidin-3-yl(propyl)carbamate

Sodium hydride (15.5 mg, 0.39 mmol, 60 wt%) was added into *N*,*N*-dimethylformamide (1.5 ml) solution of *(S)-tert-*butyl 1-(2-chloroquinazolin-4-yl)pyrrolidin-3-ylcarbamate (90 mg, 0.26 mmol) prepared in Reference Example 13 at 0 °C, and they were stirred for 30 minutes. 1-Bromopropane (28 µl, 0.31 mmol) was added into the reaction solution, and then they were stirred at room temperature overnight. Water was added to terminate the reaction, and extracted with ethyl acetate. The extract was dried with anhydrous magnesium sulfate and concentrated under reduced pressure. The resulting residue was purified with silica gel column chromatography (*n*-hexane/ethyl acetate = 2/1) to give the titled compound (57.7 mg) as a colorless oil.
¹H NMR(400MHz, CDCl₃) δ 8.09(d, 1H), 7.56(d, 1H), 7.70(t, 1H), 7.39(t, 1H), 4.60(m, 1H), 4.12(m, 2H), 3.93(m, 2H), 3.21(m, 1H), 3.10(m, 1H), 2.22(m, 2H), 1.70(m, 2H), 1.48(s, 9H), 0.91(t, 3H)

### Reference Example 15. (S)-tert-butyl butyl{1-(2-chloroquinazolin-4-yl)pyrrolidin-3-yl}carbamate

The titled compound was prepared as a colorless oil in the same manner as Reference Example 14 by using *(S)-tert-*butyl 1-(2-chloroquinazolin-4-yl)pyrrolidin-3-ylcarbamate prepared in Reference Example 13 and 1-bromobutane.
¹H NMR(400MHz, CDCl₃) δ 8.09(d, 1H), 7.75(d, 1H), 7.69(t, 1H), 7.39(t, 1H), 4.59(m, 1H), 4.09(m, 2H), 3.91(m, 2H), 3.25(m, 1H), 3.11(m, 1H), 2.22(m, 2H), 1.57(m, 2H), 1.48(s, 9H), 1.30(m, 2H), 0.92(t, 3H); (Yield: 50 %)

### Reference Example 16. (S)-tert-butyl 1-(2-chloroquinazolin-4-yl)pyrrolidin-3-yl(pentyl)carbamate

The titled compound was prepared as a colorless oil in the same manner as Reference Example 14 by using *(S)-tert-*butyl 1-(2-chloroquinazolin-4-yl)pyrrolidin-3-ylcarbamate prepared in Reference Example 13 and 1-bromopentane.
¹H NMR(400MHz, CDCl₃) δ 8.08 (d, 1H), 7.74(d, 1H), 7.67(m, 1H), 7.39(t, 1H), 4.60(m, 1H), 4.09(t, 2H), 3.97-3.86(m, 2H), 3.23(m, 1H), 3.10(m, 1H), 2.22(m, 2H), 1.59(m, 1H), 1.48(m, 9H+1H), 1.32(m, 4H), 0.91(t, 3H); (Yield: 56 %)

### Reference Example 17. (S)-tert-butyl 1-(2-chloroquinazolin-4-yl)pyrrolidin-3-yl(hexyl)carbamate

The titled compound was prepared as a colorless oil in the same manner as Reference Example 14 by using *(S)-tert-*butyl 1-(2-chloroquinazolin-4-yl)pyrrolidin-3-ylcarbamate prepared in Reference Example 13 and 1-iodohexane.
¹H NMR(400MHz, CDCl₃) δ 8.09(d, 1H), 7.74(d, 1H), 7.67(t, 1H), 7.38(t, 1H), 4.60(m, 1H), 4.11(m, 2H), 3.90(m, 2H), 3.23(m, 1H), 3.11(m, 1H), 2.22(m, 2H), 1.59(m, 2H), 1.48(s, 9H), 1.30(m, 6H), 0.89(t, 3H); (Yield: 66 %)

### Reference Example 18. 2,4-dichloro-8-methoxyquinazoline

### <Step 1> 8-methoxyquinazoline-2,4-diol

A mixture of 2-amino-3-methoxybenzoic acid (5 g, 29.9 mmol) and urea (8.98 g, 149.5 mmol) was stirred at 220 °C for 4 hours. After cooling the reaction solution, water was added thereto and the reaction solution was stirred for 1 hour. The resulting yellow solid was filtered, washed with water and dried *in vacuo* to give the titled compound (5.5 g). This compound was used in the subsequent reaction without further purification.

### <Step 2> 2,4-dichloro-8-methoxyquinazoline

A mixture of 8-methoxyquinazoline-2,4-diol (5.5 g, 28.6 mmol) prepared in Step 1 and phosphorus oxychloride (25 ml) was stirred at reflux overnight. After cooling the reaction mixture to room temperature, the reaction mixture was added into ice water and then basified to pH 7-8 with sodium hydroxide. The aqueous layer was extracted with dichloromethane, and the organic layer was dried on anhydrous magnesium sulfate and concentrated under reduced pressure. The resulting residue was purified with silica gel column chromatography (*n*-hexane/ethyl acetate = 5/1) to give the titled compound (2.1 g) as a white solid.
¹H NMR(400MHz, CDCl₃) δ 7.83(d, 1H), 7.65(t, 1H), 7.34(d, 1H), 4.09(s, 3H)

### Reference Example 19. (S)-N-{1-(2-chloro-8-methoxyquinazolin-4-yl)-pyrrolidin-3-yl}acetamide

Diisopropylethylamine (0.23 ml, 1.31 mmol) was added into ethanol/chloroform (10/10 ml) solution of 2,4-dichloro-8-methoxyquinazoline (300 mg, 1.31 mmol) prepared in Reference Example 18 and *(S)*-3-acetamidopyrrolidine (201 mg, 1.57 mmol), and then they were stirred at room temperature overnight. The reaction solution was concentrated under reduced pressure, diluted with dichloromethane, washed with water, dried with anhydrous magnesium sulfate, and concentrated under reduced pressure. The resulting residue was purified with silica gel column chromatography (dichloromethane/methanol = 20/1) to give the titled compound (357.5 mg) as a yellow oil.
¹H NMR(400MHz, CDCl₃) δ 7.65(d, 1H), 7.06(d, 1H), 6.65(s, 1H), 4.68(brs, 1H), 4.14-3.91(m, 3H+5H), 2.30(m, 1H), 2.17(m, 1H), 1. 98 (s, 3H)

### Reference Example 20. (S)-{1-(2-chloro-8-methoxyquinazolin-4-yl)-pyrrolidin-3-yl}ethylamine

The titled compound was prepared as a yellow oil in the same manner as Reference Example 19 by using 2,4-dichloro-8-methoxyquinazoline prepared in Reference Example 18 and (3*S*)-(-)-3-(ethylamino)pyrrolidine.
¹H NMR(400MHz, CDCl₃) δ 7.69(m, 1H), 7.29-7.23(m, 1H), 7.09(m, 1H), 4.13(m, 2H), 3.97(m, 3H+1H), 3.74(m, 1H), 3.50(m, 1H), 2.73(m, 2H), 2.20(m, 1H), 1.92(m, 1H), 1.13(m, 3H); (Yield: 98%)

### Reference Example 21. (R)-N-{1-(2-chloro-8-methoxyquinazolin-4-yl)piperidin-3-yl}acetamide

*(R)*-(-)-3-aminopiperidine dihydrochloride (249 mg, 1.44 mmol) was added into ethanol/chloroform (10/10 ml) solution of 2,4-dichloro-8-methoxyquinazoline (300 mg, 1.31 mmol) prepared in Reference Example 18 and diisopropylethylamine (0.68 ml, 4.23 mmol), and then they were stirred at room temperature overnight. The reaction solution was concentrated under reduced pressure, diluted with dichloromethane, washed with water, dried with anhydrous magnesium sulfate, and concentrated under reduced pressure. The resulting residue was dissolved in dichloromethane (10 ml). Triethylamine (0.33 ml, 2.39 mmol) and acetyl chloride (0.13 ml, 1.75 mmol) were added thereto at 0 °C, and they were stirred at room temperature overnight. The reaction solution was concentrated under reduced pressure, diluted with dichloromethane, washed with water, dried with anhydrous magnesium sulfate, and concentrated under reduced pressure. The resulting residue was purified with silica gel column chromatography (dichloromethane/methanol = 100/1) to give the titled compound (400 mg) as a white solid.
¹H NMR(400MHz, CDCl₃) δ 7.51(d, 1H), 7.41(m, 1H), 7.11(d, 1H), 6.73(s, 1H), 4.17(m, 1H), 4.03(s, 3H), 3.87-3.79(m, 4H), 2.02(s, 3H), 1.93(m, 2H), 1.74(m, 1H), 1.71(m, 1H)

### Reference Example 22. 2,4-dichloro-5-methylquinazoline

A mixture of 2-amino-6-methylbenzoic acid (5 g, 33.1 mmol) and urea (9.93 g, 165 mmol) was stirred at 150 °C for 6 hours. Water was added thereto at 100 °C and they were stirred at room temperature overnight. After cooling the reaction mixture to room temperature, the filtered solid was dissolved in 0.2 N sodium hydroxide aqueous solution (100 ml). The reaction mixture was stirred at reflux for 4 hours and stirred at room temperature for 1 day. Conc. hydrochloric acid aqueous solution was added thereto to neutralize to pH 7 and the resulting solid was filtered and dried *in vacuo* to give the titled compound (3 g) as a white solid.

A mixture of the prepared white solid (3 g, 17.0 mmol), *N*,*N*-dimethylaniline (4.3 ml, 34.1 mmol) and phosphorus oxychloride (12 ml) was stirred at reflux for 4 hours. After cooling the reaction mixture to room temperature, the same was added into ice water and the aqueous layer was extracted with dichloromethane. The organic layer was dried on anhydrous magnesium sulfate and concentrated under reduced pressure. The resulting residue was purified with silica gel column chromatography (*n*-hexane/ethyl acetate = 50/1) to give the titled compound (2 g) as a yellow solid.
¹H NMR(400MHz, CDCl₃) δ 7.88(d, 1H), 7.83(m, 1H), 7.50(d, 1H), 3.03(s, 3H)

### Reference Example 23. (S)-N-{1-(2-chloro-5-methylquinazolin-4-yl)-pyrrolidin-3-yl}acetamide

The titled compound was prepared as a white solid in the same manner as Reference Example 13 by using 2,4-dichloro-5-methylquinazoline prepared in Reference Example 22 and *(S)*-3-acetamidopyrrolidine.
¹H NMR(400MHz, CDCl₃) δ 7.53(m, 1H), 7.43(d, 1H), 7.26(m, 1H), 6.03(m, 1H), 4.51(m, 1H), 3.95(m, 2H), 3.78(m, 1H), 3.59(m, 1H), 2.63(s, 3H), 2.29(m, 1H), 1.92(m, 4H); (Yield: 59%)

### Reference Example 24. (S)-1-(2-chloro-5-methylquinazolin-4-yl)-N-ethylpyrrolidin-3-amine

The titled compound was prepared as a pale yellow oil in the same manner as Reference Example 13 by using 2,4-dichloro-5-methylquinazoline prepared in Reference Example 22 and (3*S*)-(-)-3-(ethylamino)pyrrolidine.
¹H NMR(400MHz, CDCl₃) δ 7.54 (m, 2H), 7.21(m, 1H), 3.84-3.74(m, 3H), 3.50(m, 1H), 3.36(m, 1H), 2.64(m, 2H+3H), 2.09(m, 1H), 1.78(m, 1H), 1.10(t, 3H); (Yield: 66%)

### Reference Example 25. (R)-N-{1-(2-chloro-5-methylquinazolin-4-yl)piperidin-3-yl}acetamide

The titled compound was prepared as a pale yellow oil in the same manner as Reference Example 21 by using 2,4-dichloro-5-methylquinazoline prepared in Reference Example 22.
¹H NMR (400MHz, CDCl₃) δ 7.64(m, 2H), 7.27(m, 1H), 4.11(m, 1H), 3.90(m, 1H), 3.57(m, 2H), 3.43(m, 1H), 2.70(s, 3H), 2.00(s, 3H), 1.78(m, 2H), 1.60(m, 2H); (Yield: 56%)

### Reference Example 26. 2,4-dichloro-8-methylquinazoline

### <Step 1> 8-methylquinazoline-2,4(1H, 3H)-dione

A mixture of 2-amino-3-methylbenzoic acid (5 g, 33.1 mmol) and urea (5.96 g, 99.2 mmol) was stirred at 190 °C for 4 hours. After cooling the reaction solution to room temperature, water (70 ml) was added thereto and the reaction solution was stirred for 1 hour. The resulting solid was filtered and dried *in vacuo* to give the titled compound (4.88 g) as a white solid.
¹H NMR(400MHz, DMSO-d₆) δ 11.29(brs, NH), 10.42(brs, NH), 7.77(d, 1H), 7.48(d, 1H), 7.10(t, 1H), 2.35(s, 3H)

### <Step 2> 2,4-dichloro-8-methylquinazoline

A mixture of 8-methylquinazoline-2,4(1*H*, 3*H*)-dione (4.88 g, 27.7 mmol) prepared in Step 1, *N*,*N*-dimethylaniline (2.8 ml, 22.2 mmol) and phosphorus oxychloride (28 ml) was stirred at reflux for 4 hours. After cooling the reaction mixture to room temperature, the reaction mixture was added into ice water. The resulting solid was filtered, washed with water, and dried *in vacuo* to give the titled compound (5.28 g) as a white solid.
¹H NMR(400MHz, CDCl₃) δ 8.12(d, 1H), 7.83(d, 1H), 7.62(t, 1H), 2.75(s, 3H)

### Reference Example 27. (S)-N-{1-(2-chloro-8-methylquinazolin-4-yl)pyrrolidin-3-yl}acetamide

The titled compound was prepared as a white solid in the same manner as Reference Example 13 by using 2,4-dichloro-8-methylquinazoline prepared in Reference Example 26 and *(S)*-3-acetamidopyrrolidine.
¹H NMR(400MHz, CD₃OD) δ 7.87(d, 1H), 7.50(d, 1H), 7.22(t, 1H), 7.10(m, 1H), 4.57(m, 1H), 4.03-3.80(m, 4H), 2.56(s, 3H), 2.20(m, 1H), 2.10(m, 1H), 2.03(s, 3H); (Yield: 73%)

### Reference Example 28. (S)-1-(2-chloro-8-methylquinazolin-4-yl)-N-ethylpyrrolidin-3-amine

The titled compound was prepared as a white solid in the same manner as Reference Example 13 by using 2,4-dichloro-8-methylquinazoline prepared in Reference Example 26 and (3*S*)-(-)-3-(ethylamino)pyrrolidine.
¹H NMR(400MHz, CDCl₃) δ 7.93(d, 1H), 7.51(d, 1H), 7.24(t, 1H), 4.11(m, 2H), 3.95(m, 1H), 3.75(m, 1H), 3.52(m, 1H), 2.73(m, 2H), 2.62(s, 3H), 2.28-2.18(m, 2H), 1.15(m, 3H); (Yield: 78%)

### Reference Example 29. (R)-N-{1-(2-chloro-8-methylquinazolin-4-yl)piperidin-3-yl}acetamide

The titled compound was prepared as a white solid in the same manner as Reference Example 21 by using 2,4-dichloro-8-methylquinazoline prepared in Reference Example 26.
¹H NMR(400MHz, CDCl₃) δ 7.79(d, 1H), 7.58(d, 1H), 7.36(t, 1H), 7.07(brs, NH), 4.15(m, 1H), 3.86-3.75(m, 4H), 2.65(s, 3H), 2.03(s, 3H), 1.92(m, 2H), 1.78-1.71(m, 2H); (Yield: 61%)

### Reference Example 30. 2,4,7-trichloroquinazoline

### <Step 1> 7-chloroquinazoline-2,4(1H, 3H)-dione

The titled compound was prepared as a white solid in the same manner as Step 1 of Reference Example 18 by using methyl 2-amino-4-chlorobenzoate.
¹H NMR(400MHz, DMSO-d₆) δ 11.34 (brs, 2NH), 7.87(d, 1H), 7.18(m, 2H); (Yield: 98%)

### <Step 2> 2,4,7-trichloroquinazoline

Diisopropylethylamine (9.21 ml, 52.9 mmol) was added to a mixture of 7-chloroquinazoline-2,4(1*H*, 3*H*)-dione (5.2 g, 26.5 mmol) prepared in Step 1 and phosphorus oxychloride (26 ml), and they were stirred at reflux for 4 hours. After cooling the reaction mixture to room temperature, the same was added into ice water, and basified to pH 7-8 by using sodium bicarbonate. The aqueous layer was extracted with dichloromethane, and the organic layer was dried on anhydrous magnesium sulfate and concentrated under reduced pressure. The resulting residue was purified with silica gel column chromatography (dichloromethane) to give the titled compound (3.88 g) as a white solid.
¹H NMR(400MHz, CDCl₃) δ 8.22(d, 1H), 8.01(s, 1H), 7.68(d, 1H)

### Reference Example 31. (S)-1-(2,7-dichloroquinazolin-4-yl)-N-methylpyrrolidin-3-amine

The titled compound was prepared as a white solid in the same manner as Reference Example 13 by using 2,4,7-trichloroquinazoline prepared in Reference Example 30 and (3*S*)-(-)-3-(methylamino)pyrrolidine.
¹H NMR(400MHz, CD₃OD) δ 8.32(d, 1H), 7.67(s, 1H), 7.51(d, 1H), 4.30-4.15(m, 4H), 4.00(m, 1H), 2.83(s, 3H), 2.58(m, 1H), 2.37(m, 1H); (Yield: 88%)

### Reference Example 32. (S)-N-{1-(2-chloro-7-fluoroquinazolin-4-yl)pyrrolidin-3-yl}acetamide

### <Step 1> 7-fluoroquinazolin-2,4(1H, 3H)-dione

A mixture of 4-fluoroanthranilic acid (5 g, 32.2 mmol) and urea (5.8 g, 96.7 mmol) was stirred at 220 °C for 1 hour. After cooling the reaction solution, water was added thereto. The reaction solution was stirred at reflux for 1 hour, and stirred again at room temperature for 3 days. The resulting solid was filtered, washed with water and dried *in vacuo* to give the titled compound (5.26 g) as a pale yellow solid.
¹H NMR(400MHz, DMSO-d₆) δ 11.29(brs, 2NH), 7.95(t, 1H), 7.03(t, 1H), 6.91(d, 1H)

### <Step 2> 2,4,-dichloro-7-fluoroquinazoline

A mixture of 7-fluoroquinazoline-2,4(1*H*, 3*H*)-dione (5.26 g, 29.2 mmol) prepared in Step 1 and phosphorus oxychloride (85 ml) was stirred at reflux for 3 days. After cooling the reaction mixture to room temperature, the same was added into ice water. The resulting solid was filtered and dried *in vacuo* to give the titled compound (3.82 g) as a yellow solid.
¹H NMR(400MHz, CDCl₃) δ 8.32(m, 1H), 7.63(d, 1H), 7.49(t, 1H)

### <Step 3> (S)-N-{1-(2chloro-7-fluoroquinazolin-4-yl)pyrrolidin-3-yl}acetamide

The titled compound was prepared as a white solid in the same manner as Reference Example 19 by using 2,4,-dichloro-7-fluoroquinazoline prepared in Step 2.
¹H NMR(400MHz, CD₃OD) δ 8.35(m, 1H), 7.27(m, 2H), 4.50(m, 1H), 4.19-4.04(m, 3H), 3.83(m, 1H), 2.31(m, 1H), 2.11(m, 1H), 1.95(s, 3H); (Yield: 33%)

### Reference Example 33. 2,4-dichloro-5,6,7,8-tetrahydroquinazoline

### <Step 1> 2-(methylthio)-5,6,7,8-tetrahydroquinazolin-4-ol

An aqueous solution (110 ml) of ethyl 2-cyclohexanone carboxylate (10 ml, 62.9 mmol), 2-methyl-2-thiopseudourea (9.6 g, 69.1 mmol) and sodium carbonate (10.7 g, 101 mmol) was stirred at room temperature for 4 days. The resulting solid was filtered, dried *in vacuo* and used in the subsequent reaction without further purification.

### <Step 2> 5,6,7,8-tetrahydroquinazoline-2,4-diol

A mixture of 2-(methylthio)-5,6,7,8-tetrahydroquinazolin-4-ol prepared in Step 1, acetic acid (65 ml) and water (30 ml) was stirred at reflux for 3 days. After cooling the reaction solution to room temperature, the resulting solid was filtered, washed with ethyl acetate and dried *in vacuo* to give the titled compound (4.85 g) as a white solid
¹H NMR(400MHz, DMSO-d₆) δ 10.84(s, 1H), 10.56(s, 1H), 2.30(m, 2H), 2.14(m, 2H), 1.61(m, 4H)

### <Step 3> 2,4-dichloro-5,6,7,8-tetrahydroquinazoline

A mixture of 5,6,7,8-tetrahydroquinazolin-2,4-diol (4.85 g, 23.9 mmol) prepared in Step 2 and phosphorus oxychloride (20 ml) was stirred at 130 °C for 3 hours. After cooling the reaction mixture to room temperature, the same was added into ice water and basified with sodium bicarbonate and sodium hydroxide. The aqueous layer was extracted with dichloromethane, and the organic layer was dried on anhydrous magnesium sulfate and concentrated under reduced pressure. The resulting residue was purified with silica gel column chromatography (*n*-hexane/ethyl acetate = 15/1) to give the titled compound (5.1 g) as a white solid.
¹H NMR(400MHz, CDCl₃) δ 2.89(m, 2H), 2.74(m, 2H), 1.88(m, 4H)

### Reference Example 34. (S)-N-{1-(2-chloro-5,6,7,8-tetrahydroquinazolin-4-yl)pyrrolidin-3-yl}acetamide

Diisopropylethylamine (0.86 ml, 4.92 mmol) was added into chloroform (18 ml) solution of 2,4-dichloro-5,6,7,8-tetrahydroquinazoline (1 g, 4.92 mmol) prepared in Reference Example 33 and *(S)*-3-acetamidopyrrolidine (0.69 g, 5.42 mmol), and then they were stirred at 50 °C overnight. Water was added to the reaction solution and extracted with dichloromethane. The extract was dried with anhydrous magnesium sulfate and concentrated under reduced pressure. The resulting residue was purified with silica gel column chromatography (dichloromethane/methanol = 60/1) to give the titled compound (1.14 g) as a white solid.
¹H NMR(400MHz, CD₃OD) δ 4.36(m, 1H), 3.92(m, 1H), 3.83(m, 1H), 3.77(m, 1H), 3.59(m, 1H), 2.79(m, 2H), 2.64(m, 2H), 2.15(m, 1H), 1.94(s, 4H), 1.79-1.69(m, 4H)

### Reference Example 35. (S)-tert-butyl 1-(2-chloro-5,6,7,8-tetrahydroquinazolin-4-yl)pyrrolidin-3-ylcarbamate

The titled compound was prepared as a white solid in the same manner as Reference Example 34 by using 2,4-dichloro-5,6,7,8-tetrahydroquinazoline prepared in Reference Example 33 and 3-(*(S)*-tert-butoxycarbonylamino)pyrrolidine.
¹H NMR(400MHz, CDCl₃) δ 4.63(s, 1H), 4.25(m, 1H), 3.91(m, 1H), 3.79-3.70(m, 2H), 3.54(m, 1H), 2.72(m, 4H), 2.17(m, 1H), 1.88(m, 1H), 1.78-1.72(m, 4H), 1.45(s, 9H); (Yield: 55%)

### Reference Example 36. (S)-tert-butyl 1-(2-chloro-5,6,7,8-tetrahydroquinazolin-4-yl)pyrrolidin-3-yl(methyl)carbamate

Diisopropylethylamine (2.7 ml, 15.5 mmol) was added into chloroform (40 ml) solution of 2,4-dichloro-5,6,7,8-tetrahydroquinazoline (1.5 g, 7.39 mmol) prepared in Reference Example 33 and (3*S*)-(-)-3-(methylamino)pyrrolidine (0.87 ml, 8.13 mmol), and then they were stirred at 50 °C overnight. Di-tert-butyldicarbonate (1.69 ml, 7.39 mmol) was added thereto, and they were stirred at room temperature overnight. The reaction mixture was diluted in dichloromethane, washed with water, dried with anhydrous magnesium sulfate, and then concentrated under reduced pressure. The resulting residue was purified with silica gel column chromatography (*n*-hexane/ethyl acetate = 5/1) to give the titled compound (2.3 g) as a colorless oil.
¹H NMR(400MHz, CDCl₃) δ 4.74(s, 1H), 3.80(m, 2H), 3.67(m, 1H), 3.54(m, 1H), 2.82(s, 3H), 2.71(m, 4H), 2.04(m, 2H), 1.85(m, 2H), 1.71(m, 1H), 1.59(m, 1H), 1.48(s, 9H)

### Reference Example 37. (S)-tert-butyl 1-(2-chloro-5,6,7,8-tetrahydroquinazolin-4-yl)pyrrolidin-3-yl(ethyl)carbamate

The titled compound was prepared as a colorless oil in the same manner as Reference Example 36 by using 2,4-dichloro-5,6,7,8-tetrahydroquinazoline prepared in Reference Example 33 and (3*S*)-(-)-3-(ethylamino)pyrrolidine.
¹H NMR(400MHz, CDCl₃) δ 4.56(s, 1H), 3.80(m, 2H), 3.68(m, 1H), 3.54(m, 1H), 3.28-3.15(m, 2H), 2.70(m, 4H), 2.04(m, 2H), 1.86(m, 2H), 1.69(m, 1H), 1.59(m, 1H), 1.48(s, 9H), 1.14(m, 3H); (Yield: 83%)

### Reference Example 38. (R)-N-{1-(2-chloro-5,6,7,8-tetrahydroquinazolin-4-yl)piperidin-3-yl}acetamide

*(R)*-(-)-3-aminopiperidine dihydrochloride (940 mg, 5.42 mmol) was added to chloroform (25 ml) solution of 2,4-dichloro-5,6,7,8-tetrahydroquinazoline (1 g, 4.92 mmol) prepared in Reference Example 33 and diisopropylethylamine (3.5 ml, 20.2 mmol), and then they were stirred at 60 °C overnight. Acetyl chloride (0.39 ml, 5.42 mmol) was added thereto at room temperature, and they were stirred for 2 days. The reaction solution was diluted with dichloromethane, washed with water, dried with anhydrous magnesium sulfate and concentrated under reduced pressure. The resulting residue was purified with silica gel column chromatography (*n*-hexane/ethyl acetate = 5/1) to give the titled compound (1.2 g) as a white solid.
¹H NMR(400MHz, CD₃OD) δ 7.41(m, 1H), 7.23(m, 1H), 7.14(m, 1H), 4.19-3.98(m, 5H), 3.15(m, 2H), 2.49(m, 1H), 2.46(m, 3H), 2.30(s, 3H), 2.25(m, 1H+3H), 1.36(m, 3H)

### Reference Example 39. (S)-1-(2-chloro-5,6,7,8-tetrahydroquinazolin-4-yl)-N-methylpiperidine-3-carboxamide

Diisopropylethylamine (3.4 ml, 19.7 mmol) was added into chloroform (25 ml) solution of 2,4-dichloro-5,6,7,8-tetrahydroquinazoline (1 g, 4.92 mmol) prepared in Reference Example 33 and *(R)*-(-)-3-piperidinecarboxylic acid (0.7 g, 5.42 mmol), and then they were stirred at 60 °C for 2 days. After cooling the reaction solution to room temperature, methylamine hydrochloride (0.33 g, 4.92 mmol), *N*-(3-dimethylaminopropyl)-*N'*-ethylcarbodiimide hydrochloride (0.94 g, 4.92 mmol) and 1-hydroxybenzotriazole hydrate (0.67 g, 4.92 mmol) were added thereto, they were stirred at room temperature overnight. The reaction solution was diluted with dichloromethane, washed with water, dried with anhydrous magnesium sulfate, and concentrated under reduced pressure. The resulting residue was crystallized by using ether/ethyl acetate to give the titled compound (436 mg) as a pale yellow solid.
¹H NMR(400MHz, CDCl₃) δ 6.22(m, 1H), 3.93(m, 1H), 3.74(m, 1H), 3.34(m, 1H), 3.10(m, 1H), 2.81(m, 4H), 2.49(m, 3H), 1.93-1.84(m, 4H), 1.70-1.60(m, 4H)

### Reference Example 40. (S)-N-{1-(2-chloroquinazolin-4-yl)pyrrolidin-3-yl}acetamide

Ethanol/chloroform (15/10 ml) solution of 2,4-dichloroquinazoline (500 mg, 2.5 mmol) prepared in Step 2 of Reference Example 13 and (3*S*)-(-)-3-acetamidopyrrolidine (480 mg, 3.7 mmol) was stirred at room temperature overnight, and concentrated under reduced pressure. The resulting residue was purified with silica gel column chromatography (ethyl acetate) to give the titled compound (500 mg) as a yellow oil.
¹H NMR(400MHz, CD₃OD) δ 8.30(d, 1H), 7.80-7.70(m, 1H), 7.63(d, 1H), 7.55-7.45(m, 1H), 4.50(t, 1H), 4.30-4.00(m, 3H), 3.90-3.80(m, 1H), 2.35-2.25(m, 1H), 2.15-2.05(m, 1H), 1.95(s, 3H)

### Reference Example 41. (S)-1-(2-chloroquinazolin-4-yl)-N-methylpyrrolidin-3-amine

Ethanol/chloroform (5/20 ml) solution of 2,4-dichloroquinazoline (500 mg, 2.5 mmol) prepared in Step 2 of Reference Example 13 and (3*S*)-(-)-3-(methylamino)pyrrolidine (380 mg, 3.8 mmol) was stirred at room temperature overnight, and concentrated under reduced pressure. The resulting residue was purified with silica gel column chromatography (dichloromethane/methanol - 20/1) to give the titled compound (300 mg) as a yellow solid.
¹H NMR(400MHz, CDCl₃) δ 8.12(d, 1H), 7.80-7.60(m, 2H), 7.37(t, 1H), 4.15-3.95(m, 3H), 3.85-3.75(m, 1H), 3.45-3.35(m, 1H), 2.51(s, 3H), 2.25-2.15(m, 1H), 2.05-1.95(m, 1H)

### Reference Example 42. (S)-1-(2-chloroquinazolin-4-yl)-N-ethylpyrrolidin-3-amine

Chloroform (20 ml) solution of 2,4-dichloroquinazoline (500 mg, 2.5 mmol) prepared in Step 2 of Reference Example 13 and (3*S*)-(-)-3-(ethylamino)pyrrolidine (420 mg, 3.8 mmol) was stirred at room temperature overnight, and concentrated under reduced pressure. The resulting residue was purified with silica gel column chromatography (dichloromethane/methanol - 20/1) to give the titled compound (500 mg) as a yellow solid.
¹H NMR(400MHz, CD₃OD) δ 8.31(d, 1H), 7.75(t, 1H), 7.62(d, 1H), 7.48(t, 1H), 4.20-3.95(m, 3H), 3.85-3.75(m, 1H), 3.55-3.45(m, 1H), 2.80-2.65(m, 2H), 2.35-2.25(m, 1H), 2.05-1.95(m, 1H), 1.17(t, 3H)

### Reference Example 43. (R)-N-{1-(2-chloroquinazolin-4-yl)piperidin-3-yl}acetamide

*(R)*-(-)-3-aminopiperidine dihydrochloride (480 mg, 2.75 mmol) was added into chloroform (20 ml) solution of 2,4-dichloroquinazoline (500 mg, 2.5 mmol) prepared in Step 2 of Reference Example 13 and diisopropylethylamine (1.3 ml, 7.5 mmol), and then the reaction solution was stirred at room temperature overnight and concentrated under reduced pressure. The resulting solution was diluted with dichloromethane, washed with water, dried with anhydrous magnesium sulfate, and concentrated under reduced pressure. The resulting residue was dissolved in dichloromethane (10 ml). Triethylamine (0.35ml, 2.5mmol) and acetyl chloride (0.18ml), 2.5 mmol) were added thereto at 0 °C, and the reaction solution was stirred at room temperature overnight and concentrated under reduced pressure. The resulting solution was diluted with dichloromethane, washed with water, dried with anhydrous magnesium sulfate, and concentrated under reduced pressure. The resulting residue was purified with silica gel column chromatography (ethyl acetate) to give the titled compound (110 mg) as a yellow solid.
¹H NMR(400MHz, CDCl₃) 5 7.97(d, 1H), 7.85-7.70(m, 2H), 7.49(t, 1H), 6.85(brs, 1H), 4.20-4.10(m, 1H), 4.00-3.85(m, 2H), 3.85-3.70(m, 2H), 2.03(s, 3H), 2.00-1.65(m, 4H)

### Reference Example 44. (S)-N-{1-(2-chloro-7-methoxyquinazolin-4-yl)pyrrolidin-3-yl}acetamide

### <Step 1> 7-methoxyquinazoline-2,4-diol

A mixture of 2-amino-4-methoxybenzoic acid (5 g, 29.9 mmol) and urea (5.4 g, 89.7 mmol) was stirred at 200 °C for 2 hours. After cooling the reaction solution, water was added thereto and the reaction solution was stirred for 1 hour. The resulting brown solid was filtered, washed with water and dried *in vacuo* to prepare the titled compound (3 g). This compound was used in the subsequent reaction without further purification.

### <Step 2> 2,4-dichloro-7-methoxyquinazoline

A mixture of 7-methoxyquinazolin-2,4-diol (3 g, 15.6 mmol) prepared in Step 1 and phosphorus oxychloride (10 ml) was stirred at reflux overnight. After cooling the reaction mixture to room temperature, the same was added into ice water, and basified to pH 7-8 by using sodium bicarbonate. The aqueous layer was extracted with dichloromethane, and the organic layer was dried on anhydrous magnesium sulfate and concentrated under reduced pressure. The resulting residue was purified with silica gel column chromatography (*n*-hexane/ethyl acetate = 10/1) to give the titled compound (0.64 g) as a white solid.
¹H NMR(400MHz, CDCl₃) δ 8.12(d, 1H), 7.37-7.20(m, 2H), 3.99(s, 3H)

### <Step 3> (5)-N-{1-(2-chloro-7-methoxyquinazolin-4-yl)pyrrolidin-3-yl}acetamide

Chloroform (5 ml) solution of 2,4-dichloro-7-methoxyquinazoline (300 mg, 1.3 mmol) prepared in Step 2 and (3*S*)-(-)-3-acetamidopyrrolidine (250 mg, 1.95 mmol) was stirred at room temperature overnight, and concentrated under reduced pressure. The resulting solution was diluted with ethyl acetate, washed with water, dried with anhydrous magnesium sulfate, and concentrated under reduced pressure to give the titled compound (350 mg) as a white solid.
¹H NMR(400MHz, CD₃OD) δ 8.19(d, 1H), 7.07(d, 1H), 7.01(d, 1H), 4.48(t, 1H), 4.25-3.95(m, 3H), 3.92(s, 3H), 3.90-3.80(m, 1H), 2.30-2.20(m, 1H), 2.15-2.05(m, 1H), 1.95(s, 3H)

### Reference Example 45. (S)-N-{1-(2-chloropyrido[3,2-d]pyrimidin-4-yl)pyrrolidin-3-yl}acetamide

Ethanol/chloroform (15/10 ml) solution of 2,4-dichloropyrido[3,2-d]pyrimidine (500 mg, 2.5 mmol) and (3*S*)-(-)-3-acetamidopyrrolidine (480 mg, 3.7 mmol) was stirred at room temperature overnight, and concentrated under reduced pressure. The resulting residue was purified with silica gel column chromatography (ethyl acetate) to give the titled compound (100 mg) as a yellow oil.
¹H NMR(400MHz, DMSO-d₆) δ 8.81(s, 1H), 8.18(d, 1H), 8.03(d, 1H), 7.85-7.75(m, 1H), 4.50-4.25(m, 3H), 3.90-3.65(m, 2H), 2.30-1.80(m, 5H)

### Reference Example 46. (S)-tert-butyl {1-(2-chloropyrido[3,2-d]pyrimidin-4-yl)pyrrolidin-3-yl}carbamate

Diisopropylethylamine (2.6 ml, 15 mmol) was slowly added into chloroform (100 ml) solution of 2,4-dichloropyrido[3,2-d]pyrimidine (2 g, 10 mmol) and 3-(*(S)-*tert-butoxycarbonylamino)pyrrolidine (2 g, 11 mmol), and the reaction solution was stirred at room temperature overnight. The resulting solution was washed with water, dried with anhydrous magnesium sulfate, and concentrated under reduced pressure. Ethyl acetate (10 ml) was added to the resulting pale yellow solid, and stirred at room temperature overnight. The resulting white solid was filtered, washed with ethyl acetate and dried *in vacuo* to give the titled compound (2.6 g).
¹H NMR(400MHz, CD₃OD) δ 8.76(s, 1H), 8.00-7.90(m, 1H), 7.75-7.65(m, 1H), 4.70-3.70(m, 5H), 2.35-1.90(m, 2H), 1.45(s, 9H)

### Reference Example 47. (S)-tert-butyl{1-(2-chloropyrido[3,2-d]pyrimidin-4-yl)pyrrolidin-3-yl}(methyl)carbamate

Sodium hydride (86 mg, 2.15 mmol, 60 wt%) was added into *N,N*-dimethylformamide (10 ml) solution of *(S)-tert-*butyl {1-(2-chloropyrido[3,2-*d*]pyrimidin-4-yl)pyrrolidin-3-yl}carbamate (0.5 g, 1.43 mmol) prepared in Reference Example 46 at 0 °C, and they were stirred for 10 minutes. Methyl iodide (0.11 ml, 1.72 mmol) was added into the reaction solution, and they were stirred at room temperature overnight. Water was added to terminate the reaction, and extracted with ethyl acetate. The extract was dried with anhydrous magnesium sulfate and concentrated under reduced pressure to give the titled compound (0.6 g) as a yellow solid.
¹H NMR(400MHz, CD₃OD) δ 8.74(s, 1H), 7.90(d, 1H), 7.70-7.65(m, 1H), 4.80-4.65(m, 2H), 4.40-4.20(m, 1H), 4.15-3.95(m, 1H), 3.80-3.70(m, 1H), 2.87(s, 3H), 2.30-2.10(m, 2H), 1.49(s, 9H)

### Reference Example 48. (S)-tert-butyl {1-(2-chloropyrido[3,2-d]pyrimidin-4-yl)pyrrolidin-3-yl}(propyl)carbamate

Sodium hydride (80 mg, 1.71 mmol, 60 wt%) was added into *N,N*-dimethylformamide (10 ml) solution of *(S)-tert-*butyl {1-(2-chloropyrido[3,2-d]pyrimidin-4-yl)pyrrolidin-3-yl}carbamate (0.4 g, 1.14 mmol) prepared in Reference Example 46 at 0 °C, and they were stirred for 10 minutes. 1-bromopropane (0.13 ml, 1.37 mmol) was added into the reaction solution, and they were stirred at room temperature for 2 hours. Water was added to terminate the reaction, and extracted with ethyl acetate. The extract was dried with anhydrous magnesium sulfate and concentrated under reduced pressure. The resulting residue was purified with silica gel column chromatography (*n*-hexane/ethyl acetate = 2/1) to give the titled compound (0.3 g) as a white solid.
¹H NMR(400MHz, CDCl₃) δ 8.70(s, 1H), 7.98(d, 1H), 7.65-7.55(m, 1H), 4.90-4.55(m, 2H), 4.30-4.05(m, 3H), 3.75-3.65(m, 1H), 3.25-3.00(m, 2H), 2.30-2.10(m, 2H), 1.65-1.55(m, 2H), 1.45(s, 9H), 0.90(t, 3H)

### Reference Example 49. (S)-tert-butyl {1-(2-chloropyrido[3,2-d]pyrimidin-4-yl)pyrrolidin-3-yl}(pentyl)carbamate

Sodium hydride (86 mg, 2.15 mmol, 60 wt%) was added into *N,N*-dimethylformamide (10 ml) solution of *(S)-tert-*butyl {1-(2-chloropyrido[3,2-d]pyrimidin-4-yl)pyrrolidin-3-yl}carbamate (0.4 g, 1.14 mmol) prepared in Reference Example 46 at 0 °C, and they were stirred for 10 minutes. 1-bromopentane (0.22 ml, 1.72 mmol) was added into the reaction solution, and they were stirred at room temperature for 3 hours. Water was added to terminate the reaction, and extracted with ethyl acetate. The extract was dried with anhydrous magnesium sulfate and concentrated under reduced pressure. The resulting residue was purified with silica gel column chromatography (*n*-hexane/ethyl acetate = 4/1) to give the titled compound (0.4 g) as a white solid.
¹H NMR(400MHz, CDCl₃) δ 8.70 (s, 1H), 7.98(d, 1H), 7.65-7.55(m, 1H), 4.90-4.55(m, 2H), 4.30-4.05(m, 2H), 3.75-3.65(m, 1H), 3.25-3.05(m, 2H), 2.25-2.05(m, 2H), 1.70-1.48(m, 2H), 1.48(s, 9H), 1.40-1.20(m, 4H), 0.90(t, 3H)

### Reference Example 50. (R)-N-{1-(2-chloropyrido[3,2-d]pyrimidin-4-yl)piperidin-3-yl}acetamide

### <Step 1> (R)-1-(2-chloropyrido[3,2-d]pyrimidin-4-yl)piperidin-3-amine

*(R)*-(-)-3-aminopiperidine dihydrochloride (1.14 g, 6.6 mmol) was added into chloroform (30 ml) solution of 2,4-dichloropyrido[3,2-d]pyrimidine (1.2 g, 6 mmol) and diisopropylethylamine (3.2 ml, 18 mmol), and they were stirred at room temperature overnight. The reaction solution was diluted with dichloromethane, washed with water, dried with anhydrous magnesium sulfate, and concentrated under reduced pressure. The resulting residue was purified with silica gel column chromatography (dichloromethane/methanol = 20/1) to give the titled compound (1 g) as a pale yellow oil.
¹H NMR(400MHz, CD₃OD) δ 8.75(s, 1H), 8.00-7.90(m, 1H), 7.75-7.65(m, 1H), 5.50(brs, 2H), 3.60-3.20(m, 2H), 3.05-2.95(m, 1H), 2.15-1.90(m, 2H), 1.80-1.65(m, 1H), 1.65-1.45(m, 1H)

### <Step 2> (R)-N-{1-(2-chloropyrido[3,2-d]pyrimidin-4-yl)piperidin-3-yl}acetamide

*(R)*-1-(2-chloropyrido[3,2-d]pyrimidin-4-yl)piperidin-3-amine (0.45 g, 1.7 mmol) prepared in Step 1 was dissolved in dichloromethane (10 ml). Triethylamine (0.48 ml, 3.4 mmol) and acetyl chloride (0.14 ml, 1.87 mmol) were added thereto at 0 °C, and they were stirred at room temperature for 4 hours. Water was added to the reaction solution, and the reaction solution was extracted with dichloromethane, dried with anhydrous magnesium sulfate and concentrated under reduced pressure to give the titled compound (0.5 g) as a yellow solid.
¹H NMR(400MHz, CDCl₃) δ 8.71(s, 1H), 8.05(d, 1H), 7.63(t, 1H), 6.49(brs, 1H), 4.67(brs, 2H), 4.18(brs, 2H), 3.94(brs, 1H), 2.10-1.70(m, 7H)

### Reference Example 51. (R)-tert-butyl{1-(2-chloropyrido[3,2-d]pyrimidin-4-yl)piperidin-3-yl}carbamate

*(R)*-1-(2-chloropyrido[3,2-d]pyrimidin-4-yl)piperidin-3-amine (0.54 g, 2.1 mmol) prepared in Step 1 of Reference Example 50 was dissolved in 1,4-dioxane (20 ml). Triethylamine (0.35 ml, 2.5 mmol) and di-*tert*-butyl dicarbonate (0.53 g, 2.5 mmol) were added thereto at room temperature, and they were stirred overnight and concentrated under reduced pressure. The resulting residue was purified with silica gel column chromatography (n-hexane/ethyl acetate = 2/1) to give the titled compound (0.6 g) as a white solid.
¹H NMR(400MHz, CDCl₃) 5 8.70(s, 1H), 8.10-8.00(m, 1H), 7.65-7.55(m, 1H), 5.20-3.70(m, 5H), 2.05-1.60(m, 4H), 1.41(s, 9H)

### Reference Example 52. (S)-N-{1-(2-chloro-6,7-dihydro-5H-cyclopenta[d]pyrimidin-4-yl)pyrrolidin-3-yl}acetamide

### <Step 1> 2-(methylthio)-6,7-dihydro-5H-cyclopenta[d]pyrimidin-4-ol

An aqueous solution (400 ml) of ethyl cyclopentanone-2-carboxylate (30 g, 0.19 mol), 2-methyl-2-thiopseudourea (0.21 mol) and sodium carbonate (20 g, 0.3 mol) was stirred at room temperature for 2 days. The resulting solid was filtered and dried *in vacuo* to give the titled compound (28 g), and this compound was used in the subsequent reaction without further purification.

### <Step 2> 6,7-dihydro-5H-cyclopenta[d]pyrimidin-2,4-diol

A mixture of 2-(methylthio)-6,7-dihydro-5H-cyclopenta[*d*]pyrimidin-4-ol (27 g, 0.15 mol) prepared in Step 1, acetic acid (200 ml) and water (90 ml) was stirred at reflux for 3 days. After cooling the reaction mixture to room temperature, the resulting solid was filtered, washed with ethyl acetate and dried in *vacuo* to give the titled compound (13.3 g) as a pale yellow solid. This compound was used in the subsequent reaction without further purification.

### <Step 3> 2,4-dichloro-6,7-dihydro-5H-cyclopenta[d]pyrimidine

A mixture of 6,7-dihydro-5*H*-cyclopenta[d]pyrimidin-2,4-diol (13.3 g, 87.4 mmol) prepared in Step 2 and phosphorus oxychloride (100 ml) was stirred at 130 °C overnight. After cooling the reaction mixture to room temperature, the same was added into ice water and basified with sodium bicarbonate. The aqueous layer was extracted with ethyl acetate, and the organic layer was dried on anhydrous magnesium sulfate and concentrated under reduced pressure. The resulting residue was purified with silica gel column chromatography (*n*-hexane/ethyl acetate = 10/1) to give the titled compound (13 g) as a pale yellow solid.
¹H NMR(400MHz, CDCl₃) δ 3.09(t, 2H), 3.00(t, 2H), 2.23(t, 2H)

### <Step 4> (S)-N-{1-(2-chloro-6,7-dihydro-5H-cyclopenta[d]pyrimidin-4-yl)pyrrolidin-3-yl}acetamide

Diisopropylethylamine (0.93 ml, 5.3 mmol) was added into chloroform (20 ml) solution of 2,4-dichloro-6,7-dihydro-5*H-*cyclopenta[d]pyrimidine (1 g, 5.3 mmol) prepared in Step 3 and *(S)*-3-acetamidopyrrolidine (0.75 g, 5.8 mmol), and they were stirred at 50 °C overnight. Water was added to the reaction solution, and extracted with dichloromethane. The extract was dried with anhydrous magnesium sulfate and concentrated under reduced pressure. The resulting residue was purified with silica gel column chromatography (dichloromethane/methanol = 60/1) of the titled compound (1.3 g) as a white solid.
¹H NMR(400MHz, CDCl₃) δ 6.92(brs, 1H), 4.55(brs, 1H), 3.85-3.65(m, 4H), 3.14(t, 2H), 2.79(q, 2H), 2.20-2.03(m, 4H), 2.03(s, 3H)

### Reference Example 53. (S)-tert-butyl {1-(2-chloro-6,7-dihydro-5H-cyclopenta[d]pyrimidin-4-yl)pyrrolidin-3-yl}carbamate

Diisopropylethylamine (3.7 ml, 21.2 mmol) was added into chloroform (100 ml) solution of 2,4-dichloro-6,7-dihydro-5H-cyclopenta[d]pyrimidine (4 g, 21.2 mmol) prepared in Step 3 of Reference Example 52 and *3-*(*(S)-tert-*butoxycarbonylamino)pyrrolidine (4.3 g, 23.3 mmol), and they were stirred at 50 °C overnight and concentrated under reduced pressure. The resulting residue was purified with silica gel column chromatography (n-hexane/ethyl acetate = 4/1) to give the titled compound (6.1 g) as a white solid.
¹H NMR(400MHz, CDCl₃) δ 4.66(brs, 1H), 4.27(brs, 1H), 4.00-3.90(m, 1H), 3.85-3.75(m, 2H), 3.65-3.55(m, 1H), 3.10(t, 2H), 2.82(t, 2H), 2.25-2.15(m, 1H), 2.10-2.00(m, 2H), 2.00-1.90(m, 1H), 1.45(s, 9H)

### Reference Example 54. (S)-tert-butyl {1-(2-chloro-6,7-dihydro-5H-cyclopenta[d]pyrimidin-4-yl)pyrrolidin-3-yl}(methyl)carbamate

Sodium hydride (0.18 g, 4.43 mmol, 60 wt%) was added into *N,N*-dimethylformamide (15 ml) solution of *(S)-tert-*butyl {1-(2-chloro-6,7-dihydro-5*H*-cyclopenta[*d*]pyrimidin-4-yl)pyrrolidin-3-yl}carbamate (1.5 g, 2.95 mmol) prepared in Reference Example 53 at 0 °C, and they were stirred for 10 minutes. Methyl iodide (0.22 ml, 3.54 mmol) was slowly added to the reaction solution, and they were stirred at room temperature overnight. Water was added to terminate the reaction, and extracted with ethyl acetate. The extract was dried with anhydrous magnesium sulfate and concentrated under reduced pressure. The resulting residue was by purified with silica gel column chromatography *(n-*hexane/ethyl acetate = 4/1) to give the titled compound (1 g) as a pale yellow solid.
¹H NMR(400MHz, CDCl₃) δ 4.78(brs, 1H), 3.95-3.85(m, 2H), 3.64(q, 1H), 3.60-3.50(m, 1H), 3.11(t, 2H), 2.85-2.75(m, 5H), 2.20-2.00(m, 4H), 1.48(s, 9H)

### Reference Example 55. (S)-tert-butyl {1-(2-chloro-6,7-dihydro-5H-cyclopenta[d]pyrimidin-4-yl)pyrrolidin-3-yl}(propyl)carbamate

The titled compound was prepared as a pale yellow solid in the same manner as Reference Example 54 by using (S)-tert-butyl {1-(2-chloro-6,7-dihydro-5*H-*cyclopenta[*d*]pyrimidin-4-yl)pyrrolidin-3-yl}carbamate prepared in Reference Example 53 and 1-bromopropane.
¹H NMR(400MHz, CDCl₃) δ 4.53(brs, 1H), 4.00-3.90(m, 2H), 3.65-3.50(m, 2H), 3.20-3.00(m, 4H), 2.82(t, 2H), 2.15-2.00(m, 4H), 1.65-1.50(m, 2H), 1.45(s, 9H), 0.89(s, 3H); (Yield: 90 %)

### Reference Example 56. (R)-N-{1-(2-chloro-6,7-dihydro-5H-cyclopenta[d]pyrimidin-4-yl)piperidin-3-yl}acetamide

*(R)*-(-)-3-aminopiperidine dihydrochloride (1 g, 5.82 mmol) was added into chloroform (30 ml) solution of 2,4-dichloro-6,7-dihydro-5H-cyclopenta[*d*]pyrimidine (1 g, 5.29 mmol) prepared in Step 3 of Reference Example 52 and diisopropylethylamine (3.7 ml, 21.2 mmol), and they were stirred at 60 °C overnight. After cooling to room temperature, acetyl chloride (0.39 ml, 5.42 mmol) was added thereto, and the reaction solution was stirred at room temperature overnight and concentrated under reduced pressure. The resulting residue was diluted with dichloromethane, washed with water, dried with anhydrous magnesium sulfate, and concentrated under reduced pressure. The resulting residue was purified with silica gel column chromatography (*n*-hexane/ethyl acetate = 4/1) to give the titled compound (1 g) as a white solid.
¹H NMR(400MHz, CDCl₃) δ 6.02(brs, 1H), 4.05-3.85(m, 3H), 3.55-3.45(m, 2H), 3.10-3.00(m, 1H), 2.95-2.80(m, 3H), 2.07(q, 2H), 1.98(s, 3H), 1.97-1.90(m, 1H), 1.65-1.55(m, 1H)

### Reference Example 57. (R)-tert-butyl{1-(2-chloro-6,7-dihydro-5H-cyclopenta[d]pyrimidin-4-yl)piperidin-3-yl}carbamate

*(R)*-(-)-3-aminopiperidine dihydrochloride (1 g, 5.82 mmol) was added into ethanol (20 ml) solution of 2,4-dichloro-6,7-dihydro-5*H*-cyclopenta[d]pyrimidine (1 g, 5.29 mmol) prepared in Step 3 of Reference Example 52 and diisopropylethylamine (3 ml, 21.2 mmol), and they were stirred at room temperature overnight and concentrated under reduced pressure. The resulting residue was diluted with dichloromethane, washed with water, dried with anhydrous magnesium sulfate, and concentrated under reduced pressure. The resulting residue was dissolved in chloroform (20 ml), and then diisopropylethylamine (1.4 ml, 8 mmol) and di-*tert*-butyl dicarbonate (1.35 g, 6.2 mmol) were added thereto, and they were stirred at room temperature overnight. The reaction solution was washed with water, dried with anhydrous magnesium sulfate, and concentrated under reduced pressure. Diethylether (20 ml) was added to the resulting residue, and they were stirred at room temperature for 1 hour. The resulting white solid was filtered to give the titled compound (0.7 g)
¹H NMR(400MHz, CDCl₃) δ 4.68(brs, 1H), 3.95-3.35(m, 5H), 3.10-2.90(m, 2H), 2.83(t, 2H), 2.10-1.90(m, 3H), 1.85-1.60(m, 2H), 1.50-1.45(m, 1H), 1.44(s, 9H)

### Reference Example 58. (R)-tert-butyl{1-(2-chloro-6,7-dihydro-5H-cyclopenta[d]pyrimidin-4-yl)piperidin-3-yl}(methyl)carbamate

Sodium hydride (0.75 mg, 1.88 mmol, 60 wt%) was added into *N,N*-dimethylformamide (5 ml) solution of *(R)-tert-*butyl {1-(2-chloro-6,7-dihydro-5*H*-cyclopenta[*d*]pyrimidin-4-yl)piperidin-3-yl}carbamate (330 mg, 0.94 mmol) prepared in Reference Example 57 at room temperature, and they were stirred for 10 minutes. Methyl iodide (0.1 ml, 1.41 mmol) was slowly added to the reaction solution, and they were stirred at room temperature overnight. Water was added terminate the reaction, and extracted with ethyl acetate. The extract was dried with anhydrous magnesium sulfate and concentrated under reduced pressure. The resulting residue was purified silica gel column chromatography *(n-*hexane/ethyl acetate = 2/1) to give the titled compound (280 mg) as a colorless oil.
¹H NMR(400MHz, CDCl₃) δ 4.50(d, 1H), 4.41(brs, 1H), 3.93(brs, 1H), 3.15-2.77(m, 4H), 2.78(s, 3H), 2.79-2.65(m, 2H), 2.15-2.00(m, 2H), 2.00-1.60(m, 4H), 1.47(s, 9H)

### Reference Example 59. (R)-tert-butyl{1-(2-chloro-5,6,7,8-tetrahydroquinazolin-4-yl)piperidin-3-yl}(methyl)carbamate

### <Step 1> (R)-1-(2-chloro-5,6,7,8- tetrahydroquinazolin-4-yl)piperidin-3-amine

*(R)*-(-)-3-aminopiperidine dihydrochloride (1.14 g, 6.6 mmol) was added into chloroform (30 ml) solution of 2,4-dichloro-5,6,7,8-tetrahydroquinazoline (1.2 g, 6 mmol) prepared in Reference Example 33 and diisopropylethylamine (3.2 ml, 18 mmol), and they were stirred at room temperature overnight. The reaction solution was diluted with dichloromethane, washed with water, dried with anhydrous magnesium sulfate, and concentrated under reduced pressure to give the titled compound (1.5 g) as a yellow oil. This compound was used in the subsequent reaction without further purification.

### <Step 2> (R)-tert-butyl(1-(2-chloro-5,6,7,8-tetrahydroquinazolin-4-yl)piperidin-3-yl)carbamate

(R)-1-(2-chloro-5,6,7,8-tetrahydroquinazolin-4-yl)piperidin-3-amine (1.5 g, 5.62 mmol) prepared in Step 1 was dissolved in chloroform (20 ml). Diisopropylethylamine (1.5 ml, 8.61 mmol) and di-*tert*-butyl dicarbonate (1.5 g, 6.87 mmol) were added thereto at room temperature, and the reaction solution was stirred overnight and concentrated under reduced pressure. The resulting residue was purified with silica gel column chromatography (*n*-hexane/ethyl acetate = 4/1) to give the titled compound (0.6 g) as a white solid. This compound was used in the subsequent reaction without further purification.

### <Step 3> (R)-tert-butyl(1-(2-chloro-5,6,7,8-tetrahydroquinazolin-4-yl)piperidin-3-yl)(methyl)carbamate

Sodium hydride (78.6 mg, 5.46 mmol, 60 wt%) was added into *N,N*-dimethylformamide (10 ml) solution of (R)-tert-butyl (1-(2-chloro-5,6,7,8-tetrahydroquinazolin-4-yl)piperidin-3-yl)carbamate (0.5 g, 2.73 mmol) prepared in Step 2 at 0 °C, and they were stirred for 10 minutes. Methyl iodide (0.25 ml, 4.1 mmol) was slowly added to the reaction solution, and they were stirred at room temperature overnight. Water was added to terminate the reaction, and extracted with ethyl acetate. The extract was dried with anhydrous magnesium sulfate, and then concentrated under reduced pressure. The resulting residue was purified with silica gel column chromatography (n-hexane/ethyl acetate = 2/1) to give the titled compound (220 mg) as a pale yellow oil.
¹H NMR(400MHz, CDCl₃) δ 4.20-3.65(m, 3H), 2.90-2.50(m, 7H), 2.51(brs, 2H), 2.00-1.60(m, 8H), 1.47(s, 9H)

### Reference Example 60. (R)-1-(2-chloro-6,7-dihydro-5H-cyclopenta[d]pyrimidin-4-yl)-N-methylpiperidine-3-carboxamide

Diisopropylethylamine (3.7 ml, 21.2 mmol) was added into chloroform (25 ml) solution of 2,4-dichloro-6,7-dihydro-5*H-*cyclopenta[d]pyrimidine (1 g, 5.29 mmol) prepared in Step 3 of Reference Example 52 and (R)-(-)-3-piperidincarboxylic acid (0.75 g, 5.82 mmol), and they were stirred at 70 °C overnight. After cooing the reaction solution to room temperature, methylamine hydrochloride (0.36 g, 5.29 mmol), *N*-(3-dimethylaminopropyl)-*N'*-ethylcarbodiimide hydrochloride (1.1 g, 5.82 mmol) and 1-hydroxybenzotriazole hydrate (0.79 g, 5.82 mmol) were added thereto, and they were stirred at room temperature overnight. The reaction solution was diluted with dichloromethane, washed with water, dried with anhydrous magnesium sulfate, and concentrated under reduced pressure. The resulting residue was crystallized by using ethyl acetate to give the titled compound (1.1 g) as a white solid.
¹H NMR(400MHz, CDCl₃) δ 6.31(brs, 1H), 4.21(d, 1H), 4.07(d, 1H), 3.60(t, 1H), 3.30(t, 1H), 2.97(t, 2H), 2.95-2.75(m, 5H), 2.42(brs, 1H), 2.15-2.00(m, 3H), 2.00-1.90(m, 1H), 1.80-1.70(m, 1H), 1.55(d, 1H)

### Examples

### Example 1. (S)-N¹-{4-(3-aminopyrrolidin-1-yl)-6,7-dimethoxyquinazolin-2-yl}-4-chlorobenzene-1,3-diamine dihydrochloride

A mixture of *(S)*-tert-butyl {1-(2-chloro-6,7-dimethoxyquinazolin-4-yl)pyrrolidin-3-yl}carbamate (35 mg, 0.09 mmol) prepared in Reference Example 1, palladium acetate (1 mg, 5 mol%), 4,5-bis(diphenylphosphino)-9,9-dimethylxanthine (5.2 mg, 10 mol%), cesium carbonate (59 mg, 0.18 mmol), 4-chloro-1,3-diaminobenzene (15.7 mg, 0.11 mmol) and 1,4-dioxane (1 ml) was stirred for 1 hour in a microwave (600 W). After cooling the reaction mixture to room temperature, the same was concentrated under reduced pressure. The resulting residue was purified with silica gel column chromatography (*n*-hexane/ethyl acetate = 1/1 → dichloromethane/methanol = 20/1). The resulting residue was dissolved in ethyl acetate, and then hydrochloric acid gas was added thereto. The resulting solid was filtered, washed, dried to prepare the titled compound (1.2 mg) as a pale yellow solid.
¹H-NMR (400MHz, CD₃OD) δ 7. 62 (brs, 1H), 7.52 (brs, 1H), 7.15(s, 1H), 6.90(s, 1H), 6.86(s, 1H), 4.41(brs, 3H), 4.26(brs, 2H), 3.98(s, 3H), 3.90(s, 3H), 2.66(brs, 1H), 2.35(brs, 1H)

### Example 2. (S)-N-{6,7-dimethoxy-4-(3-methylaminopyrrolidin-1-yl)-quinazolin-2-yl}-5-trifluoromethyl-benzene-1,3-diamine

A mixture of *(S)*-1-(2-chloro-6,7-dimethoxyquinazolin-4-yl)-N-methylpyrrolidin-3-amine (35 mg, 0.09 mmol) prepared in Reference Example 2, palladium acetate (1 mg, 5 mol%), 4,5-bis(diphenylphosphino)-9,9-dimethylxanthine (5.2 mg, 10 mol%), cesium carbonate (59 mg, 0.18 mmol), 5-(trifluoromethyl)-1,3-phenylenediamine (19 mg, 0.11 mmol) and 1,4-dioxane (1 ml) was stirred for 1 hour in a microwave (600 W). After cooling the reaction mixture to room temperature, the same was concentrated under reduced pressure. The resulting residue was purified with silica gel column chromatography (*n*-hexane/ethyl acetate = 1/1 → dichloromethane/methanol = 20/1) to prepare the titled compound (9.2 mg) as a pale yellow solid.
¹H-NMR (400MHz, CD₃OD) δ 7.55(s, 1H), 7.47(s, 1H), 7.20(s, 1H), 6.87(s, 1H), 6.55(s, 1H), 4.14(m, 1H), 3.99(m, 1H), 3.93(s, 3H), 3.89(s, 3H), 3.74(m, 1H), 3.39(m, 1H), 2.46(s, 3H), 2.26(m, 1H), 1.99(m, 1H)

### Examples 3 and 4

The titled compounds of Examples 3 and 4 were prepared in the same manner as Example 2 by reacting 3,5-diaminobenzonitrile or 5-(trifluoromethyl)-1,3-phenylenediamine respectively to (S)-1-(2-chloro-6,7-dimethoxyquinazolin-4-yl)-N-ethylpyrrolidin-3-amine prepared in Reference Example 3.

### Example 3. (S)-3-amino-5-{4-(3-ethylaminopyrrolidin-1-yl)-6,7-dimethoxyquinazolin-2-ylamino}-benzonitrile

¹H-NMR (400MHz, CD₃OD) δ 7.51(s, 1H), 7.42(s, 1H), 7.25(s, 1H), 6.87(s, 1H), 6.53(s, 1H), 4.10(m, 2H), 3.97(m, 1H), 3.92(s, 3H), 3.88(s, 3H), 3.70(m, 1H), 3.50(m, 1H), 2.79(m, 2H), 2.30(m, 1H), 1.94(m, 1H), 1.70(t, 3H); (Yield: 21 %)

### Example 4. (S)-N-{4-(3-ethylaminopyrrolidin-1-yl)-6,7-dimethoxyquinazolin-2-yl}-5-trifluoromethylbenzene-1,3-diamine

¹H-NMR (400MHz, CD₃OD) δ 7.50(s, 1H), 7.41(s, 1H), 7.20(s, 1H), 6.84(s, 1H), 6.56(s, 1H), 4.12(m, 2H), 4.00(m, 1H), 3.91(s, 3H), 3.88(s, 3H), 3.76(m, 1H), 3.53 (m, 1H), 2.80(m, 2H) 2.31(m, 1H), 1.99(m, 1H), 1.19(t, 3H); (Yield: 20 %)

### Examples 5 to 7

The titled compounds of Examples 5 to 7 were prepared in the same manner as Example 2 by reacting 3,5-diaminobenzonitrile, 5-amino-2-methylbenzonitrile or 5-(trifluoromethyl)-1,3-phenylenediamine respectively with *(S)-N*-{1-(2-chloro-6,7-dimethoxyquinazolin-4-yl)pyrrolidin-3-yl}acetamide prepared in Reference Example 4.

### Example 5. (S)-N-(1-[2-{(3-amino-5-cyanophenyl)amino}-6,7-dimethoxyquinazolin-4-yl]pyrrolidin-3-yl)acetamide

¹H-NMR (400MHz, CD₃OD) δ 7.44(s, 1H), 7.37(s, 1H), 7.29(s, 1H), 6.88(s, 1H), 6.52(s, 1H), 4.45(m, 1H), 4.14(m, 1H), 4.04(m, 1 H), 3.96(m, 1H), 3.92(s, 3H), 3.86(s, 3H), 3.78(m, 1H), 2.28(m, 1H), 2.05(m, 1H), 1.96(s, 3H); (Yield: 19%)

### Example 6. (S)-N-(1-[2-{(3-cyano-4-methylphenyl)amino}-6,7-dimethoxyquinazolin-4-yl]pyrrolidin-3-yl)acetamide

¹H-NMR (400MHz, CD₃OD) δ 8.23(d, 1H), 7.72(d, 1H), 7.43(s, 1H), 7.26(d, 1H), 6.91(s, 1H), 4.88(m, 1H), 4.18(m, 1H), 4.10(m, 1H), 4.07(m, 1H), 4.00(s, 3H), 3.96(s, 3H), 3.82(m, 1H), 2.45(s, 3H), 2.28(m, 1H), 2.07(m, 1H), 1.93(s, 3H); (Yield: 24 %)

### Example 7. (S)-N-{1-(2-[{3-amino-5-(trifluoromethyl)phenyl}amino]-6,7-dimethoxyquinazolin-4-yl)pyrrolidin-3-yl}acetamide

¹H-NMR (400MHz, CD₃OD) δ 7.50(s, 1H), 7.47(s, 1H), 7.21(s, 1H), 6.91(s, 1H), 6.56(s, 1H), 4.48(m, 1H), 4.20(m, 1H), 4.09(m, 1H), 4.02(m, 1H), 3.96(s, 3H), 3.89(s, 3H), 3.82(m, 1H), 2.27(m, 1H), 2.06(m, 1H), 1.95(s, 3H); (Yield: 21 %)

### Examples 8 to 12

The titled compounds of Examples 8 to 12 were prepared in the same manner as Example 2 by reacting 5-amino-2-fluorobenzonitrile, 5-amino-2-methylbenzonitrile, 3,5-diaminobenzonitrile, 2-(trifluoromethyl)-1,4-phenylenediamine or 5-(trifluoromethyl)-1,3-phenylenediamine respectively with *(R)-N*-{1-(2-chloro-6,7-dimethoxyquinazolin-4-yl)piperidin-3-yl}acetamide prepared in Reference Example 5.

### Example 8. (R)-N-(1-[2-{(3-cyano-4-fluoromethyl)amino}-6,7-dimethoxyquinazolin-4-yl]piperidin-3-yl)acetamide

¹H-NMR (400MHz, CD₃OD) δ 8.42(m, 1H), 7.62(m, 1H), 7.20(brs, 1H), 7.12(m, 2H), 7.03(s, 1H), 5.96(m, 1H), 4.26(brs, 1H), 3.80(m, 1H), 3.33(m, 1H), 3.12(m, 1H), 2.05(m, 1H), 2.02(s, 3H), 1.90(m, 1H), 1.82(m, 3H); (Yield: 24 %)

### Example 9. (R)-N-(1-[2-{(3-cyano-4-methylphenyl)amino}-6,7-dimethoxyquinazolin-4-yl]piperidin-3-yl)acetamide

¹H-NMR (400MHz, CD₃OD) δ 8.36(d, 1H), 7.50(m, 1H), 7.22(m, 2H), 7.10(s, 1H), 7.01(s, 1H), 6.13(m, 1H), 4.25(brs, 1H), 4.02(s, 3H), 4.00(s, 3H), 3.76(m, 1H), 3.37(m, 1H), 3.20(m, 1H), 2.49(s, 3H), 2.04(s+m, 4H), 1.82(m, 1H), 1.80(m, 3H), 1.66(m, 1H); (Yield: 23 %)

### Example 10. (R)-N-(1-[2-{(3-amino-5-cyanophenyl)amino}-6,7-dimethoxyquinazolin-4-yl]piperidin-3-yl)acetamide

¹H-NMR (400MHz, CD₃OD) δ 7.59(s, 1H), 7.44(s, 1H), 7.19(s, 1H), 7.11(s, 1H), 7.02(s, 1H), 6.54(s, 1H), 6.03(brs, 1H), 4.24(brs, 1H), 4.12(s+m, 7H), 3.91(m, 1H), 3.77(brs, 1H), 3.39(m, 1H), 3.20(m, 1H), 2.15(s, 3H), 1.95(m, 1H), 1.80(m, 4H), 1.66(m, 2H); (Yield: 34 %)

### Example 11. (R)-N-{1-(2-[{4-amino-3-(trifluoromethyl)phenyl}amino]-6,7-dimethoxyquinazolin-4-yl)piperidin-3-yl}acetamide

¹H-NMR (400MHz, CD₃OD) δ 7.96(d, 1H), 7.41(m, 1H), 7.06(m, 2H), 6.93(s, 1H), 6.72(m, 1H), 6.37(brs, 1H), 4.20(brs, 1H), 4.00(s, 3H), 3.97(s, 3H), 3.81(m, 1H), 3.54(m, 1H), 3.51(m, 1H), 3.42(m, 1H), 1.93(m, 2H), 1.88(m, 5H), 1.75(m, 2H); (Yield: 19 %)

### Example 12. (R)-N-{1-(2-[{3-amino-5-(trifluoromethyl)phenyl}amino]-6,7-dimethoxyquinazolin-4-yl)piperidin-3-yl}acetamide

¹H-NMR (400MHz, CD₃OD) δ 7.48(s, 1H), 7.23(brs, 1H), 7.14(m, 1H), 7.09(s, 1H), 6.98(s, 1H), 6.54(s, 1H), 6.19(m, 1H), 4.24(m, 1H), 4.01(s, 3H), 3.99(s, 3H), 3.88(m, 1H), 3.75(m, 1H), 3.47(m, 1H), 3.32(m, 1H), 1.99(m, 1H), 1.88(m, 6H), 1.75(m, 2H); (Yield: 23 %)

### Examples 13 and 14

The titled compounds of Examples 13 and 14 were prepared in the same manner as Example 2 by reacting 3,5-diaminobenzonitrile and 5-(trifluoromethyl)-1,3-phenylenediamine respectively with *(S)-N-*{1-(2-chloro-6-methoxyquinazolin-4-yl)pyrrolidin-3-yl}acetamide prepared in Reference Example 6.

### Example 13. (S)-N-(1-[2-{(3-amino-5-cyanophenyl)amino}-6-methoxyquinazolin-4-yl]pyrrolidin-3-yl)acetamide

¹H-NMR (400MHz, CD₃OD) δ 7.52(s, 1H), 7.48(s, 1H), 7.46(s, 1H), 7.32(s, 1H), 7.30(s, 1H), 6.55(s, 1H), 4.50(m, 1H), 4.21(m, 1H), 4.15(m, 1H), 4.06(m, 1H), 3.87(s, 3H), 2.35(m, 1H), 2.18(m, 1H), 1.95(s, 3H); (Yield: 20 %)

### Example 14. (5)-N-{1-(2-[{3-amino-5-(trifluoromethyl)phenyl}amino]-6-methoxyquinazolin-4-yl)pyrrolidin-3-yl}acetamide

¹H-NMR (400MHz, CD₃OD) δ 7.50(s, 1H), 7.42(s, 1H), 7.39(d, 1H), 7.26(d, 1H), 7.18(s, 1H), 6.55(s, 1H), 4.49(m, 1H), 4.19(m, 1H), 4.10(m, 1H), 3.98(m, 1H), 3.89(s, 3H), 2.27(m, 1H), 2.07(m, 1H), 1.95(s, 3H); (Yield: 32 %)

### Examples 15 and 16

The titled compounds of Examples 15 and 16 were prepared in the same manner as Example 2 by reacting 5-(trifluoromethyl)-1,3-phenylenediamine or 3,5-diaminobenzonitrile respectively with *(S)*-1-(2-chloro-6-methoxyquinazolin-4-yl)-*N*-methylpyrrolidin-3-amine prepared in Reference Example 7.

### Example 15. (S)-N¹-[6-methoxy-4-{3-(methylamino)pyrrolidin-1-yl}quinazolin-2-yl]-5-(trifluoromethyl)benzene-1,3-diamine

¹H-NMR (400MHz, CD₃OD) δ 7.53(s, 1H), 7.50(s, 1H), 7.42(d, 1H), 7.29(d, 1H), 7.17(s, 1H), 6.55(s, 1H), 4.12(m, 2H), 4.01(m, 1H), 3.85(s, 3H), 3.79(m, 1H), 3.41(m, 1H), 2.47(s, 3H), 2.28(m, 1H), 1.98(m, 1H); (Yield: 31 %)

### Example 16. (S)-3-amino-5-([6-methoxy-4-{3-(methylamino)pyrrolidin-1-yl}quinazolin-2-yl]amino) benzonitrile

¹H-NMR (400MHz, CD₃OD) δ 7.49(s, 1H), 7.42(s, 1H), 7.41∼7.39(m, 1H), 7.26(m, 1H), 7.24(s, 1H), 6.52(s, 1H), 4.08(m, 2H), 3.92(m, 1H), 3.83(s, 3H), 3.73(m, 1H), 3.39(m, 1H), 2.47(s, 3H), 2.26(m, 1H), 1.96(m, 1H); (Yield: 21 %)

### Examples 17 to 19

The titled compounds of Examples 17 to 19 were prepared in the same manner as Example 2 by reacting 3,5-diaminobenzonitrile, 5-(trifluoromethyl)-1,3-phenylenediamine or 2-(trifluoromethyl)-1,4-phenylenediamine respectively to *(S)*-1-(2-chloro-6-methoxyquinazolin-4-yl)-N-ethylpyrrolidin-3-amine prepared in Reference Example 8.

### Example 17. (S)-3-amino-5-([4-{3-(ethylamino)pyrrolidin-1-yl}-6-methoxyquinazolin-2-yl]amino)benzonitrile

¹H-NMR (400MHz, CD₃OD) δ 7.50(s, 1H), 7.44(s, 1H), 7.40(m, 1H), 7.26(m, 2H), 6.53(s, 1H), 4.10(m, 2H), 3.96(m, 1H), 3.83(s, 3H), 3.70(m, 1H), 3.47(m, 1H), 2.73(m, 2H), 2.28(m, 1H), 1.92(m, 1H), 1.17(t, 3H); (Yield: 27 %)

### Example 18. (S)-N¹-[4-{3-(ethylamino)pyrrolidin-1-yl}-6-methoxyquinazolin-2-yl]-5-(trifluoromethyl)benzene-1,3-diamine

¹H-NMR (400MHz, CD₃OD) δ 7.53(s, 1H), 7.47(s, 1H), 7.41(d, 1H), 7.27(d, 1H), 7.18(s, 1H), 6.55(s, 1H), 4.13(m, 2H), 3.98(m, 1H), 3.84(s, 3H), 3.74(m, 1H), 3.49(m, 1H), 2.74(m, 2H), 2.28(m, 1H), 1.94(m, 1H), 1.17(t, 3H); (Yield: 32 %)

### Example 19. (S)-N¹-[4-{3-(ethylamino)pyrrolidin-1-yl}-6-methoxyquinazolin-2-yl]-3-(trifluoromethyl)benzene-1,4-diamine

¹H-NMR (400MHz, CD₃OD) δ 7.94(s, 1H), 7.50(s, 1H), 7.45(d, 1H), 7.37(d, 1H), 7.28(d, 1H), 6.81(d, 1H), 4.11(m, 2H), 3.95(m, 1H), 3.85(s, 3H), 3.75(m, 1H), 3.50(m, 1H), 2.74(m, 2H), 2.28(m, 1H), 1.95(m, 1H), 1.18(t, 3H); (Yield: 34 %)

### Examples 20 to 24

The titled compounds of Examples 20 to 24 were prepared in the same manner as Example 2 by reacting 5-(trifluoromethyl)-1,3-phenylenediamine, 3,5-diaminobenzonitrile, 2,5-diaminobenzonitrile, 5-amino-2-methylbenzonitrile or 2-(trifluoromethyl)-1,4-phenylenediamine respectively with *(R)-N*-{1-(2-chloro-6-methoxyquinazolin-4-yl)piperidin-3-yl}acetamide prepared in Reference Example 9.

### Example 20. (R)-N-{1-(2-[{3-amino-5-(trifluoromethyl)phenyl}amino]-6-methoxyquinazolin-4-yl)piperidin-3-yl}acetamide

¹H-NMR (400MHz, CD₃OD) δ 7.50(m, 2H), 7.31(m, 1H), 7.26(s, 1H), 7.19(s, 1H), 6.56(s, 1H), 4.15(m, 2H), 3.94(m, 1H), 3.90(s, 3H), 3.24(m, 1H), 3.05(m, 1H), 2.05(m, 2H), 1.92(s, 3H), 1.66(m, 1H), 1.23(m, 1H); (Yield: 24 %)

### Example 21. (R)-N-(1-[2-{(3-amino-5-cyano phenyl)amino}-6-methoxyquinazolin-4-yl]piperidin-3-yl)acetamide

¹H-NMR (400MHz, CD₃OD) δ 7.54 (m, 2H), 7.35 (s, 1H), 7.30(m, 1H), 7.20(s, 1H), 6.55(s, 1H), 4.14(m, 2H), 3.97(m, 1H), 3.91(s, 3H), 3.24(m, 1H), 3.02(m, 1H), 2.03(m, 2H), 1.87(s, 3H), 1.63(m, 1H), 1.24(m, 1H); (Yield: 20 %)

### Example 22. (R)-N-(1-[2-{(4-amino-3-cyano phenyl)amino}-6-methoxyquinazolin-4-yl]piperidin-3-yl)acetamide

¹H-NMR (400MHz, CD₃OD) δ 7.88(s, 1H), 7.55(d, 1H), 7.45(d, 1H), 7.27(d, 1H), 7.16(s, 1H), 6.80(d, 1H), 4.10(m, 2H), 3.97(m, 1H), 3.89(s, 3H), 3.19(m, 1H), 3.01(m, 1H), 2.04(m, 2H), 1.92(s, 3H), 1.80(m, 1H), 1.62(m, 1H); (Yield: 34 %)

### Example 23. (R)-N-(1-[2-{(3-cyano-4-methyl phenyl)amino}-6-methoxyquinazolin-4-yl]piperidin-3-yl)acetamide

¹H-NMR (400MHz, CD₃OD) δ 8.31(s, 1H), 7.78(d, 1H), 7.52(d, 1H), 7.33-7.27(m, 2H), 7.21(s, 1H), 4.17(m, 2H), 3.98(m, 1H), 3.91(s, 3H), 3.23(m, 1H), 3.01(m, 1H), 2.46(s, 3H), 2.04(m, 2H), 1.94(s, 3H), 1.87(m, 1H), 1.64(m, 1H); (Yield: 30 %)

### Example 24. (R)-N-{1-(2-[{4-amino-3-(trifluoromethyl)phenyl}amino]-6-methoxyquinazolin-4-yl)piperidin-3-yl}acetamide

¹H-NMR (400MHz, CD₃OD) δ 7.94(s, 1H), 7.47(d, 1H), 7.41(m, 1H), 7.30(d, 1H), 7.19(s, 1H), 6.82(d, 1H), 4.10(m, 2H), 3.95(m, 1H), 3.90(s, 3H), 3.22(m, 1H), 3.02(m, 1H), 2.04(m, 1H), 1.92(s+m, 3+1H), 1.80(m, 1H), 1.63(m, 1H); (Yield: 28 %)

### Example 25. (S)-N¹-[4-{3-(methylamino)pyrrolidin-1-yl}-7-(trifluoromethyl)quinazolin-2-yl]-5-(trifluoromethyl)benzene-1,3-diamine

A mixture of *(S)*-1-{2-chloro-7-(trifluoromethyl)quinazolin-4-yl}-*N*-methylpyrrolidin-3-amine (30 mg, 0.09 mmol) prepared in Reference Example 10 and 5-(trifluoromethyl)-1,3-phenylenediamine (25 mg, 0.14 mmol) was stirred for 1 hour in a microwave (600 W). After cooling the reaction mixture to room temperature, the reaction mixture was concentrated under reduced pressure. The resulting residue was purified with silica gel column chromatography (*n*-hexane/ethyl acetate = 1/1 → dichloromethane/methanol = 20/1). The resulting residue was dissolved in ethyl acetate, and hydrochloric acid gas was added thereto. The resulting solid was filtered, washed and dried to prepare the titled compound (10.1 mg) as a pale yellow solid.

¹H-NMR (400MHz, CD₃OD) δ 8.31(d, 1H), 7.74 (s, 1H), 7.48(s, 1H), 7.35(d, 1H), 7.30(s, 1H), 6.59(s, 1H), 4.20(m, 2H), 4.04(m, 1H), 3.91(m, 1H), 3.59(m, 1H), 2.53(s, 3H), 2.36(m, 1H), 2.11(m, 1H)

### Examples 26 and 27

The titled compounds of Examples 26 and 27 were prepared in the same manner as Example 25 by reacting 5-(trifluoromethyl)-1,3-phenylenediamine or 3,5-diaminobenzonitrile respectively with *(S)*-1-{2-chloro-7-(trifluoromethyl)quinazolin-4-yl}-*N*-ethylpyrrolidin-3-amine prepared in Reference Example 11.

### Example 26. (S)-N¹⁻[4-{3-(ethylamino)pyrrolidin-1-yl}-7-(trifluoromethyl)quinazolin-2-yl]-5-(trifluoromethyl)benzene-1,3-diamine

¹H-NMR (400MHz, CD₃OD) δ 8.30(d, 1H), 7.71(s, 1H), 7.49(s, 1H), 7.35(d, 1H), 7.29(s, 1H), 6.59(s, 1H), 4.18(m, 2H), 4.09(m, 1H), 3.83(m, 1H), 3.61(m, 1H), 2.77(m, 2H), 2.31(m,1 H), 1.98(m,1 H), 1.16(t, 3H); (Yield: 19 %)

### Example 27. (S)-3-amino-5-([4-{3-(ethylamino)pyrrolidin-1-yl}-7-(trifluoromethyl)quinazolin-2-yl]amino)benzonitrile

¹H-NMR (400MHz, CD₃OD) δ 8.30(d, 1H), 7.74(s, 1H), 7.47(s, 1H), 7.41(s, 1H), 7.34(d, 1H), 6.58(s, 1H), 4.16(m, 1H), 4.07(m, 1H), 3.79(m, 1H), 3.59(m, 1H), 2.76(m, 2H), 2.29(m,1H), 2.01(m, 1H), 1.23(t, 3H); (Yield: 17 %)

### Examples 28 and 29

The titled compounds of Examples 28 and 29 were prepared in the same manner as Example 25 by reacting 3,5-diaminobenzonitrile or 5-(trifluoromethyl)-1,3-phenylenediamine respectively with *(S)-N*-[1-{2-chloro-7-(trifluoromethyl)quinazolin-4-yl}pyrrolidin-3-yl]acetamide prepared in Reference Example 12.

### Example 28. (S)-N-(1-[2-{(3-amino-5-cyanophenyl)amino}-7-(trifluoromethyl)quinazolin-4-yl]pyrrolidin-3-yl)acetamide

¹H-NMR (400MHz, CD₃OD) δ 8.28(d, 1H), 7.78(s, 1H), 7.44(d, 1H), 7.35(d, 1H), 6.58(s, 1H), 4.51(m, 1H), 4.25(m, 1H), 4.12(m, 1H), 4.07(m, 1H), 3.84(m, 1H), 2.33(m, 1H), 2.12(m, 1H), 1.92(s, 3H); (Yield: 20 %)

### Example 29. (S)-N-{1-(2-[{3-amino-5-(trifluoromethyl)phenyl}amino]-7-(trifluoromethyl)quinazolin-4-yl)pyrrolidin-3-yl}acetamide

¹H-NMR (400MHz, CD₃OD) δ 8.29(d, 1H), 7.73(d, 1H), 7.48(s, 1H), 7.35(d, 1H), 7.32(s, 1H), 6.59(s, 1H), 6.28(s, 1H), 4.52(m, 1H), 4.26(m, 1H), 4.15∼4.05(m, 2H), 3.84(m, 1H), 2.33(m, 1H), 2.09(m, 1H), 1.92(s, 3H); (Yield: 27 %)

### Example 30. (S)-tert-butyl 1-{2-(3-amino-5-cyanophenylamino)quinazolin-4-yl}pyrrolidin-3-ylcarbamate hydrochloride

*n*-Butanol (1 ml) solution of *(S)*-tert-butyl 1-(2-chloroquinazolin-4-yl)pyrrolidin-3-ylcarbamate (20 mg, 0.06 mmol) prepared in Reference Example 13 and 3,5-diaminobenzonitrile (9.2 mg, 0.07 mmol) was stirred for 1 hour in a microwave (400 W). After cooling, the solution was concentrated. The resulting solid was washed with dichloromethane, filtered and dried *in vacuo* to prepare the titled compound (18 mg) as a white solid.
¹H NMR(400MHz, DMSO-d₆) δ 12.68(brs, 1H), 10.29(brs, 1H), 8.25(s, 1H), 7.86(t, 1H), 7.58(d,1H), 7.45(m, 1H), 7.21(t, 2H), 6.70(s, 1H), 5.84(brs, 2NH), 4.23-3.88(m, 5H), 2.19(m, 1H), 2.03(m, 1H), 1.39(s, 9H)

### Example 31. (S)-3-amino-5-{4-(3-aminopyrrolidin-1-yl)quinazolin-2-ylamino}benzonitriledihydrochloride

Hydrochloric acid gas was added to methanol (2 ml) solution of *(S)*-tert-butyl 1-{2-(3-amino-5-cyanophenylamino)quinazolin-4-yl}pyrrolidin-3-ylcarbamate hydrochloride (20 mg, 0.04 mmol) prepared in Example 30, and the solution was stirred at room temperature overnight. After concentrating the reaction mixture, it was crystallized with methanol and dichloromethane to prepare the titled compound (14 mg) as a pale yellow solid.
¹H NMR(400MHz, DMSO-d₆) δ 12.91(brs, 1H), 10.49(brs, 1H), 8.47-8.34(m, 2H), 7.91(m, 1H), 7.64-7.52(m, 1H), 7.17(m, 2H), 6.74(s, 1H), 4.55-3.92(m, 5H), 2.40-2.27(m, 2H)

### Example 32. (S)-N¹-{4-(3-aminopyrrolidin-1-yl)quinazolin-2-yl}-5-(trifluoromethyl)benzene-1,3-diamine dihydrochloride

### <Step 1> (S)-tert-butyl 1-[2-{3-amino-5-(trifluoromethyl)phenylamino}quinazolin-4-yl]pyrrolidin-3-ylcarbamate hydrochloride

The titled compound was prepared as a white solid in the same manner as Example 30 by using *(S)-tert*-butyl 1-(2-chloroquinazolin-4-yl)pyrrolidin-3-ylcarbamate prepared in Reference Example 13 and 3-(trifluoromethyl)-1,5-phenylenediamine.
¹H NMR(400MHz, DMSO-d₆) δ 12.60(brs, 1H), 10.27(brs, 1H), 8.25(s, 1H), 7.84(m, 1H), 7.57(m, 1H), 7.46(m, 1H), 7.33(m, 1H), 6.96(s, 1H), 6.66(s, 1H), 5.88(brs, 2NH), 4.22-3.73(m, 5H), 2.19(m, 1H), 2.02(m, 1H), 1.39(s, 9H); (Yield: 56 %)

### <Step 2> (S)-N¹-{4-(3-aminopyrrolidin-1-yl)quinazolin-2-yl}-5-(trifluoromethyl)benzene-1,3-diamine dihydrochloride

The titled compound was prepared as a pale yellow solid in the same manner as Example 31 by using *(S)*-tert-butyl 1-[2-{3-amino-5-(trifluoromethyl)phenylamino}quinazolin-4-yl]pyrrolidin-3-ylcarbamate hydrochloride prepared in Step 1.
¹H NMR(400MHz, DMSO-d₆) δ 12.91(brs, 1H), 10.93(brs, 1H), 8.36-8.16(m, 2H), 7.92(m, 1H), 7.64-7.54(m, 1H), 7.32-7.14(m, 2H), 6.69(s, 1H), 4.13-4.06(m, 5H), 2.41-2.14(m, 2H); (Yield: 99 %)

### Example 33. (S)-N-[1-{2-(3-amino-5-cyanophenylamino)quinazolin-4-yl}pyrrolidin-3-yl] propionamidehydrochloride

### <Step 1> (S)-3-amino-5-{4-(3-aminopyrrolidin-1-yl)quinazolin-2-ylamino}benzonitrile

A mixture of *(S)*-3-amino-5-{4-(3-aminopyrrolidin-1-yl)quinazolin-2-ylamino}benzonitrile dihydrochloride (500 mg, 1.20 mmol) prepared in Example 31 and 0.2 N sodium hydroxide solution (10 ml) was stirred at room temperature for 2 hours. The resulting solid was filtered and dried in *vacuo* at 50 °C to prepare the titled compound (343.5 mg) as a pale yellow solid.
¹H NMR(400MHz, DMSO-d₆) δ 9.12(s, 1H), 8.11(d, 1H), 7.60(m, 1H), 7.49(m, 2H), 7.17(m, 1H), 6.43(s, 1H), 5.48(s, 2NH), 4.03(m, 2H), 3.90(m, 1H), 3.60(m, 2H), 2.06(m, 1H), 1.72(m, 1H)

### <Step 2> (S)-N-[1-{2-(3-amino-5-cyanophenylamino)quinazolin-4-yl}pyrrolidin-3-yl] propionamide hydrochloride

A mixture of *(S)*-3-amino-5-{4-(3-aminopyrrolidin-1-yl)quinazolin-2-ylamino}benzonitrile (20 mg, 0.06 mmol) prepared in Step 1, propionic acid (4.8 µl, 0.06 mmol), diisopropylethylamine (22.2 µl, 0.13 mmol), *N*-(3-dimethylaminopropyl)-*N'*-ethylcarbodiimide hydrochloride (15.9 mg, 0.08 mmol), 1-hydroxybenzotriazole hydrate (11.2 mg, 0.08 mmol) and *N,N*-dimethylacetamide (0.5 ml) was stirred at room temperature overnight. Water was added to the reaction mixture, and the resulting solid was washed with 1 N sodium hydroxide aqueous solution, dissolved in methanol, and concentrated under reduced pressure. The resulting residue was purified with silica gel column chromatography (dichloromethane/methanol = 20/1) and dissolved in ethyl acetate (1 ml), and then hydrochloric acid gas was added thereto. The resulting white solid was filtered to prepare the titled compound (4 mg).
NMR(400MHz, CD₃OD) δ 8.36(m, 1H), 7.86(m, 1H), 7.57(m, 2H), 7.31(m, 2H), 6.94(m, 1H), 4.54-4.07(m, 5H), 2.34(m, 1H), 2.24(m, 2H), 2.11(m, 1H), 1.10(m, 3H)

### Example 34. (S)-N-[1-{2-(3-amino-5-cyanophenylamino)quinazolin-4-yl}pyrrolidin-3-yl] pentanamide

A mixture of *(S)*-3-amino-5-{4-(3-aminopyrrolidin-1-yl)quinazolin-2-ylamino}benzonitrile (20 mg, 0.06 mmol) prepared in Step 1 of Example 33, valeric acid (6.9 µl, 0.06 mmol), diisopropylethylamine (22.2 µl, 0.13 mmol), N-(3-dimethylaminopropyl)-*N'*-ethylcarbodiimide hydrochloride (15.9 mg, 0.08 mmol), 1-hydroxybenzotriazole hydrate (11.2 mg, 0.08 mmol) and *N,N*-dimethylacetamide (0.5 ml) was stirred at room temperature overnight. Water was added to the reaction mixture, and the resulting solid was washed with 1 N sodium hydroxide aqueous solution, dissolved in methanol, and concentrated under reduced pressure. The resulting residue was purified with silica gel column chromatography (dichloromethane/methanol = 20/1) to prepare the titled compound (15.2 mg) as a colorless oil.
¹H NMR(400MHz, CD₃OD) δ 8.08(d, 1H), 7.56(m, 1H), 7.49(m, 2H), 7.34(s, 1H), 7.18(t, 1H), 6.56(s, 1H), 4.48(m, 1H), 4.21-4.00(m, 3H), 3.81(m, 1H), 2.29(m, 1H), 2.19(m, 2H), 2.06(m, 1H), 1.58(m, 2H), 1.31(m, 2H), 0.90(t, 3H)

### Examples 35 to 38

The titled compounds of Examples 35 to 38 were prepared in the same manner as Example 34 by reacting phenylacetic acid, 3-phenylpropionic acid, 2-(thiophen-2-yl)acetic acid or *N,N*-dimethylglycine respectively with *(S)*-3-amino-5-{4-(3-aminopyrrolidin-1-yl)quinazolin-2-ylamino}benzonitrile prepared in Step 1 of Example 33.

### Example 35. (S)-N-[1-{2-(3-amino-5-cyanophenylamino)quinazolin-4-yl}pyrrolidin-3-yl]-2-phenylacetamide

¹H NMR(400MHz, CD₃OD) δ 8.08(d, 1H), 7.58(m, 1H), 7.50(m, 2H), 7.33(s, 1H), 7.24(m, 4H), 7.18(m, 2H), 6.56(s, 1H), 4.47(m, 1H), 4.18-4.00(m, 3H), 3.85(m, 1H), 3.50(s, 2H), 2.28(m, 1H), 2.09(m, 1H); (Yield: 40 %)

### Example 36. (S)-N-[1-{2-(3-amino-5-cyanophenylamino)quinazolin-4-yl}pyrrolidin-3-yl]-3-phenylpropionamide

¹H NMR(400MHz, CD₃OD) 5 8.03(d, 1H), 7.59(t, 1H), 7.49(m, 2H), 7.32(s, 1H), 7.18(t, 1H), 7.12(m, 4H), 7.02(m, 1H), 6.56(s, 1H), 4.40(m, 1H), 4.09(m, 1H), 3.88(m, 2H), 3.70(m, 1H), 2.89(t, 2H), 2.47(t, 2H), 2.17(m, 1H), 1.96(m, 1H); (Yield: 46 %)

### Example 37. (S)-N-[1-{2-(3-amino-5-cyanophenylamino) quinazolin-4-yl}pyrrolidin-3-yl]-2-(thiophen-2-yl)acetamide

¹H NMR(400MHz, CD₃OD) δ 8.11(d, 1H), 7.58(m, 1H), 7.50-7.47(m, 2H), 7.34(s, 1H), 7.21-7.18(m, 2H), 6.90(m, 2H), 6.56(s, 1H), 4.48(m, 1H), 4.22-3.86(m, 4H), 3.71(s, 2H), 2.29(m, 1H), 2.11(m, 1H); (Yield: 16%)

### Example 38. (S)-N-[1-{2-(3-amino-5-cyanophenylamino)quinazolin-4-yl}pyrrolidin-3-yl]-2-(dimethylamino)acetamide

¹H NMR(400MHz, CD₃OD) δ 8.07(d, 1H), 7.55(t, 1H), 7.47(m, 2H), 7.30(s, 1H), 7.15(t, 1H), 6.55(s, 1H), 4.54(m, 1H), 4.18(m, 1H), 4.07(m, 1H), 3.84(m, 1H), 3.81(m, 1H), 3.01(s, 2H), 2.28(m, 6H+1H), 2.10(m, 1H); (Yield: 47 %)

### Example 39. (S)-3-amino-5-[4-{3-(propylamino)pyrrolidin-1-yl}quinazolin-2-ylamino] benzonitrile dihydrochloride

Propionaldehyde (4.6 µl, 0.06 mmol) was added into methanol (1 ml) solution of *(S)-*3-amino-5-{4-(3-aminopyrrolidin-1-yl)quinazolin-2-ylamino}benzonitrile (20 mg, 0.06 mmol) prepared in Step 1 of Example 33, and they were stirred at room temperature for 1 hour, and sodium triacetoxyborohydride (24.5 mg, 0.12 mmol) was added thereto. The reaction solution was stirred at room temperature overnight, and then water was added to terminate the reaction. The reaction solution was extracted by adding chloroform and washed with saturated sodium bicarbonate aqueous solution. The extract was dried with anhydrous magnesium sulfate and filtered, and the solution was concentrated. The resulting residue was purified with silica gel column chromatography (dichloromethane/methanol = 20/1) and dissolved in ethyl acetate (1 ml), and hydrochloric acid gas was added thereto. The resulting white solid was filtered to prepare the titled compound (5 mg).
¹H NMR (400MHz, CD₃OD) δ 8.36(m, 1H), 7.90(t, 1H), 7.62(d, 1H), 7.56(t, 1H), 7.49(s, 1H), 7.36(s, 1H), 7.03(s, 1H), 4.53-4.12(m, 5H), 3.14(m, 2H), 2.64(m, 1H), 2.44(m, 1H), 1.79(m, 2H), 1.07(t, 3H)

### Example 40. (S)-3-amino-5-[4-{3-(butylamino)pyrrolidin-1-yl}quinazolin-2-ylamino]benzonitrile

Butyraldehyde (8.6 µl, 0.10 mmol) was added into methanol (1 ml) solution of *(S)*-3-amino-5-{4-(3-aminopyrrolidin-1-yl)quinazolin-2-ylamino}benzonitrile (30 mg, 0.09 mmol) prepared in Step 1 of Example 33, and they were stirred at room temperature for 1 hour, and sodium triacetoxyborohydride (24.5 mg, 0.12 mmol) was added thereto. The reaction solution was stirred at room temperature overnight, and water was added to terminate the reaction. The reaction solution was extracted by adding chloroform and washed with saturated sodium bicarbonate aqueous solution. The extract was dried with anhydrous magnesium sulfate and filtered, and the solution was concentrated. The resulting residue was purified with silica gel column chromatography (dichloromethane/methanol = 20/1) to prepare the titled compound (2.7 mg) as a colorless oil.
¹H NMR (400MHz, CD₃OD) δ 8.12(d, 1H), 7. 58 (m, 1H), 7.52-7.47(m, 2H), 7.29(s, 1H), 7.18(m, 1H), 6.56(s, 1H), 4.14(m, 2H), 4.01(m, 1H), 3.79(m, 1H), 3.54(m, 1H), 2.75(m, 2H), 2.31(m, 1H), 1.99(m, 1H), 1.56(m, 2H), 1.40(m, 2H), 0.96(m, 3H)

### Examples 41 to 51

The titled compounds of Examples 41 to 51 were prepared in the same manner as Example 40 by reacting valeraldehyde, isovaleraldehyde, cyclopropane carboxaldehyde, pivalaldehyde, benzaldehyde, acetone, methylethylketone, 2-pentanone, 2-hexanone, 5-methyl-2-hexanone or cyclohexanone respectively with *(S)*-3-amino-5-{4-(3-aminopyrrolidin-1-yl)quinazolin-2-ylamino}benzonitrile prepared in Step 1 of Example 33.

### Example 41. (S)-3-amino-5-[4-{3-(pentylamino)pyrrolidin-1-yl}quinazolin-2-ylamino] benzonitrile

¹H NMR(400MHz, CD₃OD) δ 8.17(d, 1H), 7.57 (m, 1H), 7.52-7.47(m, 2H), 7.30(s, 1H), 7.21(t, 1H), 6.56(s, 1H), 4.15(m, 2H), 4.03(m, 1H), 3.77(m, 1H), 3.48(m, 1H), 2.71(t, 2H), 2.30(m, 1H), 1.97(m, 1H), 1.57(m, 2H), 1.36(m, 4H), 0.93(m, 3H); (Yield: 3 %)

### Example 42. (S)-3-amino-5-[4-{3-(isopentyl amino)pyrrolidin-1-yl}quinazolin-2-ylamino]benzonitrile

¹H NMR(400MHz, CD₃OD) δ 8.13(d, 1H), 7.58(t, 1H), 7.51-7.47(m, 2H), 7.31(s, 1H), 7.19(t, 1H), 6.56(s, 1H), 4.14(m, 2H), 4.01(m, 1H), 3.78(m, 1H), 3.54(m, 1H), 2.74(m, 2H), 2.31(m, 1H), 1.98(m, 1H), 1.67(m, 1H), 1.46(m, 2H), 0.94(d, 6H); (Yield: 6 %)

### Example 43. (S)-3-amino-5-[4-{3-(cyclopropylmethylamino)pyrrolidin-1-yl}quinazolin-2-ylamino]benzonitrile

¹H NMR(400MHz, CD₃OD) δ 8.13(d, 1H), 7.58(m, 1H), 7.52-7.49(m, 2H), 7.30(s, 1H), 7.19(m, 1H), 6.56(s, 1H), 4.15(m, 2H), 4.02(m, 1H), 3.80(m, 1H), 3.58(m, 1H), 2.61(d, 2H), 2.31(m, 1H), 2.01(m, 1H), 1.01(m, 1H), 0.56(m, 2H), 0.24(m, 2H); (Yield: 6 %)

### Example 44. (S)-3-amino-5-[4-{3-(neopentyl amino)pyrrolidin-1-yl}quinazolin-2-ylamino]benzonitrile

¹H NMR (400MHz, CD₃OD) δ 8.16(d, 1H), 7.58(m, 2H), 7.47(d, 1H), 7.30(s, 1H), 7.19(t, 1H), 6.56(s, 1H), 4.15(m, 2H), 3.97(m, 1H), 3.76(m, 1H), 3.44(m, 1H), 2.48(s, 2H), 2.24(m, 1H), 1.95(m, 1H), 0.95(s, 9H); (Yield: 4 %)

### Example 45 . (S)-3-amino-5-[4-{3-(benzyl amino)pyrrolidin-1-yl}quinazolin-2-ylamino]benzonitrile

¹H NMR(400MHz, CD₃OD) δ 8.08(d, 1H), 7.60(m, 1H), 7.52 (s, 1H), 7.46(d, 1H), 7.39(m, 2H), 7.31(m, 3H), 7.25(m, 1H), 7.17(m, 1H), 6.57(s, 1H), 4.14(m, 2H), 3.99(m, 1H), 3.87(s, 2H), 3.80(m, 1H), 3.49(m, 1H), 2.30(m, 1H), 2.03(m, 1H); (Yield: 3 %)

### Example 46. (S)-3-amino-5-[4-{3-(isopropyl amino)pyrrolidin-1-yl}quinazolin-2-ylamino]benzonitrile

¹H NMR(400MHz, CD₃OD) δ 8.16(d, 1H), 7.63(t, 1H), 7.55(m, 2H), 7.23(m, 2H), 6.59(s, 1H), 4.29-4.12(m, 3H), 4.12-3.94(m, 2H), 2.50(m, 1H), 2.15(m, 1H), 1.32(m, 6H), 0.90(m, 1H); (Yield: 1 %)

### Example 47. (S)-3-amino-5-[4-{3-(sec-butylamino)pyrrolidin-1-yl}quinazolin-2-ylamino]benzonitrile

¹H NMR(400MHz, CD₃OD) δ 8.14(d, 1H), 7.57(t, 1H), 7.50(m, 2H), 7.27(m, 1H), 7.20(m, 1H), 6.57(s, 1H), 4.19(m, 2H), 4.04(m, 1H), 3.77(m, 2H), 2.91(m, 1H), 2.36(m, 1H), 2.00(m, 1H), 1.68(m, 1H), 1.42(m, 1H), 1.17(m, 3H), 0.98(m, 3H); (Yield: 3 %)

### Example 48. (S)-3-amino-5-[4-{3-(pentan-2-ylamino)pyrrolidin-1-yl}quinazolin-2-ylamino]benzonitrile

¹H NMR(400MHz, CD₃OD) δ 8.12(d, 1H), 7.56(t, 1H), 7.49(m, 2H), 7.29(m, 1H), 7.18(m, 1H), 6.56(s, 1H), 4.14(m, 2H), 4.01(m, 1H), 3.74(m, 2H), 2.94(m, 1H), 2.34(m, 1H), 1.95(m, 1H), 1.58(m, 1H), 1.38(m, 3H), 1.17(m, 3H), 0.95(m, 3H); (Yield: 6 %)

### Example 49. (S)-3-amino-5-[4-{3-(hexan-2-ylamino)pyrrolidin-1-yl}quinazolin-2-ylamino]benzonitrile

¹H NMR (400MHz, CD₃OD) δ 8.10(d, 1H), 7.57(t, 1H), 7.47(m, 2H), 7.29(m, 1H), 7.16(m, 1H), 6.55(s, 1H), 4.11(m, 2H), 3.97(m, 1H), 3.69(m, 2H), 2.87(m, 1H), 2.31(m, 1H), 1.93(m, 1H), 1.60(m, 1H), 1.35(m, 5H), 1.15(m, 3H), 0.94(m, 3H); (Yield: 10 %)

### Example 50. (S)-3-amino-5-[4-{3-(5-methylhexan-2-ylamino)pyrrolidin-1-yl}quinazolin-2-ylamino]benzonitrile

¹H NMR(400MHz, CD₃OD) δ 8.11(d, 1H), 7.52 (t, 1H), 7.47(m, 2H), 7.30(m, 1H), 7.18(m, 1H), 6.56(s, 1H), 4.14(m, 2H), 3.99(m, 1H), 3.73(m, 2H), 2.87(m, 1H), 2.33(m, 1H), 1.96(m, 1H), 1.58(m, 2H), 1.36(m, 1H), 1.25(m, 2H), 1.16(m, 3H), 0.91(m, 6H); (Yield: 5 %)

### Example 51. (S)-3-amino-5-[4-{3-(cyclohexyl amino)pyrrolidin-1-yl}quinazolin-2-ylamino]benzonitrile

¹H NMR (400MHz, CD₃OD) δ 8.10(d, 1H), 7.55(t, 1H), 7.49(m, 2H), 7.28(s, 1H), 7.16(m, 1H), 6.55(s, 1H), 4.12(m, 2H), 3.96(m, 1H), 3.70(m, 2H), 2.70(m, 1H), 2.31(m, 1H), 2.02-1.92(m, 3H), 1.77(m, 2H), 1.66(m, 1H), 1.37-1.13(m, 5H); (Yield: 21 %)

### Example 52. (S)-N¹-[4-{3-(propylamino)pyrrolidin-1-yl}quinazolin-2-yl]-5-(trifluoromethyl)benzene-1,3-diamine dihydrochloride

*n*-Butanol (1.5 ml) solution of *(S)*-*tert*-butyl 1-(2-chloroquinazolin-4-yl)pyrrolidin-3-yl(propyl)carbamate (57.7 mg, 0.15 mmol) prepared in Reference Example 14 and 3-(trifluoromethyl)-1,5-phenylenediamine (31.2 mg, 0.18 mmol) was stirred at 130 °C overnight. After cooling the reaction solution, the same was concentrated under reduced pressure. The resulting residue was crystallized with n-butanol/dichloromethane and dried under reduced pressure. The resulting solid was dissolved in methanol (2 ml), and then hydrochloric acid gas was added thereto. The resulting white solid was filtered to prepare the titled compound (33.4 mg).
¹H NMR(400MHz, DMSO-d₆) δ 13.17(brs, 1H), 10.69(s, 1H), 9.77-9.54(m, 2H), 8.29(s, 1H), 7.90(t, 1H), 7.61(d, 1H), 7.53(t, 1H), 7.40-7.18(m, 2H), 6.78(s, 1H), 4.51-4.01(m, 5H), 2.98(m, 2H), 2.45(m, 2H), 1.71(m, 2H), 1.04(m, 3H)

### Example 53. (S)-N¹-[4-{3-(butylamino)pyrrolidin-1-yl}quinazolin-2-yl]-5-(trifluoromethyl)benzene-1,3-diamine dihydrochloride

The titled compound was prepared as a white solid in the same manner as Example 52 by using *(S)*-*tert*-butyl butyl{1-(2-chloroquinazolin-4-yl)pyrrolidin-3-yl}carbamate prepared in Reference Example 15.
¹H NMR(400MHz, DMSO-d₆) δ 13.03(brs, 1H), 10.66(s, 1H), 9.76-9.33(m, 2H), 8.50(s, 1H), 7.91(t, 1H), 7.60(d, 1H), 7.53(t, 1H), 7.40-7.14(m, 2H), 6.74(s, 1H), 4.29-4.01(m, 5H), 3.01(m, 2H), 2.40(m, 2H), 1.66(m, 2H), 1.36(m, 2H), 0.92(m, 3H); (Yield: 46 %)

### Example 54. (S)-N¹-[4-{3-(pentylamino)pyrrolidin-1-yl}quinazolin-2-yl]-5-(trifluoromethyl)benzene-1,3-diamine dihydrochloride

The titled compound was prepared as a white solid in the same manner as Example 52 by using *(S)*-*tert*-butyl 1-(2-chloroquinazolin-4-yl)pyrrolidin-3-yl(pentyl)carbamate prepared in Reference Example 16.
¹H NMR (400MHz, DMSO-d₆) δ 13.15(brs, 1H), 10.75(s, 1H), 9.77-9.39(m, 2H), 8.29(s, 1H), 7.92(t, 1H), 7.60(d, 1H), 7.53(t, 1H), 7.43-7.21(m, 2H), 6.82(s, 1H), 4.52-4.30(m, 5H), 3.00(m, 2H), 2.44(m, 2H), 1.69(m, 2H), 1.32(m, 4H), 0.89(m, 3H); (Yield: 43 %)

### Example 55. (S)-3-amino-5-[4-{3-(pentylamino)pyrrolidin-1-yl}quinazolin-2-ylamino]benzonitrile dihydrochloride

The titled compound was prepared as a white solid in the same manner as Example 52 by using *(S*)*-tert*-butyl 1-(2-chloroquinazolin-4-yl)pyrrolidin-3-yl(pentyl)carbamate which was prepared in Reference Example 16 and 3,5-diaminobenzonitrile.
¹H NMR(400MHz, DMSO-d₆) δ 13.09 (brs, 1H), 10.64(s, 1H), 9.75-9.28(m, 2H), 8.29(s, 1H), 7.90(t, 1H), 7.60(d, 1H), 7.50(t, 1H), 7.27-7.15(m, 2H), 6.77(s, 1H), 4.50-4.22(m, 5H), 3.01(m, 2H), 2.44(m, 1H), 2.00(m, 2H), 1.18(m, 4H), 0.89(m, 3H); (Yield: 74 %)

### Example 56. (S)-3-amino-5-[4-{3-(hexylamino)pyrrolidin-1-yl}quinazolin-2-ylamino]benzonitrile dihydrochloride

The titled compound was prepared as a white solid in the same manner as Example 52 by using *(S)*-*tert*-butyl 1-(2-chloroquinazolin-4-yl)pyrrolidin-3-yl(hexyl)carbamate prepared in Reference Example 17 and 3,5-diaminobenzonitrile.
¹H NMR (400MHz, DMSO-d₆) δ 10.85(s, 1H), 9.80-9.54(m, 2H), 8.29(s, 1H), 7.90(t, 1H), 7.59(d, 1H), 7.40(t, 1H), 7.27-7.12(m, 2H), 6.77(s, 1H), 4.35-4.06(m, 5H), 3.01(m, 2H), 2.46(m, 2H), 1.68(m, 2H), 1.34(m, 6H), 0.88(m, 3H); (Yield: 79 %)

### Example 57. (S)-N-[1-{2-(4-amino-3-cyanophenylamino)-8-methoxyquinazolin-4-yl}pyrrolidin-3-yl]acetamide

A mixture of *(S)*-*N*-{1-(2-chloro-8-methoxyquinazolin-4-yl)-pyrrolidin-3-yl}acetamide (30 mg, 0.09 mmol) prepared in Reference Example 19 and 2,5-diaminobenzonitrile (15 mg, 0.11 mmol) was stirred for 40 minutes in a microwave (400 W). After cooling the reaction solution to room temperature, the same was basified with sodium bicarbonate aqueous solution and extracted with dichloromethane. The extract was dried with anhydrous magnesium sulfate and concentrated under reduced pressure. The resulting residue was purified with silica gel column chromatography (dichloromethane/methanol = 30/1) to prepare the titled compound (1.4 mg) as a pale yellow oil.
¹H NMR(400MHz, CD₃OD) δ 7.95(s, 1H), 7.71(d, 1H), 7.49(d, 1H), 7.16(m, 2H), 6.80(d, 1H), 4.56(m, 1H), 4.21-4.00(m, 3H+3H), 3.83(m, 1H), 2.27(m, 1H), 2.06(m, 1H), 1.94(s, 3H)

### Example 58. (S)-N-[1-{2-(3-amino-5-trifluoromethylphenylamino)-8-methoxyquinazolin-4-yl}pyrrolidin-3-yl]acetamide

The titled compound was prepared as a pale yellow oil in the same manner as Example 57 by using *(S)-N-*{1-(2-chloro-8-methoxyquinazolin-4-yl)-pyrrolidin-3-yl}acetamide prepared in Reference Example 19 and 3-(trifluoromethyl)-1,5-phenylenediamine.
¹H NMR (400MHz, CDCl₃) δ 7.75(s, 1H), 7.56(s, 1H), 7. 11-7.00(m, 2H), 6.87(s, 1H), 6.51(s, 1H), 5.85(brs, 1H), 4.64(m, 1H), 4.23(m, 1H), 4.00(3H+1H), 3.85(m, 2H), 2.34(m, 1H), 2.02(m, 1H+3H); (Yield: 15 %)

### Example 59. (S)-N¹-{4-(3-methylaminopyrrolidin-1-yl)-8-methoxyquinazolin-2-yl}-5-trifluoromethylbenzene-1,3-diamine

### <Step 1> (S)-{1-(2-chloro-8-methoxyquinazolin-4-yl)-pyrrolidin-3-yl}methylamine

(3*S*)-(-)-3-(methylamino)pyrrolidine (0.17 ml, 1.57 mmol) was added to ethanol/chloroform (10/10 ml) solution of 2,4-dichloro-8-methoxyquinazoline (300 mg, 1.31 mmol) prepared in Reference Example 18, and they were stirred at room temperature overnight. The reaction solution was concentrated under reduced pressure, dissolved in dichloromethane, washed with water, dried with anhydrous magnesium sulfate, and concentrated under reduced pressure. The resulting residue was purified with silica gel column chromatography (dichloromethane/methanol = 20/1) to prepare the titled compound (371 mg) as a yellow oil.
¹H NMR (400MHz, CDCl₃) δ 7.70(d, 1H), 7.28 (t, 1H), 7.08(d, 1H), 4.13(m, 2H), 4.05(m, 1H+3H), 3.75(m, 1H), 3.40(m, 1H), 2.50 (s, 3H), 2.17(m, 1H), 1.94(m, 1H)

### <Step 2> (S)-N¹-{4-(3-methylaminopyrrolidin-1-yl)-8-methoxyquinazolin-2-yl}-5-trifluoromethylbenzene-1,3-diamine

A mixture of *(S)*-{1-(2-chloro-8-methoxyquinazolin-4-yl)-pyrrolidin-3-yl}methylamine (20 mg, 0.07 mmol) prepared in Step 1, 3-(trifluoromethyl)-1,5-phenylenediamine (14 mg, 0.08 mmol), palladium acetate (0.77 mg, 0.003 mmol), 4,5-bis(diphenylphosphino)-9,9-dimethylxanthine (4.0 mg, 0.01 mmol), cesium carbonate (44.5 mg, 0.14 mmol) and anhydrous 1,4-dioxane (1 ml) was stirred for 1 hour in a microwave (600 W). After cooling the reaction solution to room temperature, the same was filtered by celite. The filtrate was concentrated under reduced pressure. The resulting residue was purified with silica gel column chromatography (dichloromethane/methanol = 10/1) to prepare the titled compound (16.7 mg) as a pale yellow oil.
¹H NMR (400MHz, CD₃OD) δ 7.63(m, 2H), 7.04(m, 3H), 6.56(m, 1H), 4.03-3.86(m, 6H), 3.69(m, 1H), 2.43(s, 3H), 2.21(m, 1H), 1.89(m, 1H)

### Examples 60 to 62

The titled compounds of Examples 60 to 62 were prepared in the same manner as Example 57 by reacting 2-nitro-1,4-phenylenediamine, 3,5-diaminobenzonitrile or 3-(trifluoromethyl)-1,5-phenylenediamine respectively with *(S)*-{1-(2-chloro-8-methoxyquinazolin-4-yl)-pyrrolidin-3-yl}ethylamine prepared in Reference Example 20.

### Example 60. (S)-N¹-{4-(3-ethylaminopyrrolidin-1-yl)-8-methoxyquinazolin-2-yl}-3-nitrobenzene-1,4-diamine

¹H NMR(400MHz, CD₃OD) δ 8.92(s, 1H), 7.73(d, 1H), 7.38(d, 1H), 7.11(m, 2H), 6.90(d, 1H), 4.15(m, 2H), 4.04-3.98(m, 1H+3H), 3.84(m, 1H) ; 3.55(m, 1H), 2.79(m, 2H), 2.33(m, 1H), 1.98(m, 1H), 1.19(m, 3H); (Yield: 5 %)

### Example 61. (S)-3-amino-5-{4-(3-ethylaminopyrrolidin-1-yl)-8-methoxyquinazolin-2-ylamino}benzonitrile

¹H NMR(400MHz, CD₃OD) δ 7.65(m, 2H), 7.13-7.10(m, 3H), 6.54(s, 1H), 4.08-3.96(m, 6H), 3.70(m, 1H), 3.47(m, 1H), 2.74(m, 2H), 2.27(m, 1H), 1.90(m, 1H), 1.17(m, 3H); (Yield: 15 %)

### Example 62. (S)-N¹-{4-(3-ethylaminopyrrolidin-1-yl)-8-methoxyquinazolin-2-yl}-5-trifluoromethylbenzene-1,3-diamine

¹H NMR(400MHz, CD₃OD) δ 7.67-7.63(m, 1H+1H), 7.05(m, 3H), 6.56(s, 1H), 4.08(m, 2H), 4.04-3.86(m, 3H+1H), 3.67(m, 1H), 3.42(m, 1H), 2.71(m, 2H), 2.24(m, 1H), 1.87(m, 1H), 1.15(t, 3H); (Yield: 17 %)

### Examples 63 to 65

The titled compounds of Examples 63 to 65 were prepared in the same manner as Step 2 of Example 59 by reacting 3,5-diaminobenzonitrile, 3-(trifluoromethyl)-1,5-phenylenediamine or 2-(trifluoromethyl)-1,4-phenylenediamine respectively with *(R)*-*N*-{1-(2-chloro-8-methoxyquinazolin-4-yl)piperidin-3-yl}acetamide prepared in Reference Example 21.

### Example 63. (R)-N-[1-{2-(3-amino-5-cyanophenylamino)-8-methoxyquinazolin-4-yl}piperidin-3-yl]acetamide

¹H NMR(400MHz, CD₃OD) δ 7.70 (s, 1H), 7.45 (d, 1H), 7.19-7.13(m, 3H), 6.57(s, 1H), 4.25 (d, 1H), 4.09(m, 2H), 3.98(s, 3H), 3.31(m, 1H), 3.11(m, 1H), 2.06(m, 1H), 1.93(m, 3H+1H), 1.82(m, 1H), 1.60(m, 1H); (Yield: 12 %)

### Example 64 . (R)-N-[1-{2-(3-amino-5-trifluoromethylphenylamino)-8-methoxyquinazolin-4-yl}piperidin-3-yl]acetamide

¹H NMR(400MHz, CD₃OD) δ 7.72(s, 1H), 7.46(d, 1H), 7.16-7.10(m, 3H), 6.58(s, 1H), 4.24(m, 1H), 4.09-3.98(m, 5H), 3.50(m, 1H), 3.14(m, 1H), 2.02(m, 1H), 1.91(m, 3H+1H), 1.81(m, 1H), 1.62(m, 1H); (Yield: 15 %)

### Example 65. (R)-N-[1-{2-(4-amino-3-trifluoromethylphenylamino)-8-methoxyquinazolin-4-yl}piperidin-3-yl]acetamide

¹H NMR (400MHz, CD₃OD) δ 8.15(m, 1H), 7.43(m, 2H), 7.10(m, 2H), 6.81(m, 1H), 4.17(m, 1H), 4.00-3.92(m, 5H), 3.30(m, 1H), 3.09(m, 1H), 2.00(m, 1H), 1.86(m, 4H), 1.77(m, 1H), 1.61(m, 1H) ; (Yield: 17 %)

### Examples 66 and 67

The titled compounds of Examples 66 and 67 were prepared in the same manner as Step 2 of Example 59 by reacting 3,5-diaminobenzonitrile or 3-(trifluoromethyl)-1,5-phenylenediamine respectively with *(S)*-*N*-{1-(2-chloro-5-methylquinazolin-4-yl)-pyrrolidin-3-yl}acetamide prepared in Reference Example 23.

### Example 66. (S)-N-[1-{2-(3-amino-5-cyanophenylamino)-5-methylquinazolin-4-yl}pyrrolidin-3-yl]acetamide

¹H NMR(400MHz, CDCl₃) δ 7.44-7.27(m, 3H), 7.27(m, 1H), 7.01(m, 1H), 6.51(m, 1H), 5.87(m, 1H), 4.47(m, 1H), 3.92(m, 3H), 3.78-3.54(m, 3H), 2.62(s, 3H), 2.24(m, 1H), 1.91(s, 3H+1H); (Yield: 36 %)

### Example 67. (S)-N-(1-[2-{3-amino-5-(trifluoromethyl)phenylamino}-5-methylquinazolin-4-yl]pyrrolidin-3-yl)acetamide

¹H NMR (400MHz, CDCl₃) δ 7.51-7.44(m, 2H), 7.35(m, 1H), 7.23(m, 1H), 6.99(m, 1H), 6.53(m, 1H), 5.82(m, 1H), 4.47(m, 1H), 3.87(m, 3H), 3.75-3.57(m, 3H), 2.62(m, 3H), 2.24(m, 1H), 1.85(m, 3H+1H); (Yield: 27 %)

### Example 68. (S)-3-amino-5-[5-methyl-4-{3-(methylamino)pyrrolidin-1-yl}quinazolin-2-ylamino] benzonitrile

### <Step 1> (S)-1-(2-chloro-5-methylquinazolin-4-yl)-N-methylpyrrolidin-3-amine

The titled compound was prepared as a yellow oil in the same manner as Step 3 of Reference Example 13 by using 2,4-dichloro-5-methylquinazoline prepared in Reference Example 22 and (3*S*)-(-)-3-(methylamino)pyrrolidine.
¹H NMR(400MHz, CDCl₃) δ 7.56(m, 2H), 7.24(m, 1H), 4.08(m, 1H), 4.04(m, 2H), 3.83(m, 1H), 3.55(m, 1H), 2.65(s, 3H), 2.62(s, 3H), 2.22-2.12(m, 2H); (Yield: 51 %)

### <Step 2> (S)-3-amino-5-[5-methyl-4-{3-(methylamino)pyrrolidin-1-yl}quinazolin-2-ylamino] benzonitrile

*t*-Butanol (0.5 ml) solution of *(S)*-1-(2-chloro-5-methylquinazolin-4-yl)-N-methylpyrrolidin-3-amine (20 mg, 0.07 mmol) prepared in Step 1 and 3,5-diaminobenzonitrile (19 mg, 0.14 mmol) was stirred for 1 hour in a microwave (300 W). After cooling the reaction solution to room temperature, diisopropylethylamine was added thereto and the reaction solution was concentrated. The resulting residue was purified with silica gel column chromatography (dichloromethane/methanol = 20/1) to prepare the titled compound (0.7 mg) as a yellow oil.
¹H NMR (400MHz, CD₃OD) δ 7.49(m, 2H), 7.29(m, 2H), 7.09(m, 1H), 6.59 (s, 1H), 3.93-3.42(m, 5H), 2.65(s, 3H), 2.46(s, 3H), 2.23(m, 1H), 1.84(m, 1H)

### Examples 69 and 70

The titled compounds of Examples 69 and 70 were prepared in the same manner as Step 2 of Example 68 by reacting 3-(trifluoromethyl)-1,5-phenylenediamine or 2-(trifluoromethyl)-1,4-phenylenediamine respectively with *(S)*-1-(2-chloro-5-methylquinazolin-4-yl)-*N*-ethylpyrrolidin-3-amine prepared in Reference Example 24.

### Example 69. (S)-N¹-[4-{3-(ethylamino)pyrrolidin-1-yl}-5-methylquinazolin-2-yl]-5-(trifluoromethyl)benzene-1,3-diamine

¹H NMR(400MHz, CD₃OD) δ 7.49(m, 2H), 7.28(m, 1H), 7.20(s, 1H), 7.04(m, 1H), 6.58(s, 1H), 3.89-3.42(m, 5H), 2.69-2.64(m, 2H+3H), 2.19(m, 1H), 1.78(m, 1H), 1.13(t, 3H); (Yield: 5 %)

### Example 70. (S)-N¹-[4-{3-(ethylamino)pyrrolidin-1-yl}-5-methylquinazolin-2-yl]-3-(trifluoromethyl)benzene-1,4-diamine

¹H NMR (400MHz, CD₃OD) δ 7.92(m, 1H), 7.45(m, 2H), 7.19(m, 1H), 6.99(m, 1H), 6.83(m, 1H), 3.83-3.48(m, 5H), 2.61(m, 2H+3H), 2.16(m, 1H), 1.76(m, 1H), 1.11(m, 3H); (Yield: 13 %)

### Example 71. (R)-N-[1-{2-(3-amino-5-cyanophenylamino)-5-methylquinazolin-4-yl}piperidin-3-yl]acetamide

*t*-Butanol (1 ml) solution of *(R)*-*N*-{1-(2-chloro-5-methylquinazolin-4-yl)piperidin-3-yl}acetamide (20 mg, 0.06 mmol) prepared in Reference Example 25 and 3,5-diaminobenzonitrile (21 mg, 0.15 mmol) was stirred under reflux overnight. After cooling the reaction solution to room temperature, diisopropylethylamine was added thereto and the reaction solution was concentrated. The resulting residue was purified with silica gel column chromatography (dichloromethane/methanol = 50/1) to prepare the titled compound (7.7 mg) as a pale red solid.
¹H NMR(400MHz, CD₃OD) δ 7.52(s, 1H), 7.48(m, 1H), 7.37(m, 1H), 7.31(s, 1H), 7.05(m, 1H), 6.58(s, 1H), 4.12-3.64(m, 3H), 3.13(m, 1H), 2.90(m, 1H), 2.76(s, 3H), 1.95(m, 2H), 1.82(s, 3H), 1.68-1.51(m, 2H)

### Example 72. (R)-N-(1-[2-{3-amino-5-(trifluoromethyl)phenylamino}-5-methylquinazolin-4-yl]piperidin-3-yl)acetamide

The titled compound was prepared as a yellow oil in the same manner as Step 2 of Example 68 by using *(R)*-*N*-{1-(2-chloro-5-methylquinazolin-4-yl)piperidin-3-yl}acetamide prepared in Reference Example 25 and 3-(trifluoromethyl)-1,5-phenylenediamine.
¹H NMR(400MHz, CD₃OD) δ 7.49(m, 2H), 7.33(m, 1H), 7.23-7.14(m, 1H), 7.06(m, 1H), 4.16-3.62(m, 3H), 3.19-3.05(m, 2H), 2.72(s, 3H), 1.98(m, 2H), 1.75(s, 3H), 1.56(m, 2H); (Yield: 71 %)

### Example 73. (S)-N-[1-{2-(3-amino-5-cyanophenylamino)-8-methylquinazolin-4-yl}pyrrolidin-3-yl]acetamide

*t*-Butanol (0.5 ml) solution of *(S)*-*N*-{1-(2-chloro-8-methylquinazolin-4-yl)pyrrolidin-3-yl}acetamide (20 mg, 0.07 mmol) prepared in Reference Example 27, 3,5-diaminobenzonitrile (17 mg, 0.13 mmol) and diisopropylethylamine (14 µl, 0.08 mmol) was stirred for 1 hour in a microwave (500 W) . After cooling the reaction solution to room temperature, the same was concentrated under reduced pressure. The resulting residue was purified with silica gel column chromatography (dichloromethane/methanol = 30/1) to prepare the titled compound (1.3 mg) as a pale yellow oil.
¹H NMR (400MHz, CD₃OD) δ 7.96(d, 1H), 7.83(s, 1H), 7.49(d, 1H), 7.34(s, 1H), 7.08(t, 1H), 6.56(s, 1H), 4.47(m, 1H), 4.22(m, 1H), 4.11(m, 1H), 4.04(m, 1H), 3.80(m, 1H), 2.58(s, 3H), 2.27(m, 1H), 2.07(m, 1H), 1.95(s, 3H)

### Example 74. (S)-N-(1-[2-{3-amino-5-(trifluoromethyl)phenylamino}-8-methylquinazolin-4-yl]pyrrolidin-3-yl)acetamide

The titled compound was prepared as a pale yellow oil in the same manner as Example 73 by using *(S)-N*-{1-(2-chloro-8-methylquinazolin-4-yl)pyrrolidin-3-yl}acetamide prepared in Reference Example 27 and 3-(trifluoromethyl)-1,5-phenylenediamine.
¹H NMR (400MHz, CD₃OD) δ 7.96(m, 2H), 7.46(d, 1H), 7.15(s, 1H), 7.05(t, 1H), 6.56(s, 1H), 4.47(m, 1H), 4.19(m, 1H), 4.11(m, 1H), 4.01(m, 1H), 3.80(m, 1H), 2.57(s, 3H), 2.24(m, 1H), 2.07(m, 1H), 1.95(s, 3H); (Yield: 9 %)

### Examples 75 and 76

The titled compounds of Examples 75 and 76 were prepared in the same manner as Example 73 by reacting 3,5-diaminobenzonitrile or 3-(trifluoromethyl)-1,5-phenylenediamine respectively with (S)-1-(2-chloro-8-methylquinazolin-4-yl)-N-ethylpyrrolidin-3-amine prepared in Reference Example 28.

### Example 75. (S)-3-amino-5-[4-{3-(ethylamino)pyrrolidin-1-yl}-8-methylquinazolin-2-ylamino]benzonitrile

¹H NMR (400MHz, CD₃OD) δ 8.00(d, 1H), 7.83(s, 1H), 7.47(d, 1H), 7.31(s, 1H), 7.08(t, 1H), 6.56(s, 1H), 4.22-3.98(m, 3H), 3.79(m, 1H), 3.48(m, 1H), 2.79(m, 2H), 2.58(s, 3H), 2.29(m, 1H), 1.97(m, 1H), 1.17(t, 3H); (Yield: 1 %)

### Example 76. (S)-N¹-[4-{3-(ethylamino)pyrrolidin-1-yl}-8-methylquinazolin-2-yl]-5-(trifluoromethyl)benzene-1,3-diamine

¹H NMR(400MHz, CD₃OD) δ 7.99-7.95(m, 2H), 7.46(d, 1H), 7.14(s, 1H), 7.08(t, 1H), 6.56(s, 1H), 4.15-4.11(m, 2H), 3.99(m, 1H), 3.77(m, 1H), 3.48(m, 1H), 2.76(m, 2H), 2.57(s, 3H), 2.28(m, 1H), 1.95(m, 1H) 1.18(t, 3H); (Yield: 3 %)

### Example 77. (R)-N-(1-[2-{3-amino-5-(trifluoromethyl)phenylamino}-8-methylquinazolin-4-yl]piperidin-3-yl)acetamide

The titled compound was prepared as a pale yellow oil in the same manner as Step 2 of Example 68 by using *(R)-N-*{1-(2-chloro-8-methylquinazolin-4-yl)piperidin-3-yl}acetamide prepared in Reference Example 29 and 3-(trifluoromethyl)-1,5-phenylenediamine.
¹H NMR (400MHz, CD₃OD) δ 7.99(s, 1H), 7.72(d, 1H), 7.47(d, 1H), 7.17(s, 1H), 7.11(t, 1H), 6.58(s, 1H), 4.17(d, 1H), 4.08(m, 1H), 3.99(m, 1H), 3.24(m, 1H), 3.05(t, 1H), 2.59(s, 3H), 2.04(m, 1H), 1.94(m, 3H+1H), 1.78(m, 1H), 1.63(m, 1H); (Yield: 25 %)

### Example 78. (R)-N-[1-{2-(3-amino-5-cyanophenylamino)-8-methylquinazolin-4-yl}piperidin-3-yl]acetamide hydrochloride

*t*-Butanol (0.5 ml) solution of *(R)*-*N*-{1-(2-chloro-8-methylquinazolin-4-yl)piperidin-3-yl}acetamide (20 mg, 0.06 mmol) prepared in Reference Example 29 and 3,5-diaminobenzonitrile (10 mg, 0.08 mmol) was stirred for 1 hour in a microwave (300 W). After cooling the reaction solution to room temperature, the same was filtered. The filtrate was washed with dichloromethane and dried *in vacuo* to prepare the titled compound (16.7 mg) as a pale yellow solid.
¹H NMR (400MHz, CD₃OD) δ 8.22(m, 1H), 8.01(s, 1H), 7.72(d, 1H), 7.40(t, 1H), 7.22(brs, NH), 7.11(s, 1H), 6.74(s, 1H), 4.74(m, 1H), 4.55(m, 1H), 4.04(m, 1H), 3.57(m, 1H), 3.41(m, 1H), 2.56(s, 3H), 2.10(m, 2H), 1.94(s, 3H), 1.87(m, 1H), 1.74 (m, 1H)

### Examples 79 and 80

The titled compounds of Examples 79 and 80 were prepared in the same manner as Example 78 by reacting 2,5-diaminobenzonitrile or 2-(trifluoromethyl)-1,4-phenylenediamine respectively with *(R)*-*N*-{1-(2-chloro-8-methylquinazolin-4-yl)piperidin-3-yl}acetamide prepared in Reference Example 29.

### Example 79. (R)-N-[1-{2-(4-amino-3-cyanophenylamino)-8-methylquinazolin-4-yl}piperidin-3-yl]acetamide hydrochloride

¹H NMR(400MHz, CD₃OD) 5 8.17(d, 1H), 8.02(m, 1H), 7.69(m, 1H), 7.62(brs, NH), 7.43-7.37(m, 2H), 6.86(d, 1H), 4.61(m, 1H), 4.49(m, 1H), 3.99(m, 1H), 3.48(m, 1H), 3.36(m, 1H), 2.54(s, 3H), 2.11(m, 1H), 1.98(m, 1H), 1.93(s, 3H), 1.82-1.69(m, 2H); (Yield: 63 %)

### Example 80. (R)-N-(1-[2-{4-amino-3-(trifluoromethyl)phenylamino}-8-methylquinazolin-4-yl]piperidin-3-yl)acetamide hydrochloride

¹H NMR (400MHz, CD₃OD) δ 8.17(m, 1H), 8.02(m, 1H), 7.73(brs, NH), 7.67(d, 1H), 7.35(m, 2H), 6.88(d, 1H), 4.60-4.51(m, 2H), 4.00(m, 1H), 3.48(m, 1H), 3.36(m, 1H), 2.54(s, 3H), 2.11(m, 1H), 1.98(m, 1H), 1.92(s, 3H), 1.82-1.69(m, 2H); (Yield: 55 %)

### Example 81. (S)-N-[1-{2-(3-amino-5-cyanophenylamino)-7-chloroquinazolin-4-yl}pyrrolidin-3-yl]acetamide hydrochloride

### <Step 1> (S)-N-{1-(2,7-dichloroquinazolin-4-yl)pyrrolidin-3-yl}acetamide

The titled compound was prepared as a white solid in the same manner as Step 3 of Reference Example 13 by using 2,4,7-trichloroquinazoline prepared in Reference Example 30 and *(S)*-3-acetamidopyrrolidine.
¹H NMR(400MHz, CDCl₃) δ 7.98(d, 1H), 7.64(s, 1H), 7.33(d, 1H), 6.40(m, 1H), 4.68(m, 1H), 4.19-3.88(m, 4H), 2.32(m, 1H), 2.15(m, 1H), 2.04(s, 3H); (Yield: 96 %)

### <Step 2> (S)-N-[1-{2-(3-amino-5-cyanophenylamino)-7-chloroquinazolin-4-yl}pyrrolidin-3-yl]acetamide hydrochloride

The titled compound was prepared as a white solid in the same manner as Example 30 by using *(S)-N*-{1-(2,7-dichloroquinazolin-4-yl)pyrrolidin-3-yl}acetamide prepared in Step 1.
¹H NMR(400MHz, CD₃OD) δ 8.29(d, 1H), 7.60(s, 1H), 7.47(d, 1H), 7.14(s, 1H), 7.08(s, 1H), 6.81(s, 1H), 4.52(m, 1H), 4.33-4.18(m, 3H), 3.96(m, 1H), 2.36(m, 1H), 2.16(m, 1H), 1.96(s, 3H); (Yield: 69 %)

### Examples 82 and 83

The titled compounds of Examples 82 and 83 were prepared in the same manner as Example 30 by reacting 3,5-diaminobenzonitrile or 3-(trifluoromethyl)-1,5-phenylenediamine respectively with *(S)*-1-(2,7-dichloroquinazolin-4-yl)-*N*-methylpyrrolidin-3-amine prepared in Reference Example 31.

### Example 82. (S)-3-amino-5-[7-chloro-4-{3-(methylamino)pyrrolidin-1-yl}quinazolin-2-ylamino] benzonitrile hydrochloride

¹H NMR(400MHz, CD₃OD+DMSO-d₆) δ 8.29(d, 1H), 7.78-7.66(d, 1H), 7.48(m, 1H), 7.22-7.08(d, 2H), 6.39(s, 1H), 4.40-3.85(m, 5H), 2.82(s, 3H), 2.61(m, 1H), 2.40(m, 1H); (Yield: 69 %)

### Example 83. (S)-N¹- [7-chloro-4-{3-(methylamino) pyrrolidin-1-yl}quinazolin-2-yl]-5-(trifluoromethyl)benzene-1,3-diamine hydrochloride

¹H NMR (400MHz, CD₃OD+DMSO-d₆) δ 8.29(d, 1H), 7.81-7.68(d, 1H), 7.49(d, 1H), 7.19(s, 1H), 7.04(s, 1H), 6.80(s, 1H), 4.35-4.02(m, 5H), 2.79(s, 3H), 2.60(m, 1H), 2.39(m, 1H); (Yield: 66 %)

### Example 84. (S)-3-amino-5-[7-chloro-4-{3-(ethylamino)pyrrolidin-1-yl}quinazolin-2-ylamino] benzonitrile hydrochloride

### <Step 1> (S)-1-(2,7-dichloroquinazolin-4-yl)-N-ethylpyrrolidin-3-amine

The titled compound was prepared as a white solid in the same manner as Step 3 of Reference Example 13 by using 2,4,7-trichloroquinazoline prepared in Reference Example 30 and (3*S*)-(-)-3-(ethylamino)pyrrolidine.
¹H NMR (400MHz, CD₃OD) 5 8.29(d, 1H), 7.67(s, 1H), 7.51(d, 1H), 4.32-4.05(m, 5H), 3.23(q, 2H), 2.59(m, 1H), 2.35(m, 1H), 1.37(t, 3H); (Yield: 71 %)

### <Step 2> (S)-3-amino-5-[7-chloro-4-{3-(ethylamino)pyrrolidin-1-yl}quinazolin-2-ylamino] benzonitrile hydrochloride

The titled compound was prepared as a white solid in the same manner as Example 30 by using *(S)*-1-(2,7-dichloroquinazolin-4-yl)-*N*-ethylpyrrolidin-3-amine prepared in Step 1 and 3,5-diaminobenzonitrile.
¹H NMR (400MHz, CD₃OD+DMSO-d₆) δ 8.25(d, 1H), 7.78-7.66(d, 1H), 7.48(m, 1H), 7.26(s, 1H), 7.11(s, 1H), 6.78(s, 1H), 4.36-4.07(m, 5H), 3.29(m, 2H), 2.61(m, 1H), 2.37(m, 1H), 1.38(m, 3H); (Yield: 42 %)

### Examples 85 and 86

The titled compounds of Examples 85 and 86 were prepared in the same manner as Example 30 by reacting 3,5-diaminobenzonitrile or 3-(trifluoromethyl)-1,5-phenylenediamine respectively with *(S)-N*-{1-(2-chloro-7-fluoroquinazolin-4-yl)pyrrolidin-3-yl}acetamide prepared in Reference Example 32.

### Example 85. (S)-N-[1-{2-(3-amino-5-cyanophenylamino)-7-fluoroquinazolin-4-yl}pyrrolidin-3-yl]acetamide hydrochloride

¹H NMR(400MHz, CD₃OD) δ 8.38 (m, 1H), 7.30(m, 2H), 7. 15 (s, 1H), 7.09(s, 1H), 6.81(s, 1H), 4.52(m, 1H), 4.33-4.18(m, 3H), 3.94(m, 1H), 2.35(m, 1H), 2.15(m, 1H), 1.96(s, 3H); (Yield: 71 %)

### Example 86. (S)-N-(1-[2-{3-amino-5-(trifluoromethyl)phenylamino}-7-fluoroquinazolin-4-yl]pyrrolidin-3-yl)acetamide hydrochloride

¹H NMR (400MHz, CD₃OD) δ 8.37(m, 1H), 7.30-7.26(m, 2H), 7.20(s, 1H), 7.01(s, 1H), 6.81(s, 1H), 4.52(m, 1H), 4.31-4.17(m, 3H), 3.94(m, 1H), 2.37 (m, 1H), 2.14(m, 1H), 1.95(s, 3H); (Yield:71 %)

### Example 87. (S)-N-[1-{2-(3-cyano-4-methylphenylamino)-5,6,7,8-tetrahydroquinazolin-4-yl}pyrrolidin-3-yl]acetamide

*n*-Butanol (0.3 ml) solution of *(S)-N*-{1-(2-chloro-5,6,7,8-tetrahydroquinazolin-4-yl)pyrrolidin-3-yl}acetamide (30 mg, 0.10 mmol) prepared in Reference Example 34 and 5-amino-2-methylbenzonitrile (16.1 mg, 0.11 mmol) was stirred for 1 hour in a microwave (600 W). After cooling the reaction solution to room temperature, the same was concentrated under reduced pressure. The resulting residue was purified with silica gel column chromatography (dichloromethane/methanol = 60/1) to prepare the titled compound (13.7 mg) as a white solid.
¹H NMR (400MHz, CDCl₃) δ 8.18(s, 1H), 7.44 (m, 1H), 7.15(t, 1H), 7.02(s, 1H), 5.89(s, 1H), 4.51(m, 1H), 3.96(m, 1H), 3.79(m, 1H), 3.73(m, 2H), 3.56(m, 1H), 2.66(s, 4H), 2.45(s, 3H), 2.21(m, 1H), 1.95(m, 4H), 1.73(m, 4H)

### Examples 88 to 91

The titled compounds of Examples 88 to 91 were prepared in the same manner as Example 87 by reacting 3,5-diaminobenzonitrile, 3-(trifluoromethyl)-1,5-phenylenediamine, 4-chloro-1,3-diaminobenzene or 4-methyl-3-(trifluoromethyl)aniline respectively with *(S)-N*-{1-(2-chloro-5,6,7,8-tetrahydroquinazolin-4-yl)pyrrolidin-3-yl}acetamide prepared in Reference Example 34.

### Example 88. (S)-N-[1-{2-(3-amino-5-cyanophenylamino)-5,6,7,8-tetrahydroquinazolin-4-yl}pyrrolidin-3-yl]acetamide

¹H NMR(400MHz, CDCl₃) δ 7.43 (s, 1H), 7.11(s, 1H), 6.88(s, 1H), 6.48(s, 1H), 5.81(s, 1H), 4.48(m, 1H), 4.00-3.59(m, 4H+2NH), 2.65(m, 4H), 2.24(m, 1H), 2.20(s, 3H), 1.94(m, 1H), 1.77(m, 4H); (Yield: 12 %)

### Example 89. (S)-N-(1-[2-{3-amino-5-(trifluoromethyl)phenylamino}-5,6,7,8-tetrahydroquinazolin-4-yl]pyrrolidin-3-yl)acetamide

¹H NMR(400MHz, CDCl₃) δ 7.70 (brs, 1H), 7.47 (s, 1H), 6.97(s, 1H), 6.57-6.49(brs+s, 2H), 4.52(m, 1H), 4.00-3.92(m, 4H), 3.75 (m, 2H), 2.59 (m, 4H), 2.21(m, 1H), 2.04(s, 3H), 2.00(m, 1H), 1.72(m, 4H); (Yield: 29 %)

### Example 90. (S)-N-[1-{2-(3-amino-4-chlorophenylamino)-5,6,7,8-tetrahydroquinazolin-4-yl}pyrrolidin-3-yl]acetamide

¹H NMR(400MHz, CDCl₃) δ 8.92(brs, 1H), 7.87(s, 1H), 7.28(m, 1H), 7.04(m, 1H), 6.68(m, 1H), 4.52(m, 1H), 4.43(s, 2H), 4.14(m, 1H), 3.94(m, 1H), 3.88(m, 1H), 3.76(m, 1H), 2.54(m, 4H), 2.26(m, 1H), 2.13(m, 4H), 1.75(m, 4H); (Yield: 86 %)

### Example 91. (S)-N-(1-[2-{4-methyl-3-(trifluoromethyl)phenylamino}-5,6,7,8-tetrahydroquinazolin-4-yl]pyrrolidin-3-yl)acetamide

¹H NMR (400MHz, CDCl₃) δ 8.31 (s, 1H), 7.27(s, 1H), 7.12(m, 1H), 6.97(s, 1H), 5.90(m, 1H), 4.52(m, 1H), 3.94(m, 1H), 3.80-3.73(m, 2H), 3.55(m, 1H), 2.64(m, 4H), 2.40(s, 3H), 2.19(m, 1H), 1.93(m, 4H), 1.72(m, 4H); (Yield: 33 %)

### Example 92. (S)-N-[1-{2-(4-amino-3-nitrophenylamino)-5,6,7,8-tetrahydroquinazolin-4-yl}pyrrolidin-3-yl]acetamide hydrochloride

*n*-Butanol (0.3 ml) solution of *(S)-N-*{1-(2-chloro-5,6,7,8-tetrahydroquinazolin-4-yl)pyrrolidin-3-yl}acetamide (30 mg, 0.10 mmol) prepared in Reference Example 34 and 2-nitro-1,4-phenylenediamine (18.7 mg, 0.11 mmol) was stirred for 1 hour in a microwave (600 W). After cooling the reaction solution to room temperature, the resulting solid was washed with dichloromethane, filtered and dried *in vacuo* to prepare the titled compound (40.5 mg) as a red solid.
¹H NMR (400MHz, CD₃OD) δ 8.50(s, 1H), 7.38(d, 1H), 6.98(d, 1H), 4.39(m, 1H), 4.11-3.96(m, 3H), 3.72(m, 1H), 2.79(m, 2H), 2.66 (m, 2H), 2.24 (m, 1H), 1.95 (m, 3H+1H), 1.95-1.84(m, 4H)

### Example 93. (S)-N-[1-{2-(3-amino-5-chlorophenylamino)-5,6,7,8-tetrahydroquinazolin-4-yl}pyrrolidin-3-yl]acetamide hydrochloride

The titled compound was prepared as a white solid in the same manner as Example 92 by using *(S)-N-*{1-(2-chloro-5,6,7,8-tetrahydroquinazolin-4-yl)pyrrolidin-3-yl}acetamide prepared in Reference Example 34 and 5-chloro-1,3-phenylenediamine.
¹H NMR(400MHz, CD₃OD) δ 6.89(s, 1H), 6.74(s, 1H), 6.48(s, 1H), 4.41(m, 1H), 4.11-3.96(m, 3H), 3.74(m, 1H), 2.79(m, 2H), 2.66(m, 2H), 2.29(m, 1H), 1.96(m, 1H+3H), 1.83(m, 4H); (Yield: 85 %)

### Examples 94 and 95

The titled compounds of Examples 94 and 95 were prepared in the same manner as Example 31 by reacting *(S)-N*-[1-{2-(3-amino-5-cyanophenylamino)-5,6,7,8-tetrahydroquinazolin-4-yl}pyrrolidin-3-yl]acetamide prepared in Example 88 or *(S)*-*N*-(1-[2-{3-amino-5-(trifluoromethyl)phenylamino}-5,6,7,8-tetrahydroquinazolin-4-yl]pyrrolidin-3-yl)acetamide prepared in Example 89.

### Example 94. (S)-N-[1-{2-(3-amino-5-cyanophenylamino)-5,6,7,8-tetrahydroquinazolin-4-yl}pyrrolidin-3-yl]acetamide hydrochloride

¹H NMR(400MHz, CD₃OD) δ 7. 94 (s, 2H), 7.40 (m, 1H), 4. 41 (m, 1H), 4.11-3.75(m, 4H), 2.85(m, 2H), 2.73(m, 2H), 2.26(m, 1H), 2.01(m, 4H), 1.86(m, 4H); (Yield: 90 %)

### Example 95. (S)-N-(1-[2-{3-amino-5-(trifluoromethyl)phenylamino}-5,6,7,8-tetrahydroquinazolin-4-yl]pyrrolidin-3-yl)acetamide hydrochloride

¹H NMR(400MHz, CD₃OD) δ 8.12(m, 1H), 7.87 (s, 1H), 7.40(s, 1H), 4.41-3.73(m, 5H), 2.85(m, 2H), 2.74(m, 2H), 2.25(m, 1H), 2.01(m, 4H), 1.82(m, 4H); (Yield: 90 %)

### Example 96. (S)-3-amino-5-{4-(3-aminopyrrolidin-1-yl)-5,6,7,8-tetrahydroquinazolin-2-ylamino}benzonitrile dihydrochloride

*n*-Butanol (1 ml) solution of *(S*)*-tert*-butyl 1- (2-chloro-5,6,7,8-tetrahydroquinazolin-4-yl)pyrrolidin-3-ylcarbamate (40 mg, 0.11 mmol) prepared in Reference Example 35 and 3,5-diaminobenzonitrile (18.1 mg, 0.14 mmol) was stirred for 1.5 hours in a microwave (450 W). After cooling the reaction solution to room temperature, the same was concentrated under reduced pressure. The resulting residue was purified with silica gel column chromatography (dichloromethane/methanol = 60/1), dissolved in ethyl acetate (1 ml), and then hydrochloric acid gas was added thereto. The resulting white solid was filtered to prepare the titled compound (2.5 mg).
¹H NMR (400MHz, CD₃OD) δ 7.46-7.17(m, 2H), 6.95-6.85(m, 1H), 4.15-4.03(m, 5H), 2.84-2.73(m, 4H), 2.46(m, 1H), 2.22(m, 1H), 1.79(m, 4H)

### Examples 97 and 98

The titled compounds of Examples 97 and 98 were prepared in the same manner as Example 96 by reacting 3-(trifluoromethyl)-1,5-phenylenediamine or 2-(trifluoromethyl)-1,4-phenylenediamine respectively with *(S)*-*tert*-butyl 1-(2-chloro-5,6,7,8-tetrahydroquinazolin-4-yl)pyrrolidin-3-ylcarbamate prepared in Reference Example 35.

### Example 97. (S)-N¹-{4-(3-aminopyrrolidin-1-yl)-5,6,7,8-tetrahydroquinazolin-2-yl}-5-(trifluoromethyl)benzene-1,3-diamine dihydrochloride

¹H NMR(400MHz, CD₃OD) δ 7. 91-7. 72 (m, 1H), 7.25(m, 1H), 6.81(m, 1H), 4.15-4.05(m, 5H), 2.86-2.76(m, 4H), 2.45(m, 1H), 2.21(m, 1H), 1.79(m, 4H); (Yield: 24 %)

### Example 98. (S)-N¹-{4-(3-aminopyrrolidin-1-yl)-5,6,7,8-tetrahydroquinazolin-2-yl}-3-(trifluoromethyl)benzene-1,4-diamine dihydrochloride

¹H NMR(400MHz, CD₃OD) δ 7.68(m, 1H), 7.43(m, 1H), 6. 9 (m, 1H), 4.09-4.00(m, 5H), 2.82-2.70(m, 4H), 2.42(m, 1H), 2.18(m, 1H), 1.77(m, 4H); (Yield: 21 %)

### Example 99. (S)-3-amino-5-[4-{3-(methylamino)pyrrolidin-1-yl}-5,6,7,8-tetrahydroquinazolin-2-ylamino]benzonitrile dihydrochloride

A mixture of *(S*)*-tert*-butyl 1-(2-chloro-5,6,7,8-tetrahydroquinazolin-4-yl)pyrrolidin-3-yl(methyl)carbamate (50 mg, 0.14 mmol) prepared in Reference Example 36, 3,5-diaminobenzonitrile (21.8mg, 0.16mmol), palladium acetate (0.6 mg, 0.003 mmol), 4,5-bis(diphenylphosphino)-9,9-dimethylxanthine (2.4 mg, 0.005 mmol), cesium carbonate (90.0 mg, 0.24 mmol) and anhydrous 1,4-dioxane (0.7 ml) was stirred at 130 °C for 3 hours. After cooling the reaction solution to room temperature, the same was filtered by celite and the filtrate was concentrated under reduced pressure. The resulting residue was purified with silica gel column chromatography (dichloromethane/methanol = 100/1), dissolved in ethyl acetate (1 ml), and then hydrochloric acid gas was added thereto. The resulting white solid was filtered to prepare the titled compound (10.6 mg).
¹H NMR(400MHz, CD₃OD) δ 7.85(s, 1H), 7.69(m, 1H), 7.27(m, 1H), 4.25-3.96(m, 5H), 2.86-2.82(m, 5H), 2.75(m, 2H), 2.49(m, 1H), 2.31(m, 1H), 1.82(m, 4H)

### Example 100. (S)-N¹-[4-{3-(methylamino)pyrrolidin-1-yl}-5,6,7,8-tetrahydroquinazolin-2-yl]-5-(trifluoromethyl)benzene-1,3-diamine dihydrochloride

The titled compound was prepared as a white solid in the same manner as Example 99 by using *(S)*-*tert*-butyl 1-(2-chloro-5,6,7,8-tetrahydroquinazolin-4-yl)pyrrolidin-3-yl(methyl)carbamate prepared in Reference Example 36 and 3-(trifluoromethyl)-1,5-phenylenediamine.
¹H NMR(400MHz, CD₃OD) δ 7.93(s, 1H), 7.80(m, 1H), 7.31(m, 1H), 4.11-3.97(m, 5H), 2.87-2.80(m, 5H), 2.76(m, 2H), 2.48(m, 1H), 2.32(m, 1H), 1.60(m, 4H); (Yield: 30 %)

### Example 101. (S)-N¹-[4-{3-(methylamino)pyrrolidin-1-yl}-5,6,7,8-tetrahydroquinazolin-2-yl]-3-(trifluoromethyl)benzene-1,4-diamine dihydrochloride

The titled compound was prepared as a white solid in the same manner as Example 96 by using *(S)*-*tert*-butyl 1-(2-chloro-5,6,7,8-tetrahydroquinazolin-4-yl)pyrrolidin-3-yl(methyl)carbamate prepared in Reference Example 36 and 2-(trifluoromethyl)-1,4-phenylenediamine.
¹H NMR(400MHz, CD₃OD) δ 7.75(s, 1H), 7.48(s, 1H), 7.07(m, 1H), 4.14-3.92(m, 5H), 2.83-2.78(m, 5H), 2.70(m, 2H), 2.44(m, 1H), 2.26(m, 1H), 1.84(m, 4H); (Yield: 32 %)

### Examples 102 to 105

The titled compounds of Examples 102 to 105 were prepared in the same manner as Example 96 by reacting 3-(trifluoromethyl)-1,5-phenylenediamine, 2-(trifluoromethyl)-1,4-phenylenediamine, 4-chloro-1,3-diaminobenzene or 3,5-diaminobenzonitrile respectively with *(S)*-*tert*-butyl 1-(2-chloro-5,6,7,8-tetrahydroquinazolin-4-yl)pyrrolidin-3-yl(ethyl)carbamate prepared in Reference Example 37.

### Example 102. (S)-N¹-[4-{3-(ethylamino)pyrrolidin-1-yl}-5,6,7,8-tetrahydroquinazolin-2-yl]-5-(trifluoromethyl)benzene-1,3-diamine dihydrochloride

¹H NMR(400MHz, CD₃OD) δ 7. 92 (m, 2H), 7. 37 (m, 1H), 4.23-4.00(m, 5H), 3.18(m, 2H), 2.87-2.76(m, 4H), 2.48(m, 1H), 2.30(m, 1H), 1.86(m, 4H), 1.38(m, 3H); (Yield: 21 %)

### Example 103. (S)-N¹-[4-{3-(ethylamino)pyrrolidin-1-yl}-5,6,7,8-tetrahydroquinazolin-2-yl]-3-(trifluoromethyl)benzene-1,4-diamine dihydrochloride

¹H NMR (400MHz, CD₃OD) δ 7.93(m, 1H), 7.67(m, 1H), 7.30(m, 1H), 4.17-3.99(m, 5H), 3.16(m, 2H), 2.85-2.72(m, 4H), 2.46(m, 1H), 2.28(m, 1H), 1.82(m, 4H), 1.38(m, 3H); (Yield: 47 %)

### Example 104. (S)-4-chloro-N¹- [4-{3-(ethylamino)pyrrolidin-1-yl}-5,6,7,8-tetrahydroquinazolin-2-yl]benzene-1,3-diamine dihydrochloride

¹H NMR(400MHz, CD₃OD) 5 7.62-7.20(m, 3H), 4.22-3.98(m, 5H), 3.18(m, 2H), 2.84-2.72(m, 4H), 2.46(m, 1H), 2.28(m, 1H), 1.81(m, 4H), 1.38(m, 3H); (Yield: 51 %)

### Example 105. (S)-3-amino-5-[4-{3-(ethylamino)pyrrolidin-1-yl}-5,6,7,8-tetrahydroquinazolin-2-ylamino]benzonitrile dihydrochloride

¹H NMR(400MHz, CD₃OD) δ 7.76 (s, 1H), 7.59 (s, 1H), 7.19(m, 1H), 4.26-4.00(m, 5H), 3.20(m, 2H), 2.86-2.75(m, 4H), 2.49(m, 1H), 2.29(m, 1H), 1.86(m, 4H), 1.38(m, 3H); (Yield: 31 %)

### Example 106. (S)-N¹-[4-{3-(propylamino)pyrrolidin-1-yl}-5,6,7,8-tetrahydroquinazolin-2-yl]-5-(trifluoromethyl)benzene-1,3-diamine

Propionaldehyde (19.6 µl, 0.273 mmol) was added into methanol (1.5 ml) solution of *(S)*-*N*¹{4-(3-aminopyrrolidin-1-yl)-5,6,7,8-tetrahydroquinazolin-2-yl}-5-(trifluoromethyl)benzene-1,3-diamine (107 mg, 0.273 mmol) prepared by treating *(S)*-*N*¹-{4-(3-aminopyrrolidin-1-yl)-5,6,7,8-tetrahydroquinazolin-2-yl}-5-(trifluoromethyl)benzene-1,3-diamine dihydrochloride prepared in Example 97 with 2.0 N sodium hydroxide aqueous solution, and then they were stirred at room temperature for 1 hour and sodium triacetoxyborohydride (115.6 mg, 0.545 mmol) was added thereto. The reaction solution was stirred at room temperature overnight, and then water was added to terminate the reaction. The reaction mixture was extracted by adding chloroform, and the extract was washed with saturated sodium bicarbonate aqueous solution, dried by anhydrous magnesium sulfate and filtered. The solution was concentrated. The resulting residue was purified with silica gel column chromatography (ethyl acetate/methanol = 100/1) to prepare the titled compound (7.5 mg) as a colorless oil.
¹H NMR (400MHz, CD₃OD) δ 7.57(s, 1H), 7.03(s, 1H), 6.52(s, 1H), 3.89(m, 2H), 3.72(m, 1H), 3.51(m, 1H), 3.33(m, 1H), 2.73(m, 2H), 2.60(m, 4H), 2.18(m, 1H), 1.80(m, 4H), 1.65(m, 1H), 1.55(m, 2H), 0.95(m, 3H)

### Example 107. (R)-N¹-{4-(3-aminopiperidin-1-yl)-5,6,7,8-tetrahydroquinazolin-2-yl}-5-(trifluoromethyl)benzene-1,3-diamine dihydrochloride

### <Step 1> (R)-tert-butyl 1-(2-chloro-5,6,7,8-tetrahydroquinazolin-4-yl)piperidin-3-ylcarbamate

The titled compound was prepared as a white solid in the same manner as Reference Example 36 by using 2,4-dichloro-5,6,7,8-tetrahydroquinazoline prepared in Reference Example 33 and *(R)*-(-)-3-aminopiperidine dihydrochloride. This compound was used in the subsequent reaction without further purification.

### <Step 2> (R)-tert-butyl 1-[2-{3-amino-5-(trifluoromethyl)phenylamino}-5,6,7,8-tetrahydroquinazolin-4-yl]piperidin-3-ylcarbamate

The titled compound (441 mg) was prepared as a pale yellow oil in the same manner as Step 2 of Example 59 by using *(R)*-*tert*-butyl 1-(2-chloro-5,6,7,8-tetrahydroquinazolin-4-yl)piperidin-3-ylcarbamate prepared in Step 1 and 3-(trifluoromethyl)-1,5-phenylenediamine.
¹H NMR (400MHz, CDCl₃) δ 7.55 (s, 1H), 7.08(s, 1H), 6.97 (s, 1H), 6.50(s, 1H), 4.96(m, 1H), 3.82(s, 2H), 3.56(m, 1H), 3.37-3.26(m, 3H), 2.71(m, 2H), 2.50(m, 2H), 2.02(m, 1H), 1.84(m, 4H), 1.70-1.42(m, 4H), 1.42(s, 9H); (Yield: 40 %)

### <Step 3> (R)-N¹-{4-(3-aminopiperidin-1-yl)-5,6,7,8-tetrahydroquinazolin-2-yl}-5-(trifluoromethyl)benzene-1,3-diamine dihydrochloride

The titled compound was prepared as a white solid in the same manner as Example 31 by using *(R)*-*tert*-butyl 1-[2-{3-amino-5-(trifluoromethyl)phenylamino}-5,6,7,8-tetrahydroquinazolin-4-yl]piperidin-3-ylcarbamate prepared in Step 2.
¹H NMR (400MHz, CD₃OD) δ 8.02(s, 1H), 7.90(s, 1H), 4.42(m, 1H), 4.12(m, 1H), 3.61-3.31(m, 3H), 2.84(m, 2H), 2.70(m, 2H), 2.22(m, 1H), 1.99(m, 3H), 1.82(m, 4H); (Yield: 90 %)

### Examples 108 to 117

The titled compounds of Examples 108 to 117 were prepared in the same manner as Example 87 by reacting 3-aminobenzonitrile, 5-amino-2-methylbenzonitrile, 5-amino-2-fluorobenzonitrile, 3,5-diaminobenzonitrile, 3-(trifluoromethyl)-1,5-phenylenediamine, 2-(trifluoromethyl)-1,4-phenylenediamine, 4-fluoro-3-trifluoromethylphenylamine, 2-nitro-1,4-phenylenediamine, 4-chloro-1,3-diaminobenzene or 5-chloro-1,3-diaminobenzene respectively with *(R)*-*N*-{1-(2-chloro-5,6,7,8-tetrahydroquinazolin-4-yl)piperidin-3-yl}acetamide prepared in Reference Example 38.

### Example 108. (R)-N-[1-{2-(3-cyanophenylamino)-5,6,7,8-tetrahydroquinazolin-4-yl}piperidin-3-yl]acetamide

¹H NMR (400MHz, CDCl₃) δ 8.37(s, 1H), 7.50(m, 1H), 7.36(m, 1H), 7.20(m, 1H), 6.97(s, 1H), 5.93(m, 1H), 4.08(m, 1H), 3.67(m, 1H), 3.45(m, 1H), 3.16(m, 2H), 2.73(m, 2H), 2.51(m, 2H), 1.90 (s, 3H), 1.85-1.74(m, 8H); (Yield: 30 %)

### Example 109. (R)-N-[1-{2-(3-cyano-4-methylphenylamino)-5,6,7,8-tetrahydroquinazolin-4-yl}piperidin-3-yl]acetamide

¹H NMR (400MHz, CDCl₃) δ 8.28(s, 1H), 7.38(d, 1H), 7.17(d, 1H), 6.89(s, 1H), 6.01(m, 1H), 4.07(m, 1H), 3.67(d, 1H), 3.43(m, 1H), 3.17(m, 2H), 2.71(m, 2H), 2.49(m, 2H+3H), 1.94(s, 3H), 1.94-1.63(m, 8H); (Yield: 28 %)

### Example 110. (R)-N-[1-{2-(3-cyano-4-fluorophenylamino)-5,6,7,8-tetrahydroquinazolin-4-yl}piperidin-3-yl]acetamide

¹H NMR(400MHz, CDCl₃) δ 8.33(m, 1H), 7.47(m, 1H), 7.09(m, 1H), 6.96(s, 1H), 5.85(m, 1H), 4.08(m, 1H), 3.72(m, 1H), 3.46(m, 1H), 3.13-3.05(m, 2H), 2.72(m, 2H), 2.51(m, 2H), 1.95(s, 3H), 1.84-1.58(m, 8H); (Yield: 29 %)

### Example 111. (R)-N-[1-{2-(3-amino-5-cyanophenylamino)-5,6,7,8-tetrahydroquinazolin-4-yl}piperidin-3-yl]acetamide

¹H NMR(400MHz, CDCl₃) δ 7.45(s, 1H), 7.06(s, 1H), 6.88(s, 1H), 6.50 (s, 1H), 6.03(m, 1H), 4.07(m, 1H), 3.91(s, 2H), 3.73(m, 1H), 3.46(m, 1H), 3.18(m, 2H), 2.71(m, 2H), 2.50(m, 2H), 1.91 (s, 3H), 1.85-1.64(m, 8H); (Yield: 23 %)

### Example 112. (R)-N-(1-[2-{3-amino-5-(trifluoromethyl)phenylamino}-5,6,7,8-tetrahydroquinazolin-4-yl]piperidin-3-yl)acetamide

¹H NMR(400MHz, CDCl₃) δ 7.52(s, 1H), 6.94(s, 1H), 6.88(s, 1H), 6.51(s, 1H), 6.21(m, 1H), 4.05(m, 1H), 3.86(s, 2H), 3.62(m, 1H), 3.40-3.29(m, 3H), 2.72(m, 2H), 2.49(m, 2H), 1.90-1.68(m, 3H+8H); (Yield: 30 %)

### Example 113. (R)-N-(1-[2-{4-amino-3-(trifluoromethyl)phenylamino}-5,6,7,8-tetrahydroquinazolin-4-yl]piperidin-3-yl)acetamide

¹H NMR(400MHz, CDCl₃) δ 7.99(s, 1H), 7.19(d, 1H), 6.80 (s, 1H), 6. 68 (d, 1H), 6.43(m, 1H), 4.02(m, 1H+2H), 3.48(m, 2H), 3.33(m, 2H), 2.68(m, 2H), 2.47(m, 2H), 1.89-1.60(m, 3H+8H); (Yield: 37 %)

### Example 114. (R)-N-(1-[2-{4-fluoro-3-(trifluoromethyl)phenylamino}-5,6,7,8-tetrahydroquinazolin-4-yl]piperidin-3-yl)acetamide

¹H NMR(400MHz, CDCl₃) δ 8.31(m, 1H), 7.38(m, 1H), 7.11(m, 1H), 6.89(s, 1H), 5.94(m, 1H), 4.08(m, 1H), 3.61(m, 1H), 3.49(m, 1H), 3. 18 (m, 2H), 2.71(m, 2H), 2.50(m, 2H), 1.89(s, 3H), 1.78-1.68(m, 8H); (Yield: 29 %)

### Example 115. (R)-N-[1-{2-(4-amino-3-nitrophenylamino)-5,6,7,8-tetrahydroquinazolin-4-yl}piperidin-3-yl]acetamide

¹H NMR(400MHz, CDCl₃) δ 8.91 (s, 1H), 7.23(m, 1H), 6.74(d, 2H), 6.14(m, 1H), 5.89(s, 2H), 4.07(m, 1H), 3.72(m, 1H), 3.32(m, 1H), 3.29(m, 1H), 3.18(m, 1H), 2.68(m, 2H), 2.48(m, 2H), 1.93 (s, 3H), 1.89-1.63(m, 8H); (Yield: 29 %)

### Example 116. (R)-N-[1-{2-(3-amino-4-chlorophenylamino)-5,6,7,8-tetrahydroquinazolin-4-yl}piperidin-3-yl]acetamide

¹H NMR (400MHz, CDCl₃) δ 7.24(d, 1H), 7.09(d, 1H), 6.83 (s, 1H), 6.73(d, 1H), 6.39(m, 1H), 4.08(m, 1H+2H), 3.62(m, 1H), 3.40-3.34(m, 3H), 2.70(t, 2H), 2.49(m, 2H), 1.86-1.63(m, 3H+8H); (Yield: 35 %)

### Example 117. (R)-N-[1-{2-(3-amino-5-chlorophenylamino)-5,6,7,8-tetrahydroquinazolin-4-yl}piperidin-3-yl]acetamide

¹H NMR(400MHz, CDCl₃) δ 7.12(s, 1H), 6.95(s, 1H), 6.76(s, 1H), 6.36(m, 1H), 6.29(s, 1H), 4.07(m, 1H), 3.75(s, 2H), 3.58(m, 1H), 3.43-3.36(m, 3H), 2.70(t, 2H), 2.49(m, 2H), 1.88-1.74(m, 3H+8H); (Yield: 30 %)

### Example 118. (R)-N-(1-[2-{4-amino-3-(trifluoromethyl)phenylamino}-5,6,7,8-tetrahydroauinazolin-4-yl]piperidin-3-yl)acetamide hydrochloride

The titled compound was prepared as a white solid in the same manner as Example 31 by using *(R)-N-*(1-[2-{4-amino-3-(trifluoromethyl)phenylamino}-5,6,7,8-tetrahydroquinazolin-4-yl]piperidin-3-yl)acetamide prepared in Example 113.
¹H NMR(400MHz, CD₃OD) δ 7.77(s, 1H), 7.48(m, 1H), 7.10(m, 1H), 4.31(m, 1H), 4.11(m, 1H), 3.82(m, 1H), 3.22(m, 2H), 2.74(m, 2H), 2.59(m, 2H), 1.87(s, 3H), 1.87-1.61(m, 8H); (Yield: 95 %)

### Example 119. (R)-N-[1-{2-(3-amino-5-cyanophenylamino)-5,6,7,8-tetrahydroauinazolin-4-yl}piperidin-3-yl]acetamide hydrochloride

The titled compound was prepared as a white solid in the same manner as Example 99 by using *(R)*-*N*-{1-(2-chloro-5,6,7,8-tetrahydroquinazolin-4-yl)piperidin-3-yl}acetamide prepared in Reference Example 38.
¹H NMR(400MHz, CD₃OD) δ 7.97(s, 1H), 7.90(s, 1H), 7.46(s, 1H), 4.57(m, 1H), 4.16(m, 1H), 3.86(m, 1H), 3.43(m, 1H), 3.22(m, 1H), 2.79 (m, 2H), 2.65 (m, 2H), 2.05-1.68(m, 3H+8H); (Yield: 29 %)

### Example 120. (S)-1-{2-(3-amino-5-cyanophenylamino)-5,6,7,8-tetrahydroquinazolin-4-yl}-N-methylpyrrolidine-3-carboxamide

### <Step 1> (S)-1-(2-chloro-5,6,7,8-tetrahydroquinazolin-4-yl)-N-methylpyrrolidine-3-carboxamide

Diisopropylethylamine (3.4 ml, 19.7 mmol) was added into chloroform (25 ml) solution of 2,4-dichloro-5,6,7,8-tetrahydroquinazoline (1 g, 4.92 mmol) prepared in Reference Example 33 and *(S)*-(+)-pyrrolidine-3-carboxylic acid (0.62 g, 5.42 mmol), and they were stirred at 60 °C for 2 days. After cooling the reaction solution to room temperature, methylamine hydrochloride (0.33 g, 4.92 mmol), N-(3-dimethylaminopropyl)-N'-ethylcarbodiimide hydrochloride (0.94 g, 4.92 mmol) and 1-hydroxybenzotriazole hydrate (0.67 g, 4.92 mmol) were added thereto, and they were stirred at room temperature overnight. The reaction solution was diluted with dichloromethane, washed with water, dried with anhydrous magnesium sulfate and concentrated under reduced pressure. The resulting residue was crystallized by using ether/ethyl acetate to prepare the titled compound (810 mg) as a pale yellow solid.
¹H NMR (400MHz, CDCl₃) δ 5.65(s, 1H), 3.91-3.68(m, 4H), 2.89(s, 3H), 2.72(m, 4H), 2.16(m, 2H), 1.78-1.43(m, 4H)

### <Step 2> (S)-1-{2-(3-amino-5-cyanophenylamino)-5,6,7,8-tetrahydroquinazolin-4-yl}-N-methylpyrrolidine-3-carboxamide

The titled compound was prepared as a pale yellow oil in the same manner as Example 87 by using (S)-1-(2-chloro-5,6,7,8-tetrahydroquinazolin-4-yl)-*N*-methylpyrrolidine-3-carboxamide prepared in Step 1 and 3,5-diaminobenzonitrile.
¹H NMR(400MHz, CDCl₃) δ 7.50(m, 1H+1H), 7.04 (s, 1H), 6.47(s, 1H), 5.99 (s, 1H), 3.94-3.70(m, 2H+4H), 2.96(m, 1H), 2.85(s, 3H), 2.65(m, 4H), 2.22(m, 2H), 1.73-1.60(m, 4H); (Yield: 20 %)

### Examples 121 to 126

The titled compounds of Examples 121 to 126 were prepared in the same manner as Example 87 by reacting 5-amino-2-methylbenzonitrile, 5-amino-2-fluorobenzonitrile, 3-(trifluoromethyl)-1,5-phenylenediamine, 2-(trifluoromethyl)-1,4-phenylenediamine, 4-fluoro-3-trifluoromethylphenylamine or 4-chloro-1,3-diaminobenzene respectively with *(S)*-1-(2-chloro-5,6,7,8-tetrahydroquinazolin-4-yl)-*N*-methylpiperidine-3-carboxamide prepared in Reference Example 39.

### Example 121. (R)-1-{2-(3-cyano-4-methylphenylamino)-5,6,7,8-tetrahydroquinazolin-4-yl}-N-methylpiperidine-3-carboxamide

¹H NMR(400MHz, CDCl₃) δ 8.07(s, 1H), 7.81(s, 1H), 7.53(d, 1H), 7.18(d, 1H), 6.08(s, 1H), 4.06(d, 1H), 3.85(d, 1H), 3.24 (t, 1H), 3.07 (t, 1H), 2.80(s, 3H), 2.70(m, 2H), 2.47(m, 6H), 1.99(m, 1H), 1.83(m, 4H), 1.68(m, 3H); (Yield: 50 %)

### Example 122. (R)-1-{2-(3-cyano-4-fluorophenylamino)-5,6,7,8-tetrahydroquinazolin-4-yl}-N-methylpiperidine-3-carboxamide

¹H NMR (400MHz, CDCl₃) δ 8.17(s, 1H), 7.60(m, 1H), 7.10(m, 1H), 6.94(s, 1H), 5.82(m, 1H), 3.96(d, 1H), 3.74(d, 1H), 3.16(t, 1H), 2.99(t, 1H), 2.82(s, 3H), 2.72(m, 2H), 2.49(m, 3H), 2.01(m, 1H), 1.83-1.71(m, 7H); (Yield: 17 %)

### Example 123. (R)-1-[2-{3-amino-5-(trifluoromethyl)phenylamino}-5,6,7,8-tetrahydroquinazolin-4-yl]-N-methylpiperidine-3-carboxamide

¹H NMR(400MHz, CDCl₃) δ 7.29(m, 1H), 7.17(s, 1H), 6.90(s, 1H), 6.49(s, 1H), 5.87(s, 1H), 4.08(m, 1H), 3.99(s, 2H), 3.74(d, 1H), 3.20(m, 1H), 2.95(m, 1H), 2.77(s, 3H), 2.70(m, 2H), 2.56(m, 1H), 2.48(m, 2H), 1.97(m, 1H), 1.78-1.61(m, 7H); (Yield: 11 %)

### Example 124. (R)-1-[2-{4-amino-3-(trifluoromethyl)phenylamino}-5,6,7,8-tetrahydroquinazolin-4-yl]-N-methylpiperidine-3-carboxamide

¹H NMR(400MHz, CDCl₃) δ 7. 86 (s, 1H), 7.31(d, 1H), 6.71(m, 2H), 5.98(s, 1H), 3.97(s, 2H), 3.84(d, 1H), 3.64(d, 1H), 3.27(t, 1H), 3.07(t, 1H), 2.73(m, 5H), 2.47(m, 3H), 1.89-1.83(m, 4H), 1.69-1.60(m, 4H); (Yield: 14 %)

### Example 125. (R)-1-[2-{4-fluoro-3-(trifluoromethyl)phenylamino}-5,6,7,8-tetrahydroquinazolin-4-yl]-N-methylpiperidine-3-carboxamide

¹H NMR(400MHz, CDCl₃) δ 8.17(s, 1H), 7.51(m, 1H), 7.09(m, 1H), 6.92(m, 1H), 5.82(m, 1H), 3.92(m, 1H), 3.71(m, 1H), 3.19(m, 1H), 3.01(m, 1H), 2.80-2.71(m, 5H), 2.49(m, 3H), 1.96-1.71(m, 8H); (Yield: 11 %)

### Example 126. (R)-1-{2-(3-amino-4-chlorophenylamino)-5,6,7,8-tetrahydroquinazolin-4-yl}-N-methylpiperidine-3-carboxamide

¹H NMR (400MHz, CDCl₃) δ 7.45 (s, 1H), 7.07 (m, 2H), 6.61(m, 1H), 5.93(m, 1H), 4.25(s, 2H), 4.14(d, 1H), 3.76(d, 1H), 3.17(t, 1H), 3.01(t, 1H), 2.79(s, 3H), 2.69-2.46(m, 3H), 2.46(m, 2H), 1.95-1.53(m, 8H); (Yield: 20 %)

### Examples 127 to 129

The titled compounds of Examples 127 to 129 were prepared in the same manner as Example 92 by reacting 3, 5-diaminobenzonitrile, 2-nitro-1,4-phenylenediamine or 5-chloro-1,3-diaminobenzene respectively with *(S)*-1-(2-chloro-5,6,7,8-tetrahydroquinazolin-4-yl)-*N-*methylpiperidine-3-carboxamide prepared in Reference Example 39.

### Example 127. (R)-1-{2-(3-amino-5-cyanophenylamino)-5,6,7,8-tetrahydroquinazolin-4-yl}-N-methylpiperidine-3-carboxamide hydrochloride

¹H NMR (400MHz, CD₃OD) δ 7.84 (s, 1H), 7.08(s, 1H), 7.02(s, 1H), 6.75(s, 1H), 4.43(d, 1H), 4.25(d, 1H), 3.37(m, 2H), 2.72(m, 5H), 2.53(m, 3H), 2.03(m, 1H), 1.92-1.82(m, 5H), 1.68-1.62(m, 2H); (Yield: 63 %)

### Example 128. (R)-1-{2-(4-amino-3-nitrophenylamino)-5,6,7,8-tetrahydroquinazolin-4-yl}-N-methylpiperidine-3-carboxamide hydrochloride

¹H NMR(400MHz, CD₃OD) δ 8.29 (s, 1H), 7.78 (m, 1H), 7.37 (d, 1H), 7.00(d, 1H), 4.39(d, 1H), 4.21(d, 1H), 3.30(m, 2H), 2.70(m, 5H), 2.57(m, 2H), 2.47(m, 1H), 1.99-1.57(m, 8H); (Yield: 72 %)

### Example 129. (R)-1-{2-(3-amino-5-chlorophenylamino)-5,6,7,8-tetrahydroquinazolin-4-yl}-N-methylpiperidine-3-carboxamide hydrochloride

¹H NMR(400MHz, CD₃OD) δ 7.84(s, 1H), 6.83(s, 1H), 6.68(s, 1H), 6.50(s, 1H), 4.43(d, 1H), 4.24(d, 1H), 3.30(m, 2H), 2.72(m, 5H), 2.53(m, 3H), 2.00-1.64(m, 8H); (Yield: 65 %)

### Example 130. (R)-1-[2-{3-amino-5-(trifluoromethyl)phenylamino}-5,6,7,8-tetrahydroquinazolin-4-yl]-N-methylpiperidine-3-carboxamide hydrochloride

The titled compound was prepared as a white solid in the same manner as Example 99 by using (S)-1-(2-chloro-5,6,7,8-tetrahydroquinazolin-4-yl)-N-methylpiperidine-3-carboxamide prepared in Reference Example 39 and 3-(trifluoromethyl)-1,5-phenylenediamine.
¹H NMR(400MHz, CD₃OD) δ 8.07(s, 1H), 7.97(s, 1H), 7.45(s, 1H), 4.49(m, 1H), 4.25(m, 1H), 3.40(m, 2H), 2.80(m, 2H), 2.62(s, 3H), 2.58(m, 3H), 2.04-1.67(m, 8H); (Yield: 52 %)

### Example 131. (S)-N-{1-(2-[{3-amino-5-(trifluoromethyl)phenyl}amino]quinazolin-4-yl)pyrrolidin-3-yl}acetamide

A mixture of *(S)*-*N*-{1-(2-chloroquinazolin-4-yl)pyrrolidin-3-yl}acetamide (20 mg, 0.07 mmol) prepared in Reference Example 40 and 5-(trifluoromethyl)-1,3-phenylenediamine (15 mg, 0.08 mmol) was stirred for 40 minutes in a microwave (600 W). After cooling to room temperature, the resulting product was purified with silica gel column chromatography (dichloromethane/methanol = 20/1) to prepare the titled compound (10 mg) as a pale brown solid.
¹H NMR (400MHz, CD₃OD) 5 8.15(d, 1H), 7.62(t, 1H), 7.55-7.45(m, 2H), 7.23(t, 1H), 7.17(s, 1H), 6.61(s, 1H), 4.55-4.45(m, 1H), 4.30-4.00(m, 3H), 3.86(dd, 1H), 2.35-2.20(m, 1H), 2.15-2.05(m, 1H), 1.95(s, 3H)

### Examples 132 to 134

The titled compounds of Examples 132 to 134 were prepared in the same manner as Example 131 by reacting 4-fluoro-1,3-diaminobenzene, 4-chloro-1,3-diaminobenzene or 3,5-diaminobenzonitrile respectively with *(S)-N*-{1-(2-chloroquinazolin-4-yl)pyrrolidin-3-yl}acetamide prepared in Reference Example 40.

### Example 132. (S)-N-(1-[2-{(3-amino-4-fluorophenyl)amino}quinazolin-4-yl]pyrrolidin-3-yl)acetamide

¹H NMR (400MHz, CD₃OD) δ 8.16(d, 1H), 7.64(t, 1H), 7.47(dd, 1H), 7.26(t, 1H), 7.18(dd, 1H), 6.95-6.75(m, 2H), 4.48(t, 1H), 4.30-4.00(m, 3H), 3.88(dd, 1H), 2.35-2.25(m, 1H), 2.15-2.00(m, 1H), 1.95(s, 3H); (Yield: 21 %)

### Example 133. (S)-N-(1-[2-{(3-amino-4-chlorophenyl)amino}quinazolin-4-yl]pyrrolidin-3-yl)acetamide

¹H NMR (400MHz, CD₃OD) δ 8.14(d, 1H), 7.62(t, 1H), 7.47(d, 1H), 7.28(d, 1H), 7.24(t, 1H), 7.11(d, 1H), 6.92(dd, 1H), 4.48(t, 1H), 4.30-4.00(m, 3H), 3.86(dd, 1H), 2.35-2.25(m, 1H), 2.15-2.00(m, 1H), 1.95(s, 3H); (Yield: 15 %)

### Example 134. (S)-N-(1-[2-{(3-amino-5-cyanophenyl)amino}quinazolin-4-yl]pyrrolidin-3-yl)acetamide

¹H NMR(400MHz, CD₃OD) δ 8.16(d, 1H), 7.63(t, 1H), 7.51(d, 1H), 7.45(s, 1H), 7.31(s, 1H), 7.25(t, 1H), 6.60(s, 1H), 4.50(t, 1H), 4.30-4.00(m, 3H), 3.87(dd, 1H), 2.35-2.25(m, 1H), 2.15-2.00(m, 1H), 1.97(s, 3H); (Yield: 20 %)

### Example 135. (S)-N-(1-[2-{(3-amino-4-nitrophenyl)amino}quinazolin-4-yl)pyrrolidin-3-yl)acetamide

A mixture of *(S)*-*N*-{1-(2-chloroquinazolin-4-yl)pyrrolidin-3-yl}acetamide (20 mg, 0.07 mmol) prepared in Reference Example 40, 4-nitro-1,3-phenylenediamine (11.8 mg, 0.08 mmol), palladium acetate (0.77 mg, 0.003 mmol), 4,5-bis(diphenylphosphino)-9,9-dimethylxanthine (4.0 mg, 0.01 mmol), cesium carbonate (44.5 mg, 0.14 mmol) and anhydrous 1,4-dioxane (1 ml) was stirred for 1 hour in a microwave (600 W). After cooling the reaction solution to room temperature, the same was filtered by celite, and then the filtrate was concentrated under reduced pressure. The resulting residue was purified with silica gel column chromatography (dichloromethane/methanol = 20/1) to prepare the titled compound (5 mg) as a pale yellow solid.
¹H NMR (400MHz, CD₃OD) δ 8.16(d, 1H), 7.96(d, 1H), 7.74(s, 1H), 7.70-7.55(m, 2H), 7.25(t, 1H), 6.83(d, 1H), 4.50-4.40(m, 1H), 4.30-4.00(m, 3H), 3.85(d, 1H), 2.40-2.25(m, 1H), 2.15-2.00(m, 1H), 1.96(s, 3H)

### Example 136. (S)-N-(1-[2-{(4-amino-3-nitrophenyl)amino}quinazolin-4-yl)pyrrolidin-3-yl)acetamide

The titled compound was prepared as a pale yellow solid in the same manner as Example 135 by using *(S)-N-*{1-(2-chloroquinazolin-4-yl)pyrrolidin-3-yl}acetamide prepared in Reference Example 40 and 2-nitro-1,4-phenylenediamine.
¹H NMR (400MHz, CD₃OD) δ 8.71(s, 1H), 8.16(d, 1H), 7.63(t, 1H), 7.55-7.45(m, 2H), 7.24(t, 1H), 6.95(d, 1H), 4.55-4.45(m, 1H), 4.35-4.00(m, 3H), 3.89(d, 1H), 2.35-2.25(m, 1H), 2.15-2.05(m, 1H), 1.96(s, 3H); (Yield: 11 %)

### Example 137. (S)-N¹-[4-{3-(methylamino)pyrrolidin-1-yl}quinazolin-2-yl]-5-(trifluoromethyl)benzene-1,3-diamine

A mixture of *(S)*-1-(2-chloroquinazolin-4-yl)-*N-*methylpyrrolidin-3-amine (25 mg, 0.1 mmol) prepared in Reference Example 41, 5-(trifluoromethyl)-1,3-phenylenediamine (21.3 mg, 0.12 mmol), palladium acetate (0.22 mg, 0.001 mmol), 4,5-bis(diphenylphosphino)-9,9-dimethylxanthine (1.7 mg, 0.003 mmol), cesium carbonate (81.5 mg, 0.25 mmol) and anhydrous 1,4-dioxane (1 ml) was stirred for 1 hour in a microwave (600 W). After cooling the reaction solution to room temperature, the same was filtered by using celite and the filtrate was concentrated under reduced pressure. The resulting residue was purified with silica gel column chromatography (dichloromethane/methanol = 20/1) to prepare the titled compound (5 mg) as a pale yellow solid.
¹H NMR(400MHz, CD₃OD) δ 8.14(d, 1H), 7.65-7.55(m, 2H), 7.45(d, 1H), 7.20-7.10(m, 2H), 6.57(s, 1H), 4.20-4.05(m, 2H), 4.05-3.95(m, 1H), 3.85-3.75(m, 1H), 3.45-3.40(m, 1H), 2.47(s, 3H), 2.35-2.25(m, 1H), 2.05-1.95(m, 1H)

### Examples 138 and 139

The titled compounds of Examples 138 and 139 were prepared in the same manner as Example 137 by reacting 4-chloro-1,3-diaminobenzene or 3,5-diaminobenzonitrile respectively with *(S)*-1-(2-chloroquinazolin-4-yl)-*N-*methylpyrrolidin-3-amine prepared in Reference Example 41.

### Example 138. (S)-4-chloro-N¹-[4-{3-(methylamino)pyrrolidin-1-yl}quinazolin-2-yl]benzene-1,3-diamine

¹H NMR(400MHz, CD₃OD) δ 8.12(d, 1H), 7.55(d, 1H), 7.45(d, 1H), 7.36(d, 1H), 7.16(t, 1H), 7.08(d, 1H), 6.95(d, 1H), 4.20-4.05(m, 2H), 4.05-3.95(m, 1H), 3.85-3.75(m, 1H), 3.45-3.35(m, 1H), 2.46(s, 3H), 2.35-2.25(m, 1H), 2.05-1.90(m, 1H); (Yield: 22 %)

### Example 139. (S)-3-amino-5-([4-{3-(methylamino)pyrrolidin-1-yl}quinazolin-2-yl]amino)benzonitrile

¹H NMR(400MHz, CD₃OD) δ 8.11(d, 1H), 7.60-7.40(m, 3H), 7.29(d, 1H), 7.17 (t, 1H), 6.55(d, 1H), 4.20-4.00(m, 2H), 4.00-3.90(m, 1H), 3.85-3.75(m, 1H), 3.45-3.35 (m, 1H), 2.46(s, 3H), 2.30-2.20(m, 1H), 2.00-1.90(m, 1H); (Yield: 20 %)

### Examples 140 to 142

The titled compounds of Examples 140 to 142 were prepared in the same manner as Example 137 by reacting 5-(trifluoromethyl)-1,3-phenylenediamine, 3,5-diaminobenzonitrile or 4-chloro-1,3-diaminobenzene respectively with *(S)*-1-(2-chloroquinazolin-4-yl)-*N-*ethylpyrrolidin-3-amine prepared in Reference Example 42.

### Example 140. (S)-N¹-[4-{3-(ethylamino)pyrrolidin-1-yl}quinazolin-2-yl]-5-(trifluoromethyl)benzene-1,3-diamine

¹H NMR(400MHz, CD₃OD) δ 8.15(d, 1H), 7.65-7.50(m, 2H), 7.46(d, 1H), 7.20-7.15(m, 2H), 6.57(s, 1H), 4.20-4.05(m, 2H), 4.02(q, 1H), 3.85-3.75(m, 1H), 3.60-3.50(m, 1H), 2.78(q, 2H), 2.35-2.25(m, 1H), 2.10-1.90(m, 1H), 1.19(t, 3H); (Yield: 25 %)

### Example 141. (S)-3-amino-5-([4-{3-(ethylamino)pyrrolidin-1-yl}quinazolin-2-yl]amino)benzonitrile

¹H NMR(400MHz, CD₃OD) δ 8.14(d, 1H), 7.65-7.45(m, 3H), 7.29(s, 1H), 7.18(t, 1H), 6.56(s, 1H), 4.20-4.05(m, 2H), 3.99(q, 1H), 3.80-3.70(m, 1H), 3.51(t, 1H), 2.76(q, 2H), 2.35-2.25(m, 1H), 2.00-1.90(m, 1H), 1.18(t, 3H); (Yield: 21 %)

### Example 142. (S)-4-chloro-N¹-[4-{3-(ethylamino)pyrrolidin-1-yl}quinazolin-2-yl]benzene-1,3-diamine

¹H NMR(400MHz, CD₃OD) δ 8.14(d, 1H), 7.70-7.00(m, 6H), 4.25-4.10(m, 2H), 4.10-4.00(m, 1H), 3.90-3.80(m, 1H), 3.65-3.55(m, 1H), 2.85-2.70(m, 2H), 2.35-2.25(m, 1H), 2.10-1.95(m, 1H), 1.14(t, 3H); (Yield: 12 %)

### Examples 143 and 144

The titled compounds of Examples 143 and 144 were prepared in the same manner as Example 137 by reacting 5-(trifluoromethyl)-1,3-phenylenediamine or 3,5-diaminobenzonitrile respectively with *(R)-N-*{1-(2-chloroquinazolin-4-yl)piperidin-3-yl}acetamide prepared in Reference Example 43.

### Example 143. (R)-N-{1-(2-[{3-amino-5-(trifluoromethyl)phenyl}amino]quinazolin-4-yl)piperidin-3-yl}acetamide

¹H NMR(400MHz, CD₃OD) δ 7.95(d, 1H), 7.80(d, 1H), 7.70-7.45(m, 3H), 7.35-7.15(m, 2H), 4.35-4.20(m, 1H), 4.95-3.35(m, 3H), 3.25-3.05(m, 1H), 2.10-1.95(m, 2H), 1.92(s, 3H), 1.91-1.55(m, 2H); (Yield: 21 %)

### Example 144. (R)-N-(1-[2-{(3-amino-5-cyanophenyl)amino}quinazolin-4-yl]piperidin-3-yl)acetamide

¹H NMR(400MHz, CD₃OD) δ 7.92 (d, 1H), 7.70-7.55 (m, 2H), 7.50(d, 1H), 7.35(d, 1H), 7.24(s, 1H), 6.58(d, 1H), 4.25(d, 1H), 4.15-4.00 (m, 2H), 3.59(d, 1H), 3.14(t, 1H), 2.15-1.95(m, 2H), 1.93(s, 3H), 1.90-1.75(m, 1H), 1.75-1.55(m, 1H); (Yield: 18 %)

### Examples 145 to 154

The titled compounds of Examples 145 to 154 were prepared in the same manner as Example 137 by reacting 5-(trifluoromethyl)-1,3-phenylenediamine, 4-chloro-1,3-diaminobenzene, 3,5-diaminobenzonitrile, 2,5-diaminobenzonitrile, 3-methoxy-4-methylaniline, 4-methyl-3-(trifluoromethyl)aniline, 5-amino-2-methylpyridine, 4-amino-2-fluoropyridine, 6-amino-2-methylpyridine-3-carbonitrile or 6-amino-3-picholine respectively with *(S)-N*-{1-(2-chloro-7-methoxyquinazolin-4-yl)pyrrolidine-3-yl}acetamide prepared in Reference Example 44.

### Example 145. (S)-N-{1-(2-[{3-amino-5-(trifluoromethyl)phenyl}amino]-7-methoxyquinazolin-4-yl)pyrrolidin-3-yl}acetamide

¹H NMR (400MHz, CD₃OD) δ 7.98(d, 1H), 7.50(s, 1H), 7.19 (s, 1H), 6.83(s, 1H), 6.77(d, 1H), 6.58(s, 1H), 4.47(t, 1H), 4.20-3.90(m, 3H), 3.87(s, 3H), 3.77(dd, 1H), 2.30-2.20(m, 1H), 2.15-2.00(m, 1H), 1.95(s, 3H); (Yield: 23 %)

### Example 146. (S)-N-(1-[2-{(3-amino-4-chlorophenyl)amino}-7-methoxyquinazolin-4-yl]pyrrolidin-3-yl}acetamide

¹H NMR(400MHz, CD₃OD) δ 7.94 (d, 1H), 7.31(s, 1H), 7.07 (d, 1H), 6.91(d, 1H), 6.82(s, 1H), 6.75(d, 1H), 4.43(t, 1H), 4.20-3.88(m, 3H), 3.86(s, 3H), 3.85-3.70(m, 1H), 2.35-2.20(m, 1H), 2.10-2.00(m, 1H), 1.95(s, 3H); (Yield: 15 %)

### Example 147. (S)-N-(1-[2-{(3-amino-5-cyanophenyl)amino}-7-methoxyquinazolin-4-yl]pyrrolidin-3-yl)acetamide

¹H NMR(400MHz, CD₃OD) 5 7.97(d, 1H), 7.43 (s, 1H), 7.28 (s, 1H), 6.86(s, 1H), 6.78(dd, 1H), 6.56(s, 1H), 4.46(t, 1H), 4.20-3.90(m, 3H), 3.88(s, 3H), 3.76(dd, 1H), 2.30-2.20(m, 1H), 2.10-2.00(m, 1H), 1.95(s, 3H); (Yield: 20 %)

### Example 148. (S)-N-(1-[2-{(4-amino-3-cyanophenyl)amino}-7-methoxyquinazolin-4-yl]pyrrolidin-3-yl)acetamide

¹H NMR(400MHz, CD₃OD) δ 7.99(d, 1H), 7.79 (s, 1H), 7.50(d, 1H), 6.90-6.70(m, 3H), 4.46(t, 1H), 4.20-3.90(m, 3H), 3.88(s, 3H), 3.76(dd, 1H), 2.35-2.20(m, 1H), 2.15-2.00(m, 1H), 1.95 (s, 3H); (Yield: 15 %)

### Example 149. (S)-N-(1-[7-methoxy-2-{(3-methoxy-4-methylphenyl)amino}quinazolin-4-yl]pyrrolidin-3-yl)acetamide

¹H NMR(400MHz, CD₃OD) δ 8.02(d, 1H), 7.38(s, 1H), 7.02(s, 2H), 6.85(s, 1H), 6.81(d, 1H), 4.46(t, 1H), 4.30-4.00(m, 3H), 3.88(s, 3H), 3.84(s, 3H), 3.84-3.75(m, 1H), 2.35-2.20(m, 1H), 2.14(s, 3H), 2.10-2.00(m, 1H), 1.95(s, 3H); (Yield: 25 %)

### Example 150. (S)-N-(1-[2-{(3-trifluoromethyl-4-methylphenyl)amino}-7-methoxyquinazolin-4-yl]pyrrolidin-3-yl)acetamide

¹H NMR(400MHz, CD₃OD) δ 8.25(s, 1H), 8.04(d, 1H), 7.66(d, 1H), 7.27(d, 1H), 6.87(s, 1H), 6.82(d, 1H), 4.49(t, 1H), 4.30-4.00(m, 3H), 3.89(s, 3H), 3.85-3.80(m, 1H), 2.42(s, 3H), 2.35-2.25(m, 1H), 2.15-2.00(m, 1H), 1.95(s, 3H); (Yield: 26 %)

### Example 151. (S)-N-(1-[7-methoxy-2-{(6-methylpyridin-3-yl)amino}quinazolin-4-yl]pyrrolidin-3-yl)acetamide

¹H NMR (400MHz, CD₃OD) δ 8.79(s, 1H), 8.07(d, 1H), 8.02(d, 1H), 7.23(d, 1H), 6.88(s, 1H), 6.81(d, 1H), 4.47(t, 1H), 4.25-3.90(m, 3H), 3.89(s, 3H), 3.85-3.80(m, 1H), 2.48(s, 3H), 2.35-2.20(m, 1H), 2.15-2.00(m, 1H), 1.95(s, 3H); (Yield: 16 %)

### Example 152. (S)-N-(1-[2-{(2-fluoropyridin-4-yl)amino}-7-methoxyquinazolin-4-yl]pyrrolidin-3-yl)acetamide

¹H NMR (400MHz, CD₃OD) δ 8.01(d, 1H), 7.88(d, 1H), 7.75 (s, 1H), 7.45(d, 1H), 6.94(s, 1H), 6.83(d, 1H), 4.48(t, 1H), 4.20-3.95(m, 3H), 3.89(s, 3H), 3.85-3.75(m, 1H), 2.35-2.25(m, 1H), 2.15-2.00(m, 1H), 1.96(s, 3H); (Yield: 18 %)

### Example 153. (S)-N-(1-[2-{(5-cyano-6-methylpyridin-2-yl)amino}-7-methoxyquinazolin-4-yl]pyrrolidin-3-yl)acetamide

¹H NMR (400MHz, CDCl₃) δ 8.52(d, 1H), 7.90(d, 1H), 7.78 (d, 1H), 6.99(s, 1H), 6.82(d, 1H), 6.01(brs, 1H), 4.64(brs, 1H), 4.20-3.92(m, 2H), 3.92(s, 3H), 3.90-3.70(m, 2H), 2.62(s, 3H), 2.35-2.20(m, 1H), 2.20-2.05(m, 1H), 2.03(s, 3H); (Yield: 12 %)

### Example 154. (S)-N-(1-[7-methoxy-2-{(5-methylpyridin-2-yl)amino}quinazolin-4-yl]pyrrolidin-3-yl)acetamide

¹H NMR (400MHz, CD₃OD) δ 8.09 (d, 1H), 7.41(s, 1H), 7.22(t, 1H), 7.13(d, 1H), 6.91(s, 1H), 6.64(d, 1H), 4.49(t, 1H), 4.30-4.00(m, 3H), 3.91(s, 3H), 3.91-3.84(m, 1H), 3.84(s, 3H), 2.35-2.25(m, 1H), 2.15-2.00(m, 1H), 1.95(s, 3H); (Yield: 15 %)

### Example 155. (S)-N-(1-[2-{(3-amino-5-cyanophenyl)amino}pyrido[3,2-d]pyrimidin-4-yl]pyrrolidin-3-yl)acetamide

The titled compound was prepared as a pale yellow solid in the same manner as Example 137 by using *(S)-N-*{1-(2-chloropyrido[3,2-*d*]pyrimidin-4-yl)pyrrolidin-3-yl}acetamide prepared in Reference Example 45 and 3,5-diaminobenzonitrile.
¹H NMR(400MHz, CD₃OD) δ 8.43 (d, 1H), 7.74 (d, 1H), 7.55-7.44 (m, 2H), 7.34 (s, 1H), 6.55 (s, 1H), 4.80-3.65 (m, 5H), 2.35-2.15 (m, 1H), 2.15-1.97 (m, 1H), 1.95 (s, 3H); (Yield: 29 %)

### Example 156. (S)-3-amino-5-[{4-(3-aminopyrrolidin-1-yl)pyrido[3,2-d]pyrimidin-2-yl}amino]benzonitrile dihydrochloride

A mixture of (*S*)-*tert*-butyl {1-(2-chloropyrido[3,2-*d*]pyrimidin-4-yl)pyrrolidin-3-yl}carbamate (20 mg, 0.06 mmol) prepared in Reference Example 46, 3,5-diaminobenzonitrile (8.8 mg, 0.07 mmol), palladium acetate (0.22 mg, 0.001 mmol), 4,5-bis(diphenylphosphino)-9,9-dimethylxanthine (1.7 mg, 0.003 mmol), cesium carbonate (58.6 mg, 0.18 mmol) and anhydrous 1,4-dioxane (1 ml) was stirred for 1 hour in a microwave (600 W). After cooling the reaction solution to room temperature, the same was concentrated under reduced pressure. The resulting residue was purified with silica gel column chromatography (dichloromethane/methanol = 20/1) and dissolved in ethyl acetate (1 ml), and then hydrochloric acid gas was added thereto. The resulting white solid was filtered to prepare the titled compound (3 mg).
¹H NMR(400MHz, CD₃OD) δ 8.80(s, 1H), 8.06(s, 1H) , 8.00-7.80(m, 2H), 7.36(s, 1H), 7.00-6.90(m, 1H), 4.85-4.70(m, 1H), 4.50-4.00(m, 4H), 2.70-2.15(m, 2H)

### Example 157. (S)-3-amino-5-([4-{3-(methylamino)pyrrolidin-1-yl}pyrido[3,2-d]pyrimidin-2-yl]amino)benzonitrile dihydrochloride

A mixture of (*S*)-*tert*-butyl {1-(2-chloropyrido[3,2-*d*]pyrimidin-4-yl)pyrrolidin-3-yl}(methyl)carbamate (20 mg, 0.06 mmol) prepared in Reference Example 47, 3,5-diaminobenzonitrile (8.8 mg, 0.07 mmol), palladium acetate (0.22 mg, 0.001 mmol), 4,5-bis(diphenylphosphino)-9,9-dimethylxanthine (1.7 mg, 0.003 mmol), cesium carbonate (58.6 mg, 0.18 mmol) and anhydrous 1,4-dioxane (1 ml) was stirred for 1 hour in a microwave (600 W). After cooling the reaction solution to room temperature, the same was concentrated under reduced pressure. The resulting residue was purified with silica gel column chromatography (dichloromethane/methanol = 30/1) and dissolved in ethyl acetate (1 ml), and then hydrochloric acid gas was added thereto. The resulting white solid was filtered to prepare the titled compound (5 mg).
¹H NMR(400MHz, CD₃OD) δ 8.81(s, 1H), 8.09(d, 2H), 7.87(brs, 1H), 7.55(s, 1H), 7.10-7.00(m, 1H), 4.85-4.70(m, 1H), 4.50-4.00(m, 4H), 2.85(s, 3H), 2.80-2.30(m, 2H)

### Example 158. (S)-N¹-[4-{3-(methylamino)pyrrolidin-1-yl}pyrido[3,2-d]pyrimidin-2-yl]-5-(trifluoromethyl)benzene-1,3-diamine dihydrochloride

The titled compound was prepared as a pale yellow solid in the same manner as Example 157 by using *(S)*-*tert*-butyl {1-(2-chloropyrido[3,2-d]pyrimidin-4-yl)pyrrolidin-3-yl}(methyl)carbamate prepared in Reference Example 47 and 5-(trifluoromethyl)-1,3-phenylenediamine.
¹H NMR(400MHz, CD₃OD) δ 8.80 (s, 1H), 8.15-7.85(m, 4H), 7.38(s, 1H), 5.10-4.90(m, 1H), 4.50-3.80(m, 4H), 2.95-2.75(m, 3H), 2.70-2.15(m, 2H); (Yield: 18 %)

### Examples 159 to 163

The titled compounds of Examples 159 to 163 were prepared in the same manner as Example 157 by reacting 3,5-diaminobenzonitrile, 5-(trifluoromethyl)-1,3-phenylenediamine, 2-(trifluoromethyl)-1,4-phenylenediamine, 4-chloro-1,3-diaminobenzene or 5-amino-2-methylbenzonitrile respectively with (*S*)-*tert*-butyl {1-(2-chloropyrido[3,2-*d*]pyrimidin-4-yl)pyrrolidin-3-yl}(propyl)carbamate prepared in Reference Example 48.

### Example 159. (S)-3-amino-5-([4-{3-(propylamino)pyrrolidin-1-yl}pyrido[3,2-d]pyrimidin-2-yl]amino)benzonitrile dihydrochloride

¹H NMR(400MHz, CD₃OD) δ 8.80(s, 1H), 8.05 (d, 1H), 7.86(s, 2H), 7.79(s, 1H), 7.35(s, 1H), 5.00-4.90(m, 1H), 4.80-4.00(m, 4H), 3.14(t, 2H), 2.75-2.25 (m, 2H), 1.81(t, 2H), 1.07(t, 3H); (Yield: 23 %)

### Example 160. (S)-N¹-[4-{3-(propylamino)pyrrolidin-1-yl}pyrido[3,2-d]pyrimidin-2-yl]-5-(trifluoromethyl)benzene-1,3-diamine dihydrochloride

¹H NMR(400MHz, CD₃OD) 5 8.80(s, 1H), 8.20-7.80(m, 4H), 7.45(s, 1H), 4.95(brs, 1H), 4.80-4.00(m, 4H), 3.12(q, 2H), 2.60-2.30(m, 2H), 1.82(t, 2H), 1.06(t, 3H); (Yield: 21 %)

### Example 161. (S)-N¹-[4-{3-(propylamino)pyrrolidin-1-yl}pyrido[3,2-d]pyrimidin-2-yl]-3-(trifluoromethyl)benzene-1,4-diamine dihydrochloride

¹H NMR(400MHz, CD₃OD) δ 8.74(s, 1H), 8.05-7.95(m, 1H), 7.90-7.75(m, 2H), 7.55(brs, 1H), 7.12(d, 1H), 5.00-4.90(m, 1H), 4.75-4.00(m, 4H), 3.11(q, 2H), 2.75-2.30(m, 2H), 1.79(q, 2H), 1.06(t, 3H); (Yield: 15 %)

### Example 162. (S)-4-chloro-N¹-[4-{3-(propylamino)pyrrolidin-1-yl}pyrido[3,2-d]pyrimidin-2-yl]benzene-1,3-diamine dihydrochloride

¹H NMR(400MHz, CD₃OD) δ 8.76(s, 1H), 8.01 (brs, 1H), 7.82(brs, 1H), 7.50-7.30(m, 2H), 7.25-7.10(m,, 1H), 5.00-4.90(m, 1H), 4.75-4.00(m, 4H), 3.12(q, 2H), 2.70-2.25(m, 2H), 1.78(q, 2H), 1.07(t, 3H); (Yield: 18 %)

### Example 163. (S)-2-methyl-5-([4-{3-(propylamino)pyrrolidin-1-yl}pyrido[3,2-d]pyrimidin-2-yl]amino)benzonitrile dihydrochloride

¹H NMR(400MHz, CD₃OD) δ 8.77(s, 1H), 8.10-8.00(m, 2H), 7.83(s, 1H), 7.73(dd, 1H), 7.51(s, 1H), 5.00-4.90(m, 1H), 4.75-4.00(m, 4H), 3.20-3.00(m, 2H), 2.70-2.55(m, 1H), 2.55 (s, 3H), 2.54-2.25(m, 1H), 1.90-1.70(m, 2H), 1.10-1.00(m, 3H); (Yield: 28 %)

### Example 164. (S)-3-amino-5-([4-{3-(pentylamino)pyrrolidin-1-yl}pyrido[3,2-d]pyrimidin-2-yl]amino)benzonitrile dihydrochloride

The titled compound was prepared as a pale yellow solid in the same manner as Example 157 by using *(S)*-*tert*-butyl {1-(2-chloropyrido[3,2-*d*]pyrimidin-4-yl)pyrrolidin-3-yl}(pentyl)carbamate prepared in Reference Example 49 and 3,5-diaminobenzonitrile.
¹H NMR (400MHz, CD₃OD) δ 8.80(s, 1H), 8.07 (s, 1H), 7.99 (s, 2H), 7.85(s, 1H), 7.45(s, 1H), 4.80-4.00(m, 5H), 3.17(brs, 2H), 2.75-2.25 (m, 2H), 1.79(brs, 2H), 1.43(brs, 4H), 0.96(brs, 3H); (Yield: 25 %)

### Example 165. (R)-N-(1-[2-{(3-amino-5-cyanophenyl)amino}pyrido[3,2-d]pyrimidin-4-yl]piperidin-3-yl)acetamide hydrochloride

The titled compound was prepared as a pale yellow solid in the same manner as Example 157 by using *(R)*-*N*-{1-(2-chloropyrido[3,2-*d*]pyrimidin-4-yl)piperidin-3-yl}acetamide prepared in Reference Example 50 and 3,5-diaminobenzonitrile.
¹H NMR(400MHz, CD₃OD) δ 8.45(s, 1H), 7.83(d, 1H), 7.60-7.50 (m, 1H), 7.44(s, 1H), 7.33(s, 1H), 6.58(s, 1H), 5.00-4.90(m, 1H), 4.20-3.70(m, 4H), 2.10-1.90(m, 2H), 1.89(s, 3H), 1.85-1.65(m, 2H); (Yield: 30 %)

### Examples 166 and 167

The titled compounds of Examples 166 and 167 were prepared in the same manner as Example 156 by reacting 3,5-diaminobenzonitrile and 5-(trifluoromethyl)-1,3-phenylenediamine respectively with *(R)*-*tert*-butyl {1-(2-chloropyrido[3,2-*d*]pyrimidin-4-yl)piperidin-3-yl}carbamate prepared in Reference Example 51.

### Example 166. (R)-3-amino-5-[{4-(3-aminopiperidin-1-yl)pyrido[3,2-d]pyrimidin-2-yl}amino]benzonitrile dihydrochloride

¹H NMR(400MHz, CD₃OD) δ 8.79(s, 1H), 8.08 (d, 1H), 7.90-7.80(m, 1H), 7.70-7.50(m, 1H), 7.40-7.30(m, 1H), 7.00-6.85(m, 1H), 5.70-5.50(m, 1H), 5.00-4.80(m, 1H), 4.40-3.90(m, 2H), 3.67(brs, 1H), 2.27(brs, 1H), 2.07(brs, 1H), 2.00-1.85(m, 2H); (Yield: 28 %)

### Example 167. (R)-N¹-{4-(3-aminopiperidin-1-yl)pyrido[3,2-d]pyrimidin-2-yl}-5-trifluoromethyl)benzene-1,3-diamine dihydrochloride

¹H NMR(400MHz, CD₃OD) δ 8.80(s, 1H), 8.10(d, 1H), 8.00-7.80(m, 2H), 7.68(s, 1H), 7.40(s, 1H), 5.54(brs, 1H), 4.90-4.70(m, 1H), 4.42(brs, 1H), 4.15(brs, 1H), 3.69(brs, 1H), 2.27 (brs, 1H), 2.10-1.80(m, 3H); (Yield: 18 %)

### Example 168. (S)-N-(1-[2-{(3-amino-5-cyanophenyl)amino}-6,7-dihydro-5H-cyclopenta[d]pyrimidin-4-yl]pyrrolidin-3-yl)acetamide hydrochloride

*n*-Butanol (1 ml) solution of *(S)-N-*{1-(2-chloro-6,7-dihydro-5*H*-cyclopenta[*d*]pyrimidin-4-yl)pyrrolidin-3-yl}acetamide (30 mg, 0.11 mmol) prepared in Reference Example 52 and 3,5-diaminobenzonitrile (15.7 mg, 0.12 mmol) was stirred at 130 °C overnight. After cooling the reaction solution to room temperature, ethyl acetate (1 ml) solution was added thereto and they were stirred for 2 hours. The resulting white solid was filtered to prepare the titled compound (37 mg).
¹H NMR(400MHz, CD₃OD) δ 7.16(s, 1H), 7.07 (s, 1H), 6.74(s, 1H), 4.42(brs, 1H), 4.30-3.60(m, 4H), 3.16(brs, 2H), 2.91(brs, 2H), 2.26(brs, 1H), 2.16(brs, 2H), 2.03(brs, 1H), 1.95 (s, 3H)

### Examples 169 to 173

The titled compounds of Examples 169 to 173 were prepared in the same manner as Example 168 by reacting 5-(trifluoromethyl)-1,3-phenylenediamine, 4-chloro-1,3-diaminobenzene, 5-amino-2-methylbenzonitrile, 4-methyl-3-(trifluoromethyl)aniline or 2-nitro-1,4-phenylenediamine respectively with *(S)-N-*{1-(2-chloro-6,7-dihydro-5H-cyclopenta[*d*]pyrimidin-4-yl)pyrrolidin-3-yl}acetamide prepared in Reference Example 52.

### Example 169. (S)-N-{1-(2-[{3-amino-5-(trifluoromethyl)phenyl}amino]-6,7-dihydro-5H-cyclopenta[d]pyrimidin-4-yl)pyrrolidin-3-yl}acetamide hydrochloride

¹H NMR (400MHz, CD₃OD) δ 7.25(s, 1H), 6.99(s, 1H), 6.73(s, 1H), 4.43(brs, 1H), 4.30-3.70(m, 4H), 3.15(brs, 2H), 2.90(brs, 2H), 2.25(brs, 1H), 2.16(brs, 2H), 2.02(brs, 1H), 1. 95 (s, 3H); (Yield: 48 %)

### Example 170. (S)-N-(1-[2-{(3-amino-4-chlorophenyl)amino}-6,7-dihydro-5H-cyclopenta[d]pyrimidin-4-yl]pyrrolidin-3-yl)acetamide hydrochloride

¹H NMR(400MHz, CD₃OD) δ 7.19(s, 1H), 7.02(s, 1H), 6.75(s, 1H), 4.40(brs, 1H), 4.30-3.65(m, 4H), 3.19(brs, 2H), 2.88(brs, 2H), 2.40-2.00(m, 4H), 1.96(s, 3H); (Yield: 36 %)

### Example 171. (S)-N-(1-[2-{(3-cyano-4-methylphenyl)amino}-6,7-dihydro-5H-cyclopenta[d]pyrimidin-4-yl]pyrrolidin-3-yl)acetamide hydrochloride

¹H NMR(400MHz, CD₃OD) δ 7.97(s, 1H), 7.68(s, 1H), 7.44 (s, 1H), 4.43(brs, 1H), 4.30-3.60(m, 4H), 3.17(brs, 2H), 2.92(brs, 2H), 2.52(s, 3H), 2.35-2.00(m, 4H), 1.95(s, 3H); (Yield: 59 %)

### Example 172. (S)-N-{1-(2-[{4-methyl-3-(trifluoromethyl)phenyl}amino]-6,7-dihydro-5H-cyclopenta[d]pyrimidin-4-yl)pyrrolidin-3-yl}acetamide hydrochloride

¹H NMR (400MHz, CD₃OD) δ 8.15-8.00 (m, 1H), 7.61(d, 1H), 7.39(d, 1H), 4.44 (brs, 1H), 4.25-3.60(m, 4H), 3.17(brs, 2H), 2.93(t, 2H), 2.46(s, 3H), 2.35-2.00(m, 4H), 1.96(s, 3H); (Yield: 52 %)

### Example 173. (S)-N-(1-[2-{(4-amino-3-nitrophenyl)amino}-6,7-dihydro-5H-cyclopenta[d]pyrimidin-4-yl]pyrrolidin-3-yl)acetamide hydrochloride

¹H NMR(400MHz, CD₃OD) δ 8.51(s, 1H), 7.40(d, 1H), 7.01(d, 1H), 4.42(brs, 1H), 4.25-3.60(m, 4H), 3.20-3.10(m, 2H), 2.95-2.85(m, 2H), 2.35-2.00 (m, 4H), 1.95(s, 3H); (Yield: 30 %)

### Example 174. (S)-3-amino-5-[{4-(3-aminopyrrolidin-1-yl)-6,7-dihydro-5H-cyclopenta[d]pyrimidin-2-yl}amino]benzonitrile dihydrochloride

A mixture of (*S*)-*tert*-butyl {1-(2-chloro-6,7-dihydro-5*H*-cyclopenta[*d*]pyrimidin-4-yl)pyrrolidin-3-yl}carbamate (34 mg, 0.1 mmol) prepared in Reference Example 53, 3,5-diaminobenzonitrile (16 mg, 0.12 mmol), palladium acetate (0.22 mg, 0.001 mmol), 4,5-bis(diphenylphosphino)-9,9-dimethylxanthine (1.74 mg, 0.003 mmol), cesium carbonate (97.8 mg, 0.3 mmol) and anhydrous 1,4-dioxane (1 ml) was stirred at 120 °C overnight. After cooling the reaction solution to room temperature, the same was concentrated under reduced pressure. The resulting residue was purified with silica gel column chromatography (n-hexane/ethyl acetate = 2/1) and dissolved in ethyl acetate (1 ml) and methanol (1 ml), and then hydrochloric acid gas was added thereto. The resulting white solid was filtered to prepare the titled compound (20 mg).
¹H NMR(400MHz, CD₃OD) δ 7.90-7.60(m, 2H), 7.23(s, 1H), 4.40-3.80(m, 5H), 3.25-3.15(m, 2H), 2.98(t, 2H), 2.60-2.40(m, 1H), 2.30-2.10(m, 3H)

### Examples 175 to 178

The titled compounds of Examples 175 to 178 were prepared in the same manner as Example 174 by reacting 5-(trifluoromethyl)-1,3-phenylenediamine, 4-chloro-1,3-diaminobenzene, 5-amino-2-methylbenzonitrile or 4-methyl-3-(trifluoromethyl)aniline respectively with *(S)*-*tert*-butyl {1-(2-chloro-6,7-dihydro-5*H*-cyclopenta[*d*]pyrimidin-4-yl)pyrrolidin-3-yl}carbamate prepared in Reference Example 53.

### Example 175. (S)-N¹-{4-(3-aminopyrrolidin-1-yl)-6,7-dihydro-5H-cyclopenta[d]pyrimidin-2-yl}-5-(trifluoromethyl)benzene-1,3-diamine dihydrochloride

¹H NMR(400MHz, CD₃OD) δ 8.15-7.85(m, 2H), 7.40(s, 1H), 4.40-3.85(m, 5H), 3.24(brs, 2H), 3.00(brs, 2H), 2.60-2.40(m, 1H), 2.35-2.10(m, 3H); (Yield: 35 %)

### Example 176. (S)-N¹-{4-(3-aminopyrrolidin-1-yl)-6,7-dihydro-5H-cyclopenta[d]pyrimidin-2-yl}-4-chlorobenzene-1,3-diamine dihydrochloride

¹H NMR(400MHz, CD₃OD) δ 7.40-7.20(m, 2H), 7.10-6.95(m, 1H), 4.30-3.80(m, 5H), 3.19(brs, 2H), 3.00-2.90(m, 2H), 2.55-2.30(m, 1H), 2.30-2.10(m, 3H); (Yield: 25%)

### Example 177. (S)-5-[{4-(3-aminopyrrolidin-1-yl)-6,7-dihydro-5H-cyclopenta[d]pyrimidin-2-yl}amino]-2-methylbenzonitrile dihydrochloride

¹H NMR (400MHz, CD₃OD) δ 7.95(s, 1H), 7.69(d, 1H), 7.45(d, 1H), 4.30-3.90(m, 5H), 3.21(t, 2H), 2.96(t, 2H), 2.52(s, 3H), 2.52-2.50(m, 1H), 2.30-2.10(m, 3H); (Yield: 45 %)

### Example 178. (S)-4-(3-aminopyrrolidin-1-yl)-N-{4-methyl-3-(trifluoromethyl)phenyl}-6,7-dihydro-5H-cyclopenta[d]pyrimidin-2-amine dihydrochloride

¹H NMR(400MHz, CD₃OD) δ 8.01 (d, 1H), 7.70-7.60(m, 1H), 7.41(t, 1H), 4.40-3.80(m, 5H), 3.25-3.10(m, 2H), 3.00-2.90(m, 2H), 2.55-2.40(m, 1H), 2.47(s, 3H), 2.35-2.10(m, 3H); (Yield: 42 %)

### Examples 179 to 183

The titled compounds of Examples 179 to 183 were prepared in the same manner as Example 174 by reacting 3,5-diaminobenzonitrile, 5-(trifluoromethyl)-1,3-phenylenediamine, 4-chloro-1,3-diaminobenzene, 5-amino-2-methylbenzonitrile or 4-methyl-3-(trifluoromethyl)aniline respectively with (*S*)-*tert*-butyl {1-(2-chloro-6,7-dihydro-5*H*-cyclopenta[*d*]pyrimidin-4-yl)pyrrolidin-3-yl}(methyl)carbamate prepared in Reference Example 54.

### Example 179. (S)-3-amino-5-([4-{3-(methylamino)pyrrolidin-1-yl}-6,7-dihydro-5H-cyclopenta[d]pyrimidin-2-yl]amino)benzonitrile dihydrochloride

¹H NMR (400MHz, CD₃OD) δ 8.15-8.00 (m, 2H), 7.47 (s, 1H), 4.40-3.80(m, 5H), 3.24(t, 2H), 3.00(t, 2H), 2.82 (s, 3H), 2.65-2.30(m, 2H), 2.30-2.15(m, 2H); (Yield: 45 %)

### Example 180. (S)-N¹-[4-{3-(methylamino)pyrrolidin-1-yl}-6,7-dihydro-5H-cyclopenta[d]pyrimidin-2-yl]-5-(trifluoromethyl)benzene-1,3-diamine dihydrochloride

¹H NMR(400MHz, CD₃OD) δ 8.20-7.90(m, 2H), 7.43 (s, 1H), 4.40-3.80(m, 5H), 3.22(brs, 2H), 3.00(brs, 2H), 2.80(s, 3H), 2.65-2.30(m, 2H), 2.02(brs, 2H); (Yield: 41 %)

### Example 181. (S)-4-chloro-N¹-[4-{3-(methylamino)pyrrolidin-1-yl}-6,7-dihydro-5H-cyclopenta[d]pyrimidin-2-yl]benzene-1,3-diamine dihydrochloride

¹H NMR(400MHz, CD₃OD) δ 8.15-7.85(m, 1H), 7.85-7.50(m, 2H), 4.40-3.80(m, 5H), 3.22(brs, 2H), 2.96(brs, 2H), 2.78(d, 3H), 2.54(brs, 1H), 2.39(brs, 1H), 2.20(brs, 2H); (Yield: 25 %)

### Example 182. (S)-2-methyl-5-([4-{3-(methylamino)pyrrolidin-1-yl}-6,7-dihydro-5H-cyclopenta[d]pyrimidin-2-yl]amino)benzonitrile dihydrochloride

¹H NMR (400MHz, CD₃OD) δ 8.02(s, 1H), 7.65(s, 1H), 7.46(d, 1H), 4.40-3.80(m, 5H), 3.22(brs, 2H), 2.97(t, 2H), 2.81(s, 3H), 2.52(s, 3H), 2.52-2.40(m, 1H), 2.40-2.30(m, 1H), 2.21(brs, 2H); (Yield: 52 %)

### Example 183. (S)-N-{4-methyl-3-(trifluoromethyl)phenyl}-4-{3-(methylamino)pyrrolidin-1-yl}-6,7-dihydro-5H-cyclopenta[d]pyrimidin-2-amine dihydrochloride

¹H NMR(400MHz, CD₃OD) δ 8.03(d, 1H), 7.70-7.60(m, 1H), 7. 42 (t, 1H), 4.40-3.70(m, 5H), 3.21(brs, 2H), 2.96(brs, 2H), 2.79(d, 3H), 2.54(brs, 1H), 2.47(s, 3H), 2.35(brs, 1H), 2.25-2.10(m, 2H); (Yield: 45 %)

### Examples 184 to 189

The titled compounds of Examples 184 to 189 were prepared in the same manner as Example 174 by reacting 3,5-diaminobenzonitrile, 5-(trifluoromethyl)-1,3-phenylenediamine, 2-(trifluoromethyl)-1,4-phenylenediamine, 4-chloro-1,3-diaminobenzene, 4-methyl-3-(trifluoromethyl)aniline or 5-chloro-1,3-diaminobenzene respectively with (*S*)-*tert*-butyl {1-(2-chloro-6,7-dihydro-5*H*-cyclopenta[*d*]pyrimidin-4-yl)pyrrolidin-3-yl}(propyl)carbamate prepared in Reference Example 55.

### Example 184. (S)-3-amino-S-([4-{3-(propylamino)pyrrolidin-1-yl}-6,7-dihydro-5H-cyclopenta[d]pyrimidin-2-yl]amino)benzonitrile dihydrochloride

¹H NMR (400MHz, CD₃OD) δ 8.01(s, 1H), 7.90(s, 1H), 7.38(s, 1H), 4.45-3.90(m, 5H), 3.23(brs, 2H), 3.11(t, 2H), 2.99(brs, 2H), 2.65-2.30(m, 2H), 2.21(brs, 2H), 1.80(brs, 2H), 1.06(t, 3H); (Yield: 42 %)

### Example 185. (S)-N¹-[4-{3-(propylamino)pyrrolidin-1-yl}-6,7-dihydro-5H-cyclopenta[d]pyrimidin-2-yl]-5-(trifluoromethyl)benzene-1,3-diamine dihydrochloride

¹H NMR(400MHz, CD₃OD) δ 8.25-7.95(m, 2H), 7.46(s, 1H), 4.50-3.80(m, 5H), 3.25(brs, 2H), 3.10(brs, 2H), 3.00(brs, 2H), 2.65-2.30(m, 2H), 2.21(brs, 2H), 1.81(brs, 2H), 1.06(t, 3H); (Yield: 35 %)

### Example 186. (S)-N¹-[4-{3-(propylamino)pyrrolidin-1-yl}-6,7-dihydro-5H-cyclopenta[d]pyrimidin-2-yl]-3-(trifluoromethyl)benzene-1,4-diamine dihydrochloride

¹H NMR(400MHz, CD₃OD) δ 8.15-7.25(m, 3H), 4.50-3.70(m, 5H), 3.21(brs, 2H), 3.08(brs, 2H), 2.94(brs, 2H), 2.54(brs, 1H), 2.38(brs, 1H), 2.18(brs, 2H), 1.80(brs, 2H), 1.10-0.90(m, 3H); (Yield: 29 %)

### Example 187. (S)-4-chloro-N¹-[4-{3-(propylamino)pyrrolidin-1-yl}-6,7-dihydro-5H-cyclopenta[d]pyrimidin-2-yl]benzene-1,3-diamine dihydrochloride

¹H NMR(400MHz, CD₃OD) δ 7.80(s, 1H), 7.60-7.35(m, 2H), 4.50-3.70(m, 5H), 3.19(brs, 2H), 3.09(brs, 2H), 2.95(brs, 2H), 2.54(brs, 1H), 2.41(brs, 1H), 2.18(brs, 2H), 1.80(brs, 2H), 1.06(t, 3H); (Yield: 28 %)

### Example 188. (S)-N-{4-methyl-3-(trifluoromethyl)phenyl}-4-{3-(propylamino)pyrrolidin-1-yl}-6,7-dihydro-5H-cyclopenta[d]pyrimidin-2-amine dihydrochloride

¹H NMR(400MHz, CD₃OD) δ 8.10-8.00(m, 1H), 7.59(d, 1H), 7.42(t, 1H), 4.50-3.70(m, 5H), 3.21(brs, 2H), 3.08(brs, 2H), 2.96(brs, 2H), 2.55-2.48(m, 1H), 2.47(s, 3H), 2.31(brs, 1H), 2.20(brs, 2H), 1.77(brs, 2H), 1.05(t, 3H); (Yield: 45 %)

### Example 189. (S)-5-chloro-N¹-[4-{3-(propylamino)pyrrolidin-1-yl}-6,7-dihydro-5H-cyclopenta[d]pyrimidin-2-yl]benzene-1,3-diamine dihydrochloride

¹H NMR(400MHz, CD₃OD) δ 7.85-7.60(m, 2H), 7.16(s, 1H), 4.50-3.70(m, 5H), 3.22(brs, 2H), 3.10(brs, 2H), 2.97(d, 2H), 2.54(brs, 1H), 2.37(brs, 1H), 2.20(brs, 2H), 1.82(t, 2H), 1.06(t, 3H); (Yield: 32 %)

### Examples 190 to 198

The titled compounds of Examples 190 to 198 were prepared in the same manner as Example 168 by reacting 3,5-diaminobenzonitrile, 5-(trifluoromethyl)-1,3-phenylenediamine, 2-(trifluoromethyl)-1,4-phenylenediamine, 4-chloro-1,3-diaminobenzene, 5-amino-2-methylbenzonitrile, 4-methyl-3-(trifluoromethyl)aniline, 4-fluoro-3-trifluoromethylphenylamine, 5-chloro-1,3-diaminobenzene or 2-nitro-1,4-phenylenediamine respectively with *(R)*-*N*-{1-(2-chloro-6,7-dihydro-5*H*-cyclopenta[*d*]pyrimidin-4-yl)piperidin-3-yl}acetamide prepared in Reference Example 56.

### Example 190. (R)-N-(1-[2-{(3-amino-5-cyanophenyl)amino}-6,7-dihydro-5H-cyclopenta[d]pyrimidin-4-yl]piperidin-3-yl)acetamide hydrochloride

¹H NMR(400MHz, CD₃OD) δ 7.06(s, 1H), 6.99(s, 1H), 6.71(s, 1H), 4.59(d, 1H), 4.37(d, 1H), 3.85(brs, 1H), 3.37(d, 1H), 3.19(t, 2H), 3.10-3.00(m, 1H), 2.91(t, 2H), 2.19(t, 2H), 2.03(brs, 1H), 1.97(s, 3H), 1.93(brs, 1H), 1.68(q, 2H); (Yield: 35 %)

### Example 191. (R)-N-{1-(2-[{3-amino-5-(trifluoromethyl)phenyl}amino]-6,7-dihydro-5H-cyclopenta[d]pyrimidin-4-yl)piperidin-3-yl}acetamide hydrochloride

¹H NMR(400MHz, CD₃OD) δ 7.09(s, 1H), 6.93(s, 1H), 6.73(s, 1H), 4.54(d, 1H), 4.38(d, 1H), 3.85(s, 1H), 3.37(t, 1H), 3.30-3.15(m, 2H), 3.10-3.00(m, 1H), 2.91(t, 2H), 2.18(t, 2H), 2.03(brs, 1H), 1.95(s, 3H), 1.90(brs, 1H), 1.67(brs, 2H); (Yield: 40 %)

### Example 192. (R)-N-{1-(2-[{4-amino-3-(trifluoromethyl)phenyl}amino]-6,7-dihydro-5H-cyclopenta[d]pyrimidin-4-yl)piperidin-3-yl}acetamide hydrochloride

¹H NMR(400MHz, CD₃OD) δ 7.51(s, 1H), 7.29(d, 1H), 6.87(d, 1H), 4.46(d, 1H), 4.35(d, 1H), 3.82(s, 1H), 3.35-3.30(m, 1H), 3.20(t, 2H), 3.05-2.95(m, 1H), 2.87(t, 2H), 2.16(t, 2H), 2.01(brs, 1H), 1.94(s, 3H), 1.87(brs, 1H), 1.63(t, 2H); (Yield: 25 %)

### Example 193. (R)-N-(1-[2-{(3-amino-4-chlorophenyl)amino}-6,7-dihydro-5H-cyclopenta[d]pyrimidin-4-yl]piperidin-3-yl)acetamide hydrochloride

¹H NMR(400MHz, CD₃OD) δ 7.17(d, 1H), 6.96(s, 1H), 6.65(d, 1H), 4.54(brs, 1H), 4.35(brs, 1H), 3.85(brs, 1H), 3.36(brs, 1H), 3.20(t, 2H), 3.03(brs, 1H), 2.88(brs, 2H), 2.16(t, 2H), 2.03(brs, 1H), 1.96(s, 3H), 1.91(brs, 1H), 1.66(brs, 2H); (Yield: 23 %)

### Example 194. (R)-N-(1-[2-{(3-cyano-4-methylphenyl)amino}-6,7-dihydro-5H-cyclopenta[d]pyrimidin-4-yl]piperidin-3-yl)acetamide hydrochloride

¹H NMR(400MHz, CD₃OD) δ 7.89(s, 1H), 7.60(d, 1H), 7.43(d, 1H), 4.51(d, 1H), 4.35(d, 1H), 3.84(s, 1H), 3.36(t, 1H), 3.25-3.15(m, 2H), 3.10-3.00(m, 1H), 2.93(t, 2H), 2.52(s, 3H), 2.18(t, 2H), 2.03(brs, 1H), 1.95(s, 3H), 1.92(brs, 1H), 1.66(brs, 2H); (Yield: 46 %)

### Example 195. (R)-N-{1-(2-[{4-methyl-3-(trifluoromethyl)phenyl}amino]-6,7-dihydro-5H-cyclopenta[d]pyrimidin-4-yl)piperidin-3-yl}acetamide hydrochloride

¹H NMR(400MHz, CD₃OD) δ 7.91(s, 1H), 7. 54 (d, 1H), 7.39(d, 1H), 4.49(d, 1H), 4.38(d, 1H), 3.84(brs, 1H), 3.35(t, 1H), 3.30-3.15(m, 2H), 3.10-3.00(m, 1H), 2.93(t, 2H), 2.47(s, 3H), 2.18(t, 2H), 2.02(brs, 1H), 1.94(s, 3H), 1.89(brs, 1H), 1.65(t, 2H); (Yield: 44 %)

### Example 196. (R)-N-{1-(2-[{4-fluoro-3-(trifluoromethyl)phenyl}amino]-6,7-dihydro-5H-cyclopenta[d]pyrimidin-4-yl)piperidin-3-yl}acetamide hydrochloride

¹H NMR(400MHz, CD₃OD) δ 7.95(s, 1H), 7.71(brs, 1H), 7.37(t, 1H), 4.49(d, 1H), 4.35(d, 1H), 3.83(brs, 1H), 3.40-3.30(m, 1H), 3.30-3.15(m, 2H), 3.10-3.00(m, 1H), 2.94(t, 2H), 2.19(t, 2H), 2.02(brs, 1H), 1.94(s, 3H), 1.89(brs, 1H), 1.65(t, 2H); (Yield: 51 %)

### Example 197. (R)-N-(1-[2-{(3-amino-5-chlorophenyl)amino}-6,7-dihydro-5H-cyclopenta[d]pyrimidin-4-yl]piperidin-3-yl}acetamide hydrochloride

¹H NMR(400MHz, CD₃OD) δ 6. 76 (s, 1H), 6.67(s, 1H), 6.50 (s, 1H), 4.56(d, 1H), 4.39(d, 1H), 3.86(brs, 1H), 3.40-3.30(m, 1H), 3.25-3.10(m, 2H), 3.10-3.00(m, 1H), 2.89(t, 2H), 2.25-2.15(m, 2H), 2.05(brs, 1H), 1.95(s, 3H), 1.95-1.90(m, 1H), 1.80-1.60(m, 2H); (Yield: 24 %)

### Example 198. (R)-N-(1-[2-{(4-amino-3-nitrophenyl)amino}-6,7-dihydro-5H-cyclopenta[d]pyrimidin-4-yl]piperidin-3-yl}acetamide hydrochloride

¹H NMR(400MHz, CD₃OD) δ 8.33(s, 1H), 7.38(d, 1H), 7.00(d, 1H), 4.55(brs, 1H), 4.42(d, 1H), 3.82(brs, 1H), 3.40-3.30(m, 1H), 3.30-3.10(m, 2H), 3.10-3.00(m, 1H), 2.89(t, 2H), 2.17(t, 2H), 2.03(brs, 1H), 1.94(s, 3H), 1.90(brs, 1H), 1.67(q, 2H); (Yield: 22 %)

### Examples 199 to 203

The titled compounds of Examples 199 to 203 were prepared in the same manner as Example 174 by reacting 3,5-diaminobenzonitrile, 5-(trifluoromethyl)-1,3-phenylenediamine, 2-(trifluoromethyl)-1,4-phenylenediamine, 4-chloro-1,3-diaminobenzene or 2-nitro-1,4-phenylenediamine respectively with (*R*)-*tert*-butyl {1-(2-chloro-6,7-dihydro-5*H*-cyclopenta[*d*]pyrimidin-4-yl)piperidin-3-yl}carbamate prepared in Reference Example 57.

### Example 199. (R)-3-amino-5-[{4-(3-aminopiperidin-1-yl)-6,7-dihydro-5H-cyclopenta[d]pyrimidin-2-yl}amino]benzonitrile dihydrochloride

¹H NMR(400MHz, CD₃OD) δ 7.91(s, 1H), 7.80(s, 1H), 7.41(s, 1H), 4.59(d, 1H), 4.32(brs, 1H), 3.75-3.40(m, 3H), 3.16(brs, 2H), 3.00(brs, 2H), 2.30-2.15(m, 3H), 2.05-1.95(m, 1H), 1.90-1.75(m, 2H); (Yield: 25 %)

### Example 200. (R)-N¹-{4-(3-aminopiperidin-1-yl)-6,7-dihydro-5H-cyclopenta[d]pyrimidin-2-yl}-5-(trifluoromethyl)benzene-1,3-diamine dihydrochloride

¹H NMR(400MHz, CD₃OD) δ 8.07(s, 1H), 7.89(s, 1H), 7.42(s, 1H), 4.56(d, 1H), 4.35(brs, 1H), 3.75-3.35(m, 3H), 3.30-3.10(m, 2H), 3.10-2.95(m, 2H), 2.30-2.15(m, 3H), 2.00-1.90(m, 1H), 1.90-1.70(m, 2H); (Yield: 26 %)

### Example 201. (R)-N¹-{4-(3-aminopiperidin-1-yl)-6,7-dihydro-5H-cyclopenta[d]pyrimidin-2-yl}-3-(trifluoromethyl)benzene-1,4-diamine dihydrochloride

¹H NMR(400MHz, CD₃OD) δ 8.00(s, 1H), 7.76(d, 1H), 7.51(d, 1H), 4.54(d, 1H), 4.35(brs, 1H), 3.75-3.30(m, 3H), 3.30-3.05(m, 2H), 3.05-2.90(m, 2H), 2.30-2.10(m, 3H), 2.00-1.60(m, 3H); (Yield: 18 %)

### Example 202. (R)-N¹-{4-(3-aminopiperidin-1-yl)-6,7-dihydro-5H-cyclopenta[d]pyrimidin-2-yl}-4-chlorobenzene-1,3-diamine dihydrochloride

¹H NMR(400MHz, CD₃OD) δ 7. 72 (s, 1H), 7. 62 (s, 1H), 7.52(d, 1H), 4.54(d, 1H), 4.24(brs, 1H), 3.80-3.40(m, 3H), 3.30-3.05(m, 2H), 3.05-2.95(m, 2H), 2.30-2.15(m, 3H), 2.00-1.65(m, 3H); (Yield: 20 %)

### Example 203. (R)-N¹-{4-(3-aminopiperidin-1-yl)-6,7-dihydro-5H-cyclopenta[d]pyrimidin-2-yl}-3-nitrobenzene-1,4-diamine dihydrochloride

¹H NMR(400MHz, CD₃OD) δ 8.70(s, 1H), 7.37(d, 1H), 7.05(d, 1H), 4.68(brs, 1H), 4.35(brs, 1H), 3.70-3.30(m, 3H), 3.25-3.05(m, 2H), 3.05-2.95(m, 2H), 2.30-2.10(m, 3H), 1.96(brs, 1H), 1.76(brs, 2H); (Yield: 15 %)

### Examples 204 to 211

The titled compounds of Examples 204 to 211 were prepared in the same manner as Example 174 by reacting 3,5-diaminobenzonitrile, 5-(trifluoromethyl)-1,3-phenylenediamine, 2-(trifluoromethyl)-1,4-phenylenediamine, 4-chloro-1,3-diaminobenzene, 5-chloro-1,3-diaminobenzene, 5-amino-2-methylbenzonitrile, 4-methyl-3-(trifluoromethyl)aniline or 2-nitro-1,4-phenylenediamine respectively with (*R*)-*tert*-butyl {1-(2-chloro-6,7-dihydro-5*H*-cyclopenta[*d*]pyrimidin-4-yl)piperidin-3-yl}(methyl)carbamate prepared in Reference Example 58.

### Example 204. (R)-3-amino-5-([4-{3-(methylamino)piperidin-1-yl}-6,7-dihydro-5H-cyclopenta[d]pyrimidin-2-yl]amino)benzonitrile dihydrochloride

¹H NMR(400MHz, CD₃OD) δ 7.89(s, 1H), 7.80(s, 1H), 7.44(s, 1H), 4.50(d, 1H), 4.16(brs, 1H), 3.86(brs, 1H), 3.66(brs, 1H), 3.43(brs, 1H), 3.25-3.10(m, 2H), 3.00(brs, 2H), 2.69(s, 3H), 2.30-2.15(m, 3H), 2.05-1.65(m, 3H); (Yield: 42 %)

### Example 205. (R)-N¹-[4-{3-(methylamino)piperidin-1-yl}-6,7-dihydro-5H-cyclopenta[d]pyrimidin-2-yl]-5-(trifluoromethyl)benzene-1,3-diamine dihydrochloride

¹H NMR(400MHz, CD₃OD) δ 7.96(s, 1H), 7.77(s, 1H), 7.41(s, 1H), 4.45(brs, 1H), 4.30-3.60(m, 3H), 3.41(brs, 1H), 3.25-3.10(m, 2H), 3.05-2.95(m, 2H), 2.64(s, 3H), 2.23(brs, 3H), 2.05-1.65(m, 3H); (Yield: 40 %)

### Example 206. (R)-N¹-[4-{3-(methylamino)piperidin-1-yl}-6,7-dihydro-5H-cyclopenta[d]pyrimidin-2-yl]-3-(trifluoromethyl)benzene-1,4-diamine dihydrochloride

¹H NMR(400MHz, CD₃OD) δ 7.83(s, 1H), 7.66(d, 1H), 7.36(d, 1H), 4.39(brs, 1H), 4.20-3.50(m, 3H), 3.35(brs, 1H), 3.14(brs, 2H), 2.00-2.90(m, 2H), 2.62(s, 3H), 2.30-2.10(m, 3H), 2.05-1.65(m, 3H); (Yield: 25 %)

### Example 207. (R)-4-chloro-N¹-[4-{3-(methylamino)piperidin-1-yl}-6,7-dihydro-5H-cyclopenta[d]pyrimidin-2-yl]benzene-1,3-diamine dihydrochloride

¹H NMR(400MHz, CD₃OD) δ 7.58(s, 2H), 7.41(d, 1H), 4.41(brs, 1H), 4.25-3.55(m, 3H), 3.38(brs, 1H), 3.14(d, 2H), 2.97(t, 2H), 2.64(s, 3H), 2.30-2.10(m, 3H), 2.05-1.65(m, 3H); (Yield: 22 %)

### Example 208. (R)-5-chloro-N¹-[4-{3-(methylamino)piperidin-1-yl}-6,7-dihydro-5H-cyclopenta[d]pyrimidin-2-yl]benzene-1,3-diamine dihydrochloride

¹H NMR(400MHz, CD₃OD) 5 7.65(s, 1H), 7.48(s, 1H), 7.19(s, 1H), 4.48(d, 1H), 4.16(brs, 1H), 3.89(brs, 1H), 3.63(brs, 1H), 3.41(brs, 1H), 3.16(d, 2H), 2.98(t, 2H), 2.67(s, 3H), 2.30-2.15(m, 3H), 2.05-1.65(m, 3H); (Yield: 29 %)

### Example 209. (R)-2-methyl-5-([4-{3-(methylamino)piperidin-1-yl}-6,7-dihydro-5H-cyclopenta[d]pyrimidin-2-yl]amino)benzonitrile dihydrochloride

¹H NMR(400MHz, CD₃OD) δ 7.85(s, 1H), 7.63(d, 1H), 7.49(d, 1H), 4.44(brs, 1H), 4.12(brs, 1H), 3.81(brs, 1H), 3.64(brs, 1H), 3.35(brs, 1H), 3.14(d, 2H), 2.96(t, 2H), 2.64(s, 3H), 2.53(s, 3H), 2.30-2.15(m, 3H), 2.05-1.65(m, 3H); (Yield: 40 %)

### Example 210. (R)-N-{4-methyl-3-(trifluoromethyl)phenyl}-4-{3-(methylamino)piperidin-1-yl}-6,7-dihydro-5H-cyclopenta[d]pyrimidin-2-amine dihydrochloride

¹H NMR(400MHz, CD₃OD) δ 7.82 (s, 1H), 7.58 (s, 1H), 7.45(d, 1H), 4.40(brs, 1H), 4.12(brs, 1H), 3.87(brs, 1H), 3.63(brs, 1H), 3.31(brs, 1H), 3.14(brs, 2H), 2.95(d, 2H), 2.59(s, 3H), 2.48(s, 3H), 2.30-2.15(m, 3H), 2.00-1.65(m, 3H); (Yield: 38 %)

### Example 211. (R)-N¹-[4-{3-(methylamino)piperidin-1-yl}-6,7-dihydro-5H-cyclopenta[d]pyrimidin-2-yl]-3-nitrobenzene-1,4-diamine dihydrochloride

¹H NMR(400MHz, CD₃OD) δ 8.30(s, 1H), 7.40(d, 1H), 7.06(d, 1H), 4.44(brs, 1H), 4.30-3.50(m, 3H), 3.35-3.30(m, 1H), 3.20-3.05(m, 2H), 3.05-2.90(m, 2H), 2.65(s, 3H), 2.30-2.10(m, 3H), 2.05-1.65(m, 3H); (Yield: 26 %)

### Examples 212 to 218

The titled compounds of Examples 212 to 218 were prepared in the same manner as Example 174 by reacting 3,5-diaminobenzonitrile, 5-(trifluoromethyl)-1,3-phenylenediamine, 4-chloro-1,3-diaminobenzene, 5-chloro-1,3-diaminobenzene, 5-amino-2-methylbenzonitrile, 4-methyl-3-(trifluoromethyl)aniline or 2-nitro-1,4-phenylenediamine respectively with *(R)-tert-*butyl {1-(2-chloro-5,6,7,8-tetrahydroquinazolin-4-yl)piperidin-3-yl}(methyl)carbamate prepared in Reference Example 59.

### Example 212. (R)-3-amino-5-([4-{3-(methylamino)piperidin-1-yl}-5,6,7,8-tetrahydroquinazolin-2-yl]amino)benzonitrile dihydrochloride

¹H NMR(400MHz, CD₃OD) 5 7.72 (s, 1H), 7.59 (s, 1H), 7.26 (s, 1H), 4.45(d, 1H), 4.08(d, 1H), 3.70-3.35(m, 3H), 2.82(t, 2H), 2.80-2.60(m, 5H), 2.30-2.20(m, 1H), 2.05-1.70(m, 5H); (Yield: 35 %)

### Example 213. (R)-N¹-[4-{3-(methylamino)piperidin-1-yl}-5,6,7,8-tetrahydroquinazolin-2-yl]-5-(trifluoromethyl)benzene-1,3-diamine dihydrochloride

¹H NMR(400MHz, CD₃OD) δ 7.91(s, 1H), 7.80(s, 1H), 7.39(s, 1H), 4.40(d, 1H), 4.08(d, 1H), 3.68(t, 1H), 3.50-3.30(m, 2H), 2.83(t, 2H), 2.80-2.60(m, 5H), 2.25(brs, 1H), 2.05-1.70(m, 7H); (Yield: 30 %)

### Example 214. (R)-4-chloro-N¹-[4-{3-(methylamino)piperidin-1-yl}-5,6,7,8-tetrahydroquinazolin-2-yl]benzene-1,3-diamine dihydrochloride

¹H NMR(400MHz, CD₃OD) δ 7.65-7.55(m, 2H), 7.38 (t, 1H), 4.37(d, 1H), 4.02(d, 1H), 3.65(t, 1H), 3.48(t, 1H), 3.36(brs, 1H), 2.80(t, 2H), 2.75-2.55(m, 5H), 2.23(brs, 1H), 2.00-1.70(m, 7H); (Yield: 20 %)

### Example 215. (R)-5-chloro-N¹-[4-{3-(methylamino)piperidin-1-yl}-5,6,7,8-tetrahydroquinazolin-2-yl]benzene-1,3-diamine dihydrochloride

¹H NMR(400MHz, CD₃OD) δ 7.34 (s, 1H), 7.17(s, 1H), 6.94 (s, 1H), 4.42(d, 1H), 4.08(d, 1H), 3.60(t, 1H), 3.46(t, 1H), 3.35(brs, 1H), 2.80(t, 2H), 2.75-2.55(m, 5H), 2.30-2.20(m, 1H), 2.00-1.70(m, 7H); (Yield: 26 %)

### Example 216. (R)-2-methyl-5-([4-{3-(methylamino)piperidin-1-yl}-5,6,7,8-tetrahydroquinazolin-2-yl]amino)benzonitrile dihydrochloride

¹H NMR(400MHz, CD₃OD) δ 7. 91 (s, 1H), 7.64(d, 1H), 7.48(d, 1H), 4.40(d, 1H), 4.08(d, 1H), 3.55(t, 1H), 3.43(t, 1H), 3.34(brs, 1H), 2.80(t, 2H), 2.78-2.55(m, 5H), 2.53(s, 3H), 2.24(brs, 1H), 2.00-1.70(m, 7H); (Yield: 46 %)

### Example 217. (R)-N-{4-methyl-3-(trifluoromethyl)phenyl}-4-{3-(methylamino)piperidin-1-yl}-5,6,7,8-tetrahydroquinazolin-2-amine dihydrochloride

¹H NMR(400MHz, CD₃OD) δ 7.85(s, 1H), 7.60(d, 1H), 7.45 (d, 1H), 4.35(d, 1H), 4.08(d, 1H), 3.59(t, 1H), 3.45(t, 1H), 3.31(brs, 1H), 2.80(t, 2H), 2.78-2.55(m, 5H), 2.48(s, 3H), 2.22(brs, 1H), 2.00-1.70(m, 7H); (Yield: 41 %)

### Example 218. (R)-N¹-[4-{3-(methylamino)piperidin-1-yl}-5,6,7,8-tetrahydroquinazolin-2-yl]-3-nitrobenzene-1,4-diamine dihydrochloride

¹H NMR(400MHz, CD₃OD) δ 8.37(s, 1H), 7.39(d, 1H), 7.05(d, 1H), 4.40(d, 1H), 4.08(d, 1H), 3.52(t, 1H), 3.45(t, 1H), 3.33(brs, 1H), 2.77(t, 2H), 2.75-2.55(m, 5H), 2.23(brs, 1H), 2.00-1.65(m, 7H); (Yield: 22 %)

### Examples 219 to 224

The titled compounds of Examples 219 to 224 were prepared in the same manner as Example 168 by reacting 3,5-diaminobenzonitrile, 5-(trifluoromethyl)-1,3-phenylenediamine, 2-(trifluoromethyl)-1,4-phenylenediamine, 5-chloro-1,3-diaminobenzene, 4-methyl-3-(trifluoromethyl)aniline or 2-nitro-1,4-phenylenediamine respectively with *(R)-1-*(2-chloro-6,7-dihydro-5*H-*cyclopenta[*d*]pyrimidin-4-yl)-*N*-methylpiperidine-3-carboxamide prepared in Reference Example 60.

### Example 219. (R)-1-[2-{(3-amino-5-cyanophenyl)amino}-6,7-dihydro-5H-cyclopenta[d]pyrimidin-4-yl]-N-methylpiperidine-3-carboxamide hydrochloride

¹H NMR(400MHz, CD₃OD) δ 7.03(d, 2H), 6.74(s, 1H), 4.59(brs, 1H), 4.38(d, 1H), 3.39(t, 2H), 3.08(brs, 2H), 2.91(brs, 2H), 2.72(s, 3H), 2.52(brs, 1H), 2.25-2.15(m, 2H), 2.10-2.00(m, 1H), 1.95-1.80(m, 2H), 1.70-1.60(m, 1H); (Yield: 47 %)

### Example 220. (R)-1-(2-[{3-amino-5-(trifluoromethyl)phenyl}amino]-6,7-dihydro-5H-cyclopenta[d]pyrimidin-4-yl)-N-methylpiperidine-3-carboxamide hydrochloride

¹H NMR(400MHz, CD₃OD) δ 7.10(s, 1H), 6.93(s, 1H), 6.74(s, 1H), 4.60(brs, 1H), 4.40(d, 1H), 3.50-3.30(m, 2H), 3.07(brs, 2H), 2.91(t, 2H), 2.70(s, 3H), 2.51(brs, 1H), 2.17(t, 2H), 2.01(brs, 1H), 1.95-1.80(m, 2H), 1.70-1.60(m, 1H); (Yield: 45 %)

### Example 221. (R)-1-(2-[{4-amino-3-(trifluoromethyl)phenyl}amino]-6,7-dihydro-5H-cyclopenta[d]pyrimidin-4-yl)-N-methylpiperidine-3-carboxamide hydrochloride

¹H NMR(400MHz, CD₃OD) δ 7.50(s, 1H), 7.29(d, 1H), 6.86(d, 1H), 4.54(brs, 1H), 4.39(d, 1H), 3.45-3.30(m, 2H), 3.05(brs, 2H), 2.88(brs, 2H), 2.71(s, 3H), 2.47(brs, 1H), 2.25-2.15(m, 2H), 1.98(brs, 1H), 1.95-1.80(m, 2H), 1.70-1.50(m, 1H); (Yield: 35 %)

### Example 222. (R)-1-[2-{(3-amino-5-chlorophenyl)amino}-6,7-dihydro-5H-cyclopenta[d]pyrimidin-4-yl]-N-methylpiperidine-3-carboxamide hydrochloride

¹H NMR(400MHz, CD₃OD) δ 6.80(s, 1H), 6.67(s, 1H), 6.48(s, 1H), 4.65(brs, 1H), 4.40(d, 1H), 3.45-3.30(m, 2H), 3.05(brs, 2H), 2.89(brs, 2H), 2.72(s, 3H), 2.51(brs, 1H), 2.16(brs, 2H), 2.01(brs, 1H), 2.00-1.80(m, 2H), 1.64(brs, 1H); (Yield: 38 %)

### Example 223. (R)-N-methyl-1-(2-[{4-methyl-3-(trifluoromethyl)phenyl}amino]-6,7-dihydro-5H-cyclopenta[d]pyrimidin-4-yl)piperidine-3-carboxamide hydrochloride

¹H NMR(400MHz, CD₃OD) δ 7.89(s, 1H), 7.54(d, 1H), 7.38(d, 1H), 4.51(brs, 1H), 4.37(d, 1H), 3.55-3.30(m, 2H), 3.09(brs, 2H), 2.93(t, 2H), 2.71(s, 3H), 2.60-2.40(m, 4H), 2.18(t, 2H), 1.99(brs, 1H), 1.95-1.80(m, 2H), 1.70-1.60(m, 1H); (Yield: 47 %)

### Example 224. (R)-1-[2-{(4-amino-3-nitrophenyl)amino}-6,7-dihydro-5H-cyclopenta[d]pyrimidin-4-yl]-N-methylpiperidine-3-carboxamide hydrochloride

¹H NMR(400MHz, CD₃OD) δ 8.29(s, 1H), 7.38(d, 1H), 7.00(d, 1H), 4.55(brs, 1H), 4.39(d, 1H), 3.45-3.30(m, 2H), 3.06(brs, 2H), 2.89(t, 2H), 2.71(s, 3H), 2.47(brs, 1H), 2.17(t, 2H), 1.99(d, 1H), 1.95-1.80(m, 2H), 1.70-1.60(m, 1H); (Yield: 25 %)

### Test Example 1. Evaluation of agonistic activity in CHO-K1 cells expressing human 5-HT₄₍ₐ₎

As CHO-K1 cells stably expressing human 5-HT₄₍ₐ₎, we used the GeneBlAzer HTR4-CRE-bla CHO-K1 cells (Invitrogen corp.). The cells were cultured in DMEM medium supplemented with 10 % bovine fetal serum (FBS), 25 mM HEPES (pH 7.4), 600 µg/ml Hygromycin B, 0.1 mM non-essential amino acids, 100 unit/ml penicillin and 100 µg/ml streptomycin under the condition of 37 °C and 5 % CO₂. Subcultures were performed three times per a week, each being at less than 80 % confluence. At the previous day before treating test compounds, the cells were collected by using 0.5 % trypsin/EDTA, and then diluted with a DMEM supplemented with 1 % FBS, 25 mM HEPES and 0.1 mM non-essential amino acids into 3.125 x 10⁵ cells/ml. 32 µl of the diluted cells were added into 384-well plate (10,000 cells/well) and then incubated overnight. The compounds to be used as a test material and a control drug were prepared as 500X of the various final treating concentrations of the drug with 100 % DMSO, and then treated to the medium after diluting them to 100-folds to be 1 % of the final DMSO concentration equally. After culturing overnight, 8 µl of the medium having 1 % of DMSO was added into the cell-free control well and the non-stimulating control well, respectively. 8 µl of the control drug or the test material (which had been prepared by diluting 100-folds with the medium as mentioned in the above) having 1 % of DMSO, were added to the respective remaining wells. After culturing in an incubator for 5 hours, the wells of the 384-plate were treated with the substrate solution (8 µl/well) prepared according to the vendor's instruction (i.e. Invitrogen's instruction), and then incubated in the dark room for additional 2 hours. Agonistic activities on 5-HT₄ receptor were evaluated, on the basis of fluorescent values of the cleavage-products, which is generated cAMP-concentration dependently per equal time by beta-lactamase. After exciting to 410 nm of wavelength by using Genios Pro Fluorescence Detector, we measured the fluorescence values at two emission wavelengths (first wavelength: 465 nm, second wavelength: 535 nm). Data were analyzed on the basis of the ratio of fluorescence intensities of each well at the respective wavelengths. For all plates, the concentrations-response curve (1 pM - 100 nM) of the control drug (Tegaserod) was included. Each EC₅₀ values of the test compounds were calculated by nonlinear regression analysis using GraphPad Prism program, based on the concentration-reactivity values according to 8-different concentrations of the test compounds. The results were represented in Table 1 below.

**[Table 1]**

| Example | EC50 (nM) |
|---|---|
| 7 | 0.03 |
| 14 | 0.02 |
| 21 | 0.01 |
| 28 | 0.39 |
| 31 | 0.03 |
| 37 | 0.03 |
| 41 | 0.0024 |
| 46 | 0.05 |
| 64 | 0.03 |
| 68 | 0.08 |
| 80 | 0.04 |
| 81 | 0.06 |
| 85 | 0.04 |
| 88 | 0.01 |
| 105 | 0.01 |
| 111 | 0.00095 |
| 123 | 0.01 |
| 133 | 0.03 |
| 141 | 0.01 |
| 147 | 0.01 |
| 159 | 0.01 |
| 167 | 0.09 |
| 168 | 0.01 |
| 174 | 0.02 |
| 184 | 0.01 |
| 198 | 0.01 |
| 199 | 0.02 |
| 204 | 0.01 |
| 213 | 0.016 |
| 224 | 0.0087 |

As shown in Table 1, the compounds of the present invention have excellent activities as a 5-HT₄ receptor agonist, and thus they can be usefully applied for preventing or treating of the dysfunction in gastrointestinal motility.

### [Industrial Applicability]

The compound according to the present invention, i.e., the bicyclic derivative comprising pyrimidine ring or pharmaceutically acceptable salt thereof act as a 5-HT₄ receptor agonist, and thus can be usefully applied to the prevention or treatment of gastrointestinal diseases such as dysfunction in gastrointestinal motility, for example, gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, diabetic gastric atony and the like.

## Claims

1. A compound of formula 1 as below, or a pharmaceutically acceptable salt thereof: 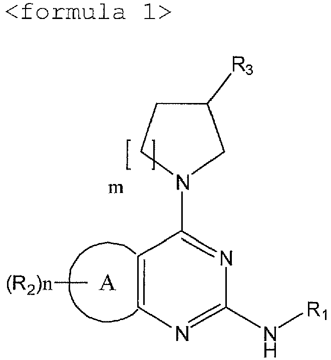 wherein,
R₁ is phenyl group; or pyridine group (wherein the phenyl group or pyridine group can be unsubstituted or substituted with one or more of substituents selected from the group consisting of halogen, amino, nitro, cyano, C₁₋₅ alkyl, C₁₋₅ alkyl substituted with halogen, C₁₋₅ alkoxy, C₁₋₅ alkoxy substituted with halogen, and hydroxy),
R₂ is each independently hydrogen; halogen; amino; mono- or di- C₁₋₅ alkyl amino; nitro; cyano; C₁₋₅ alkyl; C₁₋₅ alkyl substituted with halogen; C₁₋₅ alkoxy; C₁₋₅ alkoxy substituted with halogen; C₁₋₅ alkoxy carbonyl; hydroxy; or hydroxycarbonyl,
R₃ is a substituent selected from the group consisting of formulae I to III, as below; 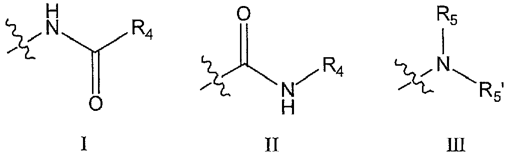
R₄ is C₁₋₅ alkyl; C₁₋₅ alkyl substituted with phenyl, thiophene (wherein the phenyl group or thiophen group can be unsubstituted or substituted with one or more of substituents selected from the group consisting of halogen, amino, C₁₋₅ alkyl, C₁₋₅ alkoxy, and hydroxy), or di-C₁₋₅ alkyl amino group; or C₁₋₅ alkoxy,
R₅ and R₅' are each independently hydrogen; C₁₋₈ alkyl; C₁₋₈ alkyl substituted with phenyl or C₃₋₈ cycloalkyl (wherein the phenyl group can be unsubstituted or substituted with one or more of substituents selected from the group consisting of halogen, amino, C₁₋₅ alkyl, C₁₋₅ alkoxy and hydroxy) ; or C₃₋₈ cycloalkyl,
ring A is C₅₋₆ cycloalkyl; phenyl; or 5- to 6-membered heteroaryl comprising nitrogen atom,
m is 1 or 2,
n is integer of 0 to 2.

2. The compound of formula 1, or the pharmaceutically acceptable salt thereof according to the claim 1, wherein:
R₁ is phenyl group; or pyridine group (wherein the phenyl group or pyridine group can be unsubstituted or substituted with one or more of substituents selected from the group consisting of halogen, amino, nitro, cyano, C₁₋₅ alkyl, C₁₋₅ alkyl substituted with halogen, and C₁₋₅ alkoxy),
R₂ is each independently hydrogen; halogen; C₁-₅ alkyl; C₁₋₅ alkyl substituted with halogen; or C₁₋₅ alkoxy,
R₃ is a substituent selected from the group consisting of formulae I to III as below; 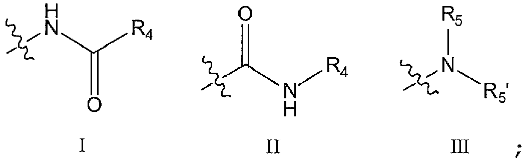
R₄ is C₁₋₅ alkyl; C₁₋₅ alkyl substituted with phenyl, thiophene, or di-C₁₋₅ alkyl amino group; or C₁₋₅ alkoxy,
R₅ and R₅' are each independently hydrogen; C₁₋₈ alkyl; C₁₋₈ alkyl substituted with phenyl or C₃₋₈ cycloalkyl; or C₃₋₈ cycloalkyl,
ring A is C₅₋₆ cycloalkyl; phenyl; or 5- to 6-membered heteroaryl comprising nitrogen atom,
m is 1 or 2,
n is integer of 0 to 2.

3. The compound of formula 1, or the pharmaceutically acceptable salt thereof according to claim 1, selected from the group consisting of:
*(S)-N*¹-{4-(3-aminopyrrolidin-1-yl)-6,7-dimethoxyquinazolin-2-yl}-4-chlorobenzene-1,3-diamine dihydrochloride;
*(S)-N*-{6,7-dimethoxy-4-(3-methylaminopyrrolidin-1-yl)-quinazolin-2-yl}-5-trifluoromethyl-benzene-1,3-diamine;
*(S)*-3-amino-5-{4-(3-ethylaminopyrrolidin-1-yl)-6,7-dimethoxyquinazolin-2-ylamino}-benzonitrile;
*(S)*-*N*-{4-(3-ethylaminopyrrolidin-1-yl)-6,7-dimethoxyquinazolin-2-yl}-5-trifluoromethylbenzene-1,3-diamine;
*(S)-N-*(1-[2-{(3-amino-5-cyanophenyl)amino}-6,7-dimethoxyquinazolin-4-yl]pyrrolidin-3-y1)acetamide;
*(S)-N-*(1-[2-{(3-cyano-4-methylphenyl)amino}-6,7-dimethoxyquinazolin-4-yl]pyrrolidin-3-yl)acetamide;
(*S*)-*N*-{1-(2-[{3-amino-5-(trifluoromethyl)phenyl}amino]-6,7-dimethoxyquinazolin-4-yl)pyrrolidin-3-yl}acetamide;
*(R)-N-*(1-[2-{(3-cyano-4-fluorophenyl)amino}-6,7-dimethoxyquinazolin-4-yl]piperidin-3-yl)acetamide;
*(R)*-*N*-(1-[2-{(3-cyano-4-methylphenyl)amino}-6,7-dimethoxyquinazolin-4-yl]piperidin-3-yl)acetamide;
*(R)-N*-(1-[2-{(3-amino-5-cyanophenyl)amino}-6,7-dimethoxyquinazolin-4-yl]piperidin-3-yl)acetamide;
*(R)*-*N*-{1-(2-[{4-amino-3-(trifluoromethyl)phenyl}amino]-6,7-dimethoxyquinazolin-4-yl)piperidin-3-yl}acetamide;
(*R*)-*N*-{1-(2-[{3-amino-5-(trifluoromethyl)phenyl}amino]-6,7-dimethoxyquinazolin-4-yl)piperidin-3-yl}acetamide;
(*S*)-*N*-(1-[2-{(3-amino-5-cyanophenyl)amino}-6-methoxyquinazolin-4-yl]pyrrolidin-3-yl)acetamide;
(*S*)-*N*-{1-(2-[{3-amino-5-(trifluoromethyl)phenyl}amino]-6-methoxyquinazolin-4-yl)pyrrolidin-3-yl}acetamide;
(*S*)-*N*¹-[6-methoxy-4-{3-(methylamino)pyrrolidin-1-yl]quinazolin-2-yl]-5-(trifluoromethyl)benzene-1,3-diamine;
*(S)*-3-amino-5-([6-methoxy-4-{3-(methylamino)pyrrolidin-1-yl}quinazolin-2-yl]amino)benzonitrile;
*(S)*-3-amino-5-([4-{3-(ethylamino)pyrrolidin-1-yl}-6-methoxyquinazolin-2-yl]amino)benzonitrile;
*(S)*-*N*¹-[4-{3-(ethylamino)pyrrolidin-1-yl}-6-methoxyquinazolin-2-yl]-5-(trifluoromethyl)benzene-1,3-diamine;
(*S*)-*N*¹-[4-{3-(ethylamino)pyrrolidin-1-yl}-6-methoxyquinazolin-2-yl]-3-(trifluoromethyl)benzene-1,4-diamine;
*(R)-N-*{1-(2-[{3-amino-5-(trifluoromethyl)phenyl}amino]-6-methoxyquinazolin-4-yl)piperidin-3-yl}acetamide;
*(R)-N-*(1-[2-{(3-amino-5-cyanophenyl)amino}-6-methoxyquinazolin-4-yl]piperidin-3-yl)acetamide;
*(R)-N-*(1-[2-{(4-amino-3-cyanophenyl)amino}-6-methoxyquinazolin-4-yl]piperidin-3-yl)acetamide;
*(R)-N-*(1-[2-{(3-cyano-4-methylphenyl)amino}-6-methoxyquinazolin-4-yl]piperidin-3-yl)acetamide;
(*R*)-*N*-{1-(2-[{4-amino-3-(trifluoromethyl)phenyl}amino]-6-methoxyquinazolin-4-yl)piperidin-3-yl}acetamide;
(*S*)-*N*¹-[4-{3-(methylamino)pyrrolidin-l-yl}-7-(trifluoromethyl)quinazolin-2-yl]-5-(trifluoromethyl)benzene-1,3-diamine;
*(S)-N*¹-[4-{3-(ethylamino)pyrrolidin-1-yl}-7-(trifluoromethyl)quinazolin-2-yl]-5-(trifluoromethyl)benzene-1,3-diamine;
*(S)*-3-amino-5-([4-{3-(ethylamino)pyrrolidin-1-yl}-7-(trifluoromethyl)quinazolin-2-yl]amino)benzonitrile;
(*S*)-*N*-(1-[2-{(3-amino-5-cyanophenyl)amino}-7-(trifluoromethyl)quinazolin-4-yl]pyrrolidin-3-yl)acetamide;
(*S*)-*N*-{1-(2-[{3-amino-5-(trifluoromethyl)phenyl}amino]-7-(trifluoromethyl)quinazolin-4-yl)pyrrolidin-3-yl}acetamide;
(*S*)-*tert*-butyl 1-{2-(3-amino-5-cyanophenylamino)quinazolin-4-yl}pyrrolidin-3-ylcarbamate hydrochloride;
*(S)*-3-amino-5-{4-(3-aminopyrrolidin-1-yl)quinazolin-2-ylamino}benzonitrile dihydrochloride;
*(S)-N*¹-{4-(3-aminopyrrolidin-1-yl)quinazolin-2-yl}-5-(trifluoromethyl)benzene-1,3-diamine dihydrochloride;
(*S*)-*N*-[1-{2-(3-amino-5-cyanophenylamino)quinazolin-4-yl}pyrrolidin-3-yl]propionamide hydrochloride;
*(S)-N-*[1-{2-(3-amino-5-cyanophenylamino)quinazolin-4-yl}pyrrolidin-3-yl]pentanamide;
*(S)-N-*[1-{2-(3-amino-5-cyanophenylamino)quinazolin-4-yl}pyrrolidin-3-yl]-2-phenylacetamide;
*(S)-N-*[1-{2-(3-amino-5-cyanophenylamino)quinazolin-4-yl}pyrrolidin-3-yl]-3-phenylpropionamide;
*(S)-N-*[1-{2-(3-amino-5-cyanophenylamino)quinazolin-4-yl}pyrrolidin-3-yl]-2-(thiophen-2-yl)acetamide;
*(S)-N-*[1-{2-(3-amino-5-cyanophenylamino)quinazolin-4-yl}pyrrolidin-3-yl]-2-(dimethylamino)acetamide;
*(S)*-3-amino-5-[4-{3-(propylamino)pyrrolidin-1-yl}quinazolin-2-ylamino]benzonitrile dihydrochloride;
*(S)*-3-amino-5-[4-{3-(butylamino)pyrrolidin-1-yl}quinazolin-2-ylamino]benzonitrile;
*(S)*-3-amino-5-[4-{3-(pentylamino)pyrrolidin-1-yl}quinazolin-2-ylamino]benzonitrile;
*(S)*-3-amino-5-[4-{3-(isopentylamino)pyrrolidin-1-yl}quinazolin-2-ylamino]benzonitrile;
*(S)*-3-amino-5-[4-{3-(cyclopropylmethylamino)pyrrolidin-1-yl}quinazolin-2-ylamino]benzonitrile;
*(S)*-3-amino-5-[4-{3-(neopentylamino)pyrrolidin-1-yl}quinazolin-2-ylamino]benzonitrile;
*(S)*-3-amino-5-[4-{3-(benzylamino)pyrrolidin-1-yl}quinazolin-2-ylamino]benzonitrile;
*(S)*-3-amino-5-[4-{3-(isopropylamino)pyrrolidin-1-yl}quinazolin-2-ylamino]benzonitrile;
*(S)*-3-amino-5-[4-{3-(sec-butylamino)pyrrolidin-1-yl}quinazolin-2-ylamino]benzonitrile;
*(S)*-3-amino-5-[4-{3-(pentan-2-ylamino)pyrrolidin-1-yl}quinazolin-2-ylamino]benzonitrile;
*(S)*-3-amino-5-[4-{3-(hexan-2-ylamino)pyrrolidin-1-yl}quinazolin-2-ylamino]benzonitrile;
*(S)*-3-amino-5-[4-{3-(5-methylhexan-2-ylamino)pyrrolidin-1-yl}quinazolin-2-ylamino]benzonitrile;
*(S)*-3-amino-5-[4-{3-(cyclohexylamino)pyrrolidin-1-yl}quinazolin-2-ylamino]benzonitrile;
*(S)*-*N*¹-[4-{3-(propylamino)pyrrolidin-1-yl}quinazolin-2-yl]-5-(trifluoromethyl)benzene-1,3-diamine dihydrochloride;
*(S)-N*¹-[4-{3-(butylamino)pyrrolidin-1-yl}quinazolin-2-yl]-5-(trifluoromethyl)benzene-1,3-diamine dihydrochloride;
*(S)-N*¹-[4-{3-(pentylamino)pyrrolidin-1-yl}quinazolin-2-yl]-5-(trifluoromethyl)benzene-1,3-diamine dihydrochloride;
*(S)*-3-amino-5-[4-{3-(pentylamino)pyrrolidin-1-yl}quinazolin-2-ylamino]benzonitrile dihydrochloride;
*(S)*-3-amino-5-[4-{3-(hexylamino)pyrrolidin-1-yl}quinazolin-2-ylamino]benzonitrile dihydrochloride;
*(S)-N-*[1-{2-(4-amino-3-cyanophenylamino)-8-methoxyquinazolin-4-yl}pyrrolidin-3-yl]acetamide;
*(S)-N-*[1-{2-(3-amino-5-trifluoromethylphenylamino)-8-methoxyquinazolin-4-yl}pyrrolidin-3-yl]acetamide;
*(S)-N*¹*-*{4-(3-methylaminopyrrolidin-1-yl)-8-methoxyquinazolin-2-yl}-5-trifluoromethylbenzene-1,3-diamine;
*(S)-N*¹-{4-(3-ethylaminopyrrolidin-1-yl)-8-methoxyquinazolin-2-yl}-3-nitrobenzene-1,4-diamine;
*(S)*-3-amino-5-{4-(3-ethylaminopyrrolidin-1-yl)-8-methoxyquinazolin-2-ylamino}benzonitrile;
*(S)*-*N*¹-{4-(3-ethylaminopyrrolidin-1-yl)-8-methoxyquinazolin-2-yl}-5-trifluoromethylbenzene-1,3-diamine;
*(R)-N-*[1-{2-(3-amino-5-cyanophenylamino)-8-methoxyquinazolin-4-yl}piperidin-3-yl]acetamide;
*(R)-N-*[1-{2-(3-amino-5-trifluoromethylphenylamino)-8-methoxyquinazolin-4-yl}piperidin-3-yl]acetamide;
*(R)-N-*[1-{2-(4-amino-3-trifluoromethylphenylamino)-8-methoxyquinazolin-4-yl}piperidin-3-yl]acetamide;
*(S)-N-*[1-{2-(3-amino-5-cyanophenylamino)-5-methylquinazolin-4-yl}pyrrolidin-3-yl]acetamide;
*(S)-N-*(1-[2-{3-amino-5-(trifluoromethyl)phenylamino}-5-methylquinazolin-4-yl]pyrrolidin-3-yl)acetamide;
*(S)*-3-amino-5-[5-methyl-4-{3-(methylamino)pyrrolidin-1-yl}quinazolin-2-ylamino]benzonitrile;
*(S)-N*¹*-*[4-{3-(ethylamino)pyrrolidin-1-yl}-5-methylquinazolin-2-yl]-5-(trifluoromethyl)benzene-1,3-diamine;
*(S)-N*¹-[4-{3-(ethylamino)pyrrolidin-1-yl}-5-methylquinazolin-2-yl]-3-(trifluoromethyl)benzene-1,4-diamine;
*(R)-N-*[1-{2-(3-amino-5-cyanophenylamino)-5-methylquinazolin-4-yl}piperidin-3-yl]acetamide;
*(R)-N-*(1-[2-{3-amino-5-(trifluoromethyl)phenylamino}-5-methylquinazolin-4-yl]piperidin-3-yl)acetamide;
*(S)-N-*[1-{2-(3-amino-5-cyanophenylamino)-8-methylquinazolin-4-yl}pyrrolidin-3-yl]acetamide;
*(S)-N-*(1-[2-{3-amino-5-(trifluoromethyl)phenylamino}-8-methylquinazolin-4-yl]pyrrolidin-3-yl)acetamide;
*(S)*-3-amino-5-[4-{3-(ethylamino)pyrrolidin-1-yl}-8-methylquinazolin-2-ylamino]benzonitrile;
*(S)-N*¹-[4-{3-(ethylamino)pyrrolidin-1-yl}-8-methylquinazolin-2-yl]-5-(trifluoromethyl)benzene-1,3-diamine;
*(R)-N-*(1-[2-{3-amino-5-(trifluoromethyl)phenylamino}-8-methylquinazolin-4-yl]piperidin-3-yl)acetamide;
*(R)-N-*[1-{2-(3-amino-5-cyanophenylamino)-8-methylquinazolin-4-yl}piperidin-3-yl]acetamide hydrochloride;
*(R)-N-*[1-{2-(4-amino-3-cyanophenylamino)-8-methylquinazolin-4-yl}piperidin-3-yl]acetamide hydrochloride;
*(R)-N-*(1-[2-{4-amino-3-(trifluoromethyl)phenylamino}-8-methylquinazolin-4-yl]piperidin-3-yl)acetamide hydrochloride;
*(S)-N-*[1-{2-(3-amino-5-cyanophenylamino)-7-chloroquinazolin-4-yl}pyrrolidin-3-yl]acetamide hydrochloride;
*(S)*-3-amino-5-[7-chloro-4-{3-(methylamino)pyrrolidin-1-yl}quinazolin-2-ylamino]benzonitrile hydrochloride;
*(S)-N*¹*-*[7-chloro-4-{3-(methylamino)pyrrolidin-1-yl}quinazolin-2-yl]-5-(trifluoromethyl)benzene-1,3-diamine hydrochloride;
*(S)*-3-amino-5-[7-chloro-4-{3-(ethylamino)pyrrolidin-1-yl}quinazolin-2-ylamino]benzonitrile hydrochloride;
*(S)*-*N*-[1-{2-(3-amino-5-cyanophenylamino)-7-fluoroquinazolin-4-yl}pyrrolidin-3-yl]acetamide hydrochloride;
*(S)-N-*(1-[2-{3-amino-5-(trifluoromethyl)phenylamino}-7-fluoroquinazolin-4-yl]pyrrolidin-3-yl)acetamide hydrochloride;
*(S)-N-*[1-{2-(3-cyano-4-methylphenylamino)-5,6,7,8-tetrahydroquinazolin-4-yl}pyrrolidin-3-yl]acetamide;
*(S)-N-*[1-{2-(3-amino-5-cyanophenylamino)-5,6,7,8-tetrahydroquinazolin-4-yl}pyrrolidin-3-yl]acetamide;
*(S)-N-*(1-[2-{3-amino-5-(trifluoromethyl)phenylamino}-5,6,7,8-tetrahydroquinazolin-4-yl]pyrrolidin-3-yl)acetamide;
*(S)-N-*[l-f2-(3-amino-4-chlorophenylamino)-5,6,7,8-tetrahydroquinazolin-4-yl}pyrrolidin-3-yl]acetamide;
*(S)*-*N*-(1-[2-{4-methyl-3-(trifluoromethyl)phenylamino}-5,6,7,8-tetrahydroquinazolin-4-yl]pyrrolidin-3-yl)acetamide;
*(S)*-*N*-[1-{2-(4-amino-3-nitrophenylamino)-5,6,7,8-tetrahydroquinazolin-4-yl}pyrrolidin-3-yl]acetamide hydrochloride;
*(S)*-*N*-[1-{2-(3-amino-5-chlorophenylamino)-5,6,7,8-tetrahydroquinazolin-4-yl}pyrrolidin-3-yl]acetamide hydrochloride;
*(S)*-*N*-[1-{2-(3-amino-5-cyanophenylamino)-5,6,7,8-tetrahydroquinazolin-4-yl}pyrrolidin-3-yl]acetamide hydrochloride;
*(S)*-*N*-(1-[2-{3-amino-5-(trifluoromethyl)phenylamino}-5,6,7,8-tetrahydroquinazolin-4-yl]pyrrolidin-3-yl)acetamide hydrochloride;
*(S)*-3-amino-5-{4-(3-aminopyrrolidin-1-yl)-5,6,7,8-tetrahydroquinazolin-2-ylamino}benzonitrile dihydrochloride;
*(S)*-*N*¹-{4-(3-aminopyrrolidin-1-yl) -5,6,7,8-tetrahydroquinazolin-2-yl}-5-(trifluoromethyl)benzene-1,3-diamine dihydrochloride;
*(S)*-*N*¹-{4-(3-aminopyrrolidin-1-yl)-5,6,7,8-tetrahydroquinazolin-2-yl}-3-(trifluoromethyl)benzene-1,4-diamine dihydrochloride;
*(S)*-3-amino-5-[4-{3-(methylamino)pyrrolidin-1-yl}-5,6,7,8-tetrahydroquinazolin-2-ylamino]benzonitrile dihydrochloride;
(*S*)-*N*¹-[4-{3-(methylamino)pyrrolidin-1-yl}-5,6,7,8-tetrahydroquinazolin-2-yl]-5-(trifluoromethyl)benzene-1,3-diamine dihydrochloride;
*(S)-N*¹-[4-{3-(methylamino)pyrrolidin-1-yl}-5,6,7,8-tetrahydroquinazolin-2-yl]-3-(trifluoromethyl)benzene-1,4-diamine dihydrochloride;
(*S*)-*N*¹-[4-{3-(ethylamino)pyrrolidin-1-yl}-5,6,7,8-tetrahydroquinazolin-2-yl]-5-(trifluoromethyl)benzene-1,3-diamine dihydrochloride;
*(S)*-*N*¹-[4-{3-(ethylamino)pyrrolidin-1-yl}-5,6,7,8-tetrahydroquinazolin-2-yl]-3-(trifluoromethyl)benzene-1,4-diamine dihydrochloride;
*(S)*-4-chloro-*N*¹-[4-{3-(ethylamino)pyrrolidin-1-yl}-5,6,7,8-tetrahydroquinazolin-2-yl]benzene-1,3-diamine dihydrochloride;
*(S)*-3-amino-5-[4-{3-(ethylamino)pyrrolidin-1-yl}-5,6,7,8-tetrahydroquinazolin-2-ylamino]benzonitrile dihydrochloride;
*(S)*-*N*¹-[4-{3-(propylamino)pyrrolidin-1-yl}-5,6,7,8-tetrahydroquinazolin-2-yl]-5-(trifluoromethyl)benzene-1,3-diamine;
*(R)*-*N*¹-{4-(3-aminopiperidin-1-yl)-5,6,7,8-tetrahydroquinazolin-2-yl}-5-(trifluoromethyl)benzene-1,3-diamine dihydrochloride;
*(R)*-*N*-[1-{2-(3-cyanophenylamino)-5,6,7,8-tetrahydroquinazolin-4-yl}piperidin-3-yl]acetamide;
*(R)*-*N*-[1-{2-(3-cyano-4-methylphenylamino)-5,6,7,8-tetrahydroquinazolin-4-yl}piperidin-3-yl]acetamide;
*(R)*-*N*-[1-{2-(3-cyano-4-fluorophenylamino)-5,6,7,8-tetrahydroquinazolin-4-yl}piperidin-3-yl]acetamide;
*(R)*-*N*-[1-{2-(3-amino-5-cyanophenylamino)-5,6,7,8-tetrahydroquinazolin-4-yl}piperidin-3-yl]acetamide;
*(R)*-*N*-(1-[2-{3-amino-5-(trifluoromethyl)phenylamino}-5,6,7,8-tetrahydroquinazolin-4-yl]piperidin-3-yl)acetamide;
*(R)*-*N*-(1-[2-{4-amino-3-(trifluoromethyl)phenylamino}-5,6,7,8-tetrahydroquinazolin-4-yl]piperidin-3-yl)acetamide;
*(R)*-*N*-(1-[2-{4-fluoro-3-(trifluoromethyl)phenylamino}-5,6,7,8-tetrahydroquinazolin-4-yl]piperidin-3-yl)acetamide;
*(R)*-*N*-[1-{2-(4-amino-3-nitrophenylamino)-5,6,7,8-tetrahydroquinazolin-4-yl}piperidin-3-yl]acetamide;
*(R)*-*N*-[1-{2-(3-amino-4-chlorophenylamino)-5,6,7,8-tetrahydroquinazolin-4-yl}piperidin-3-yl]acetamide;
*(R)*-*N*-[1-{2-(3-amino-5-chlorophenylamino)-5,6,7,8-tetrahydroquinazolin-4-yl}piperidin-3-yl]acetamide;
*(R)*-*N*-(1-[2-{4-amino-3-(trifluoromethyl)phenylamino}-5,6,7,8-tetrahydroquinazolin-4-yl]piperidin-3-yl)acetamide hydrochloride;
*(R)*-*N*-[1-{2-(3-amino-5-cyanophenylamino)-5,6,7,8-tetrahydroquinazolin-4-yl}piperidin-3-yl]acetamide hydrochloride;
*(S)*-1-{2-(3-amino-5-cyanophenylamino)-5,6,7,8-tetrahydroquinazolin-4-yl}-*N*-methylpyrrolidine-3-carboxamide;
*(R)*-1-{2- (3-cyano-4-methylphenylamino)-5,6,7,8-tetrahydroquinazolin-4-yl}-*N*-methylpiperidine-3-carboxamide;
*(R)*-1-{2-(3-cyano-4-fluorophenylamino)-5,6,7,8-tetrahydroquinazolin-4-yl}-*N*-methylpiperidine-3-carboxamide;
*(R)*-1-[2-{3-amino-5-(trifluoromethyl)phenylamino}-5,6,7,8-tetrahydroquinazolin-4-yl]-*N*-methylpiperidine-3-carboxamide;
*(R)*-1-[2-{4-amino-3-(trifluoromethyl)phenylamino}-5,6,7,8-tetrahydroquinazolin-4-yl]-*N*-methylpiperidine-3-carboxamide;
*(R)*-1-[2-{4-fluoro-3-(trifluoromethyl)phenylamino}-5,6,7,8-tetrahydroquinazolin-4-yl]-*N*-methylpiperidine-3-carboxamide;
*(R)*-1-{2-(3-amino-4-chlorophenylamino)-5,6,7,8-tetrahydroquinazolin-4-yl}-*N*-methylpiperidine-3-carboxamide;
*(R)*-1-{2-(3-amino-5-cyanophenylamino)-5,6,7,8-tetrahydroquinazolin-4-yl}-*N*-methylpiperidine-3-carboxamide hydrochloride;
*(R)*-1-{2-(4-amino-3-nitrophenylamino)-5,6,7,8-tetrahydroquinazolin-4-yl}-*N*-methylpiperidine-3-carboxamide hydrochloride;
*(R)*-1-{2-(3-amino-5-chlorophenylamino)-5,6,7,8-tetrahydroquinazolin-4-yl}-*N*-methylpiperidine-3-carboxamide hydrochloride;
(*R*)-1-[2-{3-amino-5-(trifluoromethyl)phenylamino}-5,6,7,8-tetrahydroquinazolin-4-yl]-*N*-methylpiperidine-3-carboxamide hydrochloride;
*(S)*-*N*-{1-(2-[{3-amino-5-(trifluoromethyl)phenyl}amino]quinazolin-4-yl)pyrrolidin-3-yl}acetamide;
*(S)-N-*(1-[2-{(3-amino-4-fluorophenyl)amino}quinazolin-4-yl]pyrrolidin-3-yl)acetamide;
*(S)*-*N*-(1-[2-{(3-amino-4-chlorophenyl)amino}quinazolin-4-yl]pyrrolidin-3-yl)acetamide;
*(S)*-*N*-(1-[2-{(3-amino-5-cyanophenyl)amino}quinazolin-4-yl]pyrrolidin-3-yl)acetamide;
*(S)*-*N*-(1-[2-{(3-amino-4-nitrophenyl)amino}quinazolin-4-yl)pyrrolidin-3-yl)acetamide;
*(S)*-*N*-(1-[2-{(4-amino-3-nitrophenyl)amino}quinazolin-4-yl)pyrrolidin-3-yl)acetamide;
*(S)*-*N*¹-[4-{3-(methylamino)pyrrolidin-1-yl}quinazolin-2-yl]-5-(trifluoromethyl)benzene-1,3-diamine;
*(S)*-4-chloro-*N*¹-[4-{3-(methylamino)pyrrolidin-1-yl}quinazolin-2-yl]benzene-1,3-diamine;
*(S)*-3-amino-5-([4-{3-(methylamino)pyrrolidin-1-yl}quinazolin-2-yl]amino)benzonitrile;
(*S*)-*N*¹-[4-{3-(ethylamino)pyrrolidin-1-yl}quinazolin-2-yl]-5-(trifluoromethyl)benzene-1,3-diamine;
*(S)*-3-amino-5-([4-{3-(ethylamino)pyrrolidin-1-yl}quinazolin-2-yl]amino)benzonitrile;
(*S*)-4-chloro-*N*¹-[4-{3-(ethylamino)pyrrolidin-1-yl}quinazolin-2-yl]benzene-1,3-diamine;
*(R)*-*N*-{1-(2-[{3-amino-5-(trifluoromethyl)phenyl}amino]quinazolin-4-yl)piperidin-3-yl}acetamide;
*(R)*-*N*-(1-[2-{(3-amino-5-cyanophenyl)amino}quinazolin-4-yl]piperidin-3-yl)acetamide;
*(S)*-*N*-{1-(2-[{3-amino-5-(trifluoromethyl)phenyl}amino]-7-methoxyquinazolin-4-yl)pyrrolidin-3-yl}acetamide;
(S)-*N*-(1-[2-{(3-amino-4-chlorophenyl)amino}-7-methoxyquinazolin-4-yl]pyrrolidin-3-yl}acetamide;
*(S)*-*N*-(1-[2-{(3-amino-5-cyanophenyl)amino}-7-methoxyquinazolin-4-yl]pyrrolidin-3-yl)acetamide;
(*S*)-*N*-(1-[2-{(4-amino-3-cyanophenyl)amino}-7-methoxyquinazolin-4-yl]pyrrolidin-3-yl)acetamide;
*(S)-N-*(1-[7-methoxy-2-{(3-methoxy-4-methylphenyl)amino}quinazolin-4-yl]pyrrolidin-3-yl)acetamide;
*(S)*-*N*-(1-[2-{(3-trifluoromethyl-4-methylphenyl)amino}-7-methoxyquinazolin-4-yl]pyrrolidin-3-yl)acetamide;
*(S)*-*N*-(1-[7-methoxy-2-{(2-methylpyridin-5-yl)amino}quinazolin-4-yl]pyrrolidin-3-yl)acetamide;
*(S)*-*N*-(1-[2-{(2-fluoropyridin-4-yl)amino}-7-methoxyquinazolin-4-yl]pyrrolidin-3-yl)acetamide;
*(S)*-*N*-(1-[2-{(5-cyano-6-methylpyridin-2-yl)amino}-7-methoxyquinazolin-4-yl]pyrrolidin-3-yl)acetamide;
*(S)*-*N*-(1-[7-methoxy-2-{(5-methylpyridin-2-yl)amino}quinazolin-4-yl]pyrrolidin-3-yl)acetamide;
*(S)*-*N*-(1-[2-{(3-amino-5-cyanophenyl)amino}pyrido[3,2-*d*]pyrimidin-4-yl]pyrrolidin-3-yl)acetamide;
*(S)*-3-amino-5-[{4-(3-aminopyrrolidin-1-yl)pyrido[3,2-*d*]pyrimidin-2-yl}amino]benzonitrile dihydrochloride;
*(S)*-3-amino-5-([4-{3-(methylamino)pyrrolidin-1-yl}pyrido[3,2-*d*]pyrimidin-2-yl]amino)benzonitrile dihydrochloride;
*(S)*-*N¹*-[4-{3-(methylamino)pyrrolidin-1-yl}pyrido[3,2-*d*]pyrimidin-2-yl]-5-(trifluoromethyl)benzene-1,3-diamine dihydrochloride;
*(S)*-3-amino-5-([4-{3-(propylamino)pyrrolidin-1-y1}pyrido[3,2-*d*]pyrimidin-2-yl]amino)benzonitrile dihydrochloride;
*(S)*-*N¹*-[4-{3-(propylamino)pyrrolidin-1-yl}pyrido[3,2-*d*]pyrimidin-2-yl]-5-(trifluoromethyl)benzene-1,3-diamine dihydrochloride;
*(S)*-*N¹*-[4-{3-(propylamino)pyrrolidin-1-yl}pyrido[3,2-*d*]pyrimidin-2-yl]-3-(trifluoromethyl)benzene-1,4-diamine dihydrochloride;
*(S)*-4-chloro-*N¹*-[4-{3-(propylamino)pyrrolidin-1-yl}pyrido[3,2-*d*]pyrimidin-2-yl]benzene-1,3-diamine dihydrochloride;
*(S)*-2-methyl-5-([4-{3-(propylamino)pyrrolidin-1-yl}pyrido[3,2-*d*]pyrimidin-2-yl]amino)benzonitrile dihydrochloride;
*(S)*-3-amino-5-([4-{3-(pentylamino)pyrrolidin-1-y1}pyrido[3,2-*d*]pyrimidin-2-yl]amino)benzonitrile dihydrochloride;
*(R)*-*N*-(1-[2-{(3-amino-5-cyanophenyl)amino}pyrido[3,2-*d*]pyrimidin-4-yl]piperidin-3-yl)acetamide hydrochloride;
*(R)*-3-amino-5-[{4-(3-aminopiperidin-1-yl)pyrido[3,2-*d*]pyrimidin-2-yl}amino]benzonitrile dihydrochloride;
*(R)*-*N¹*-{4-(3-aminopiperidin-1-yl)pyrido[3,2-*d*]pyrimidin-2-yl]-S-(trifluoromethyl)benzene-1,3-diamine dihydrochloride;
*(S)*-*N*-(1-[2-{(3-amino-5-cyanophenyl)amino}-6,7-dihydro-5*H*-cyclopenta[*d*]pyrimidin-4-yl]pyrrolidin-3-yl)acetamide hydrochloride;
*(S)*-*N*-{1-(2-[{3-amino-5-(trifluoromethyl)phenyl}amino]-6,7-dihydro-5*H*-cyclopenta[*d*]pyrimidin-4-yl)pyrrolidin-3-yl}acetamide hydrochloride;
*(S)*-*N*-(1-[2-{(3-amino-4-chlorophenyl)amino}-6,7-dihydro-5*H*-cyclopenta[*d*]pyrimidin-4-yl]pyrrolidin-3-yl)acetamide hydrochloride;
*(S)*-*N*-(1-[2-{(3-cyano-4-methylphenyl)amino}-6,7-dihydro-5*H*-cyclopenta[*d*]pyrimidin-4-yl]pyrrolidin-3-yl)acetamide hydrochloride;
*(S)*-*N*-{1-(2-[{4-methyl-3-(trifluoromethyl)phenyl}amino]-6,7-dihydro-5*H*-cyclopenta[*d*]pyrimidin-4-yl)pyrrolidin-3-yl}acetamide hydrochloride;
*(S)*-*N*-(1-[2-{(4-amino-3-nitrophenyl)amino}-6,7-dihydro-5*H*-cyclopenta[*d*]pyrimidin-4-yl]pyrrolidin-3-yl)acetamide hydrochloride;
*(S)*-3-amino-5-[{4-(3-aminopyrrolidin-1-yl)-6,7-dihydro-5*H*-cyclopenta[*d*]pyrimidin-2-yl}amino]benzonitrile dihydrochloride;
*(S)-N¹*-{4-(3-aminopyrrolidin-1-yl)-6,7-dihydro-5*H-*cyclopenta[*d*]pyrimidin-2-yl}-5-(trifluoromethyl)benzene-1,3-diamine dihydrochloride;
*(S)*-*N¹*-{4-(3-aminopyrrolidin-1-yl)-6,7-dihydro-5*H-*cyclopenta[*d*]pyrimidin-2-yl}-4-chlorobenzene-1,3-diamine dihydrochloride;
*(S)*-5-[{4-(3-aminopyrrolidin-1-yl)-6,7-dihydro-5*H-*cyclopenta[*d*]pyrimidin-2-yl}amino]-2-methylbenzonitrile dihydrochloride;
*(S)*-4-(3-aminopyrrolidin-1-yl)-*N*-{4-methyl-3-(trifluoromethyl)phenyl}-6,7-dihydro-5*H-*cyclopenta[*d*]pyrimidin-2-amine dihydrochloride;
*(S)*-3-amino-5-([4-{3-(methylamino)pyrrolidin-1-yl}-6,7-dihydro-5*H*-cyclopenta[*d*]pyrimidin-2-yl]amino)benzonitrile dihydrochloride;
*(S)*-*N¹*-[4-{3-(methylamino)pyrrolidin-1-yl}-6,7-dihydro-5*H*-cyclopenta[*d*]pyrimidin-2-yl]-5-(trifluoromethyl)benzene-1,3-diamine dihydrochloride;
*(S)*-4-chloro-*N*¹-[4-{3-(methylamino)pyrrolidin-1-yl}-6,7-dihydro-5*H*-cyclopenta[*d*]pyrimidin-2-yl]benzene-1,3-diamine dihydrochloride;
*(S)*-2-methyl-5-([4-{3-(methylamino)pyrrolidin-1-yl}-6,7-dihydro-5*H*-cyclopenta[*d*]pyrimidin-2-yl]amino)benzonitrile dihydrochloride;
*(S)*-*N*-{4-methyl-3-(trifluoromethyl)phenyl}-4-{3-(methylamino)pyrrolidin-1-yl}-6,7-dihydro-5*H-*cyclopenta[*d*]pyrimidin-2-amine dihydrochloride;
*(S)*-3-amino-5-([4-{3-(propylamino)pyrrolidin-1-yl}-6,7-dihydro-5*H*-cyclopenta[*d*]pyrimidin-2-yl]amino)benzonitrile dihydrochloride;
*(S)*-*N¹*-[4-{3-(propylamino)pyrrolidin-1-yl}-6,7-dihydro-5*H*-cyclopenta[*d*]pyrimidin-2-yl]-5-(trifluoromethyl)benzene-1,3-diamine dihydrochloride;
*(S)*-*N¹*-[4-{3-(propylamino)pyrrolidin-1-yl}-6,7-dihydro-5*H*-cyclopenta[d]pyrimidin-2-yl]-3-(trifluoromethyl)benzene-1,4-diamine dihydrochloride;
*(S)*-4-chloro-*N¹*-[4-{3-(propylamino)pyrrolidin-1-yl}-6,7-dihydro-5*H*-cyclopenta[*d*]pyrimidin-2-yl]benzene-1,3-diamine dihydrochloride;
*(S)*-*N*-{4-methyl-3-(trifluoromethyl)phenyl}-4-{3-(propylamino)pyrrolidin-1-yl}-6,7-dihydro-5*H-*cyclopenta[*d*]pyrimidin-2-amine dihydrochloride;
*(S)*-5-chloro-*N¹*-[4-{3-(propylamino)pyrrolidin-1-yl}-6,7-dihydro-5*H*-cyclopenta[*d*]pyrimidin-2-yl]benzene-1,3-diamine dihydrochloride;
*(R)*-*N*-(1-[2-{(3-amino-5-cyanophenyl)amino}-6,7-dihydro-5*H*-cyclopenta[*d*]pyrimidin-4-yl]piperidin-3-yl)acetamide hydrochloride;
*(R)*-*N*-{1-(2-[{3-amino-5-(trifluoromethyl)phenyl}amino]-6,7-dihydro-5H-cyclopenta[*d*]pyrimidin-4-yl)piperidin-3-yl}acetamide hydrochloride;
*(R)*-*N*-{1-(2-[{4-amino-3-(trifluoromethyl)phenyl}amino]-6,7-dihydro-5*H*-cyclopenta[*d*]pyrimidin-4-yl)piperidin-3-yl}acetamide hydrochloride;
*(R)-N*-(1-[2-{(3-amino-4-chlorophenyl)amino}-6,7-dihydro-5*H*-cyclopenta[*d*]pyrimidin-4-yl]piperidin-3-yl)acetamide hydrochloride;
*(R)*-*N*-(1-[2-{(3-cyano-4-methylphenyl)amino}-6,7-dihydro-5*H*-cyclopenta[*d*]pyrimidin-4-yl]piperidin-3-yl)acetamide hydrochloride;
*(R)-N*-{1-(2-[{4-methyl-3-(trifluoromethyl)phenyl}amino]-6,7-dihydro-5*H*-cyclopenta[*d*]pyrimidin-4-yl)piperidin-3-yl}acetamide hydrochloride;
*(R)-N*-{1-(2-[{4-fluoro-3-(trifluoromethyl)phenyl}amino]-6,7-dihydro-5*H*-cyclopenta[*d*]pyrimidin-4-yl)piperidin-3-yl}acetamide hydrochloride;
*(R)-N*-(1-[2-{(3-amino-5-chlorophenyl)amino}-6,7-dihydro-5*H*-cyclopenta[*d*]pyrimidin-4-yl]piperidin-3-yl}acetamide hydrochloride;
*(R)-N-*(1-[2-{(4-amino-3-nitrophenyl)amino}-6,7-dihydro-5*H*-cyclopenta[*d*]pyrimidin-4-yl]piperidin-3-yl}acetamide hydrochloride;
*(R)*-3-amino-5-[{4-(3-aminopiperidin-1-yl)-6,7-dihydro-5*H-*cyclopenta[*d*]pyrimidin-2-yl}amino]benzonitrile dihydrochloride;
*(R)-N¹-*{4*-*(3-aminopiperidin-1-yl)-6,7-dihydro-5*H-*cyclopenta[*d*]pyrimidin-2-yl}-5-(trifluoromethyl)benzene-1,3-diamine dihydrochloride;
*(R)*-*N¹*-{4-(3-aminopiperidin-1-yl)-6,7-dihydro-5*H-*cyclopenta[*d*]pyrimidin-2-yl}-3-(trifluoromethyl)benzene-1,4-diamine dihydrochloride;
*(R)*-*N¹*-{4-(3-aminopiperidin-1-yl)-6,7-dihydro-5*H-*cyclopenta[*d*]pyrimidin-2-yl}-4-chlorobenzene-1,3-diamine dihydrochloride;
*(R)-N¹-*{4*-*(3-aminopiperidin-1-yl)-6,7-dihydro-5*H-*cyclopenta[*d*]pyrimidin-2-yl}-3-nitrobenzene-1,4-diamine dihydrochloride;
*(R)*-3-amino-5-([4-{3-(methylamino)piperidin-1-yl}-6,7-dihydro-5*H*-cyclopenta[*d*]pyrimidin-2-yl]amino)benzonitrile dihydrochloride;
*(R)-N¹-*[4-{3-(methylamino)piperidin-1-yl}-6,7-dihydro-5H-cyclopenta[*d*]pyrimidin-2-yl]-5-(trifluoromethyl)benzene-1,3-diamine dihydrochloride;
*(R)-N¹-*[4-{3-(methylamino)piperidin-1-yl}-6,7-dihydro-5*H-*cyclopenta[*d*]pyrimidin-2-yl]-3-(trifluoromethyl)benzene-1,4-diamine dihydrochloride;
*(R)*-4-chloro-*N¹*-[4-{3-(methylamino)piperidin-1-yl}-6,7-dihydro-5*H*-cyclopenta[*d*]pyrimidin-2-yl]benzene-1,3-diamine dihydrochloride;
*(R)*-5-chloro-*N¹*-[4-{3-(methylamino)piperidin-1-yl}-6,7-dihydro-5*H*-cyclopenta[*d*]pyrimidin-2-yl]benzene-1,3-diamine dihydrochloride;
*(R)-*2-methyl-5-([4-{3-(methylamino)piperidin-1-yl}-6,7-dihydro-5*H*-cyclopenta[*d*]pyrimidin-2-yl]amino)benzonitrile dihydrochloride;
*(R)-N*-{4-methyl-3-(trifluoromethyl)phenyl}-4-{3-(methylamino)piperidin-1-yl}-6,7-dihydro-5*H-*cyclopenta[*d*]pyrimidin-2-amine dihydrochloride;
*(R)-N¹-*[4-{3-(methylamino)piperidin-1-y1}-6,7-dihydro-5*H-*cyclopenta[*d*]pyrimidin-2-yl]-3-nitrobenzene-1,4-diamine dihydrochloride;
*(R)*-3-amino-5-([4-{3-(methylamino)piperidin-1-yl}-5,6,7,8-tetrahydroquinazolin-2-yl]amino)benzonitrile dihydrochloride;
*(R)*-*N¹*-[4-{3-(methylamino)piperidin-1-yl}-5,6,7,8-tetrahydroquinazolin-2-yl]-5-(trifluoromethyl)benzene-1,3-diamine dihydrochloride;
*(R)*-4-chloro-*N¹*-[4-{3-(methylamino)piperidin-1-yl}-5,6,7,8-tetrahydroquinazolin-2-yl]benzene-1,3-diamine dihydrochloride;
*(R)*-5-chloro-*N¹*-[4-{3-(methylamino)piperidin-1-yl}-5,6,7,8-tetrahydroquinazolin-2-yl]benzene-1,3-diamine dihydrochloride;
*(R)*-2-methyl-5-([4-{3-(methylamino)piperidin-1-yl}-5,6,7,8-tetrahydroquinazolin-2-yl]amino)benzonitrile dihydrochloride;
*(R)-N*-{4-methyl-3-(trifluoromethyl)phenyl}-4-{3-(methylamino)piperidin-1-yl}-5,6,7,8-tetrahydroquinazolin-2-amine dihydrochloride;
*(R)-N¹-*[4-{3-(methylamino)piperidin-1-yl}-5,6,7,8-tetrahydroquinazolin-2-yl]-3-nitrobenzene-1,4-diamine dihydrochloride;
*(R)*-1-[2-{(3-amino-5-cyanophenyl)amino}-6,7-dihydro-5*H-*cyclopenta[*d*]pyrimidin-4-yl]-*N*-methylpiperidine-3-carboxamide hydrochloride;
*(R)*-1-(2-[{3-amino-5-(trifluoromethyl)phenyl}amino]-6,7-dihydro-5*H*-cyclopenta[*d*]pyrimidin-4-yl)-*N*-methylpiperidine-3-carboxamide hydrochloride;
*(R)*-1-(2-[{4-amino-3-(trifluoromethyl)phenyl}amino]-6,7-dihydro-5*H*-cyclopenta[d]pyrimidin-4-yl)-*N*-methylpiperidine-3-carboxamide hydrochloride;
*(R)*-1-[2-{(3-amino-5-chlorophenyl)amino}-6,7-dihydro-5*H-*cyclopenta[*d*]pyrimidin-4-yl]-*N*-methylpiperidine-3-carboxamide hydrochloride;
*(R)*-*N*-methyl-1-(2-[{4-methyl-3-(trifluoromethyl)phenyl}amino]-6,7-dihydro-5*H-*cyclopenta[*d*]pyrimidin-4-yl)piperidine-3-carboxamide hydrochloride; and
*(R)*-1-[2-{(4-amino-3-nitrophenyl)amino}-6,7-dihydro-5*H-*cyclopenta[*d*]pyrimidin-4-yl]-*N*-methylpiperidine-3-carboxamide hydrochloride.

4. A compound of formula 7 as below, or a pharmaceutically acceptable salt thereof: 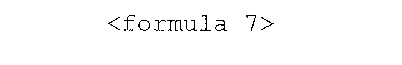 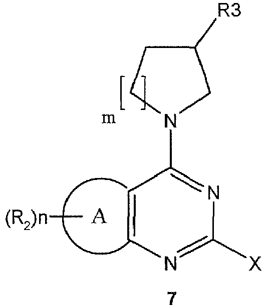 wherein,
R₂, R₃, ring A, m and n are as defined as in claim 1, and
X is halogen.

5. A method for preparing a compound of formula 1 or a pharmaceutically acceptable salt thereof, which comprises,
performing a halogenation of a compound of formula 4 as below to prepare a compound of formula 5 as below;
reacting the compound of formula 5 with a compound of formula 6 as below to prepare a compound of formula 7 as below; and
reacting the compound of formula 7 with R₁-NH₂ to prepare the compound of formula 1: 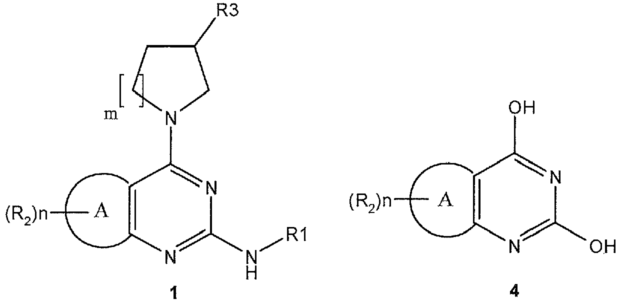 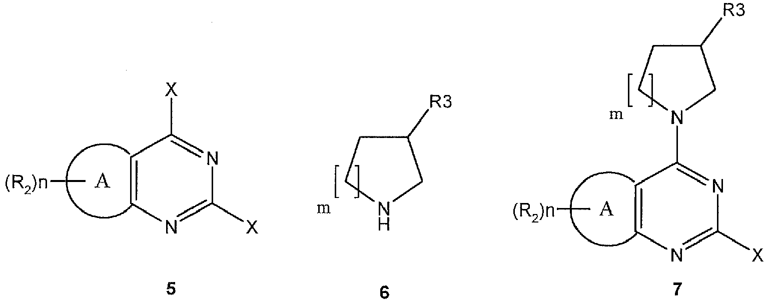 wherein,
R₁, R₂, R₃, ring A, m and n are as defined in claim 1, and
X is halogen.

6. A method for preparing a compound of formula 1b or a pharmaceutically acceptable salt thereof, which comprises reacting a compound of formula 1a as below with an organic acid or an acyl halide: 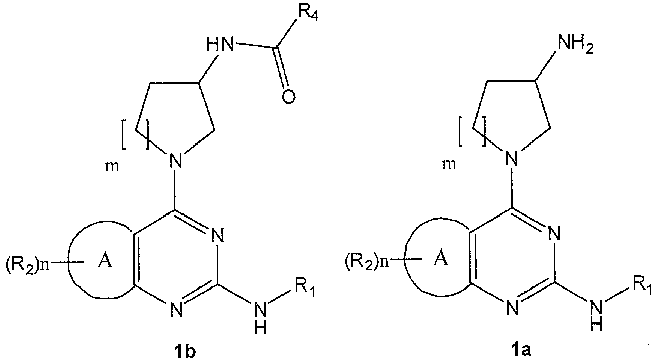 wherein,
R₁, R₂, R₄, ring A, m and n are as defined in claim 1.

7. A method for preparing a compound of the formula 1b or a pharmaceutically acceptable salt thereof, which comprises reacting a compound of formula 7a as below with an organic acid or an acyl halide to prepare a compound of formula 7b; and reacting the compound of formula 7b with R₁-NH₂: 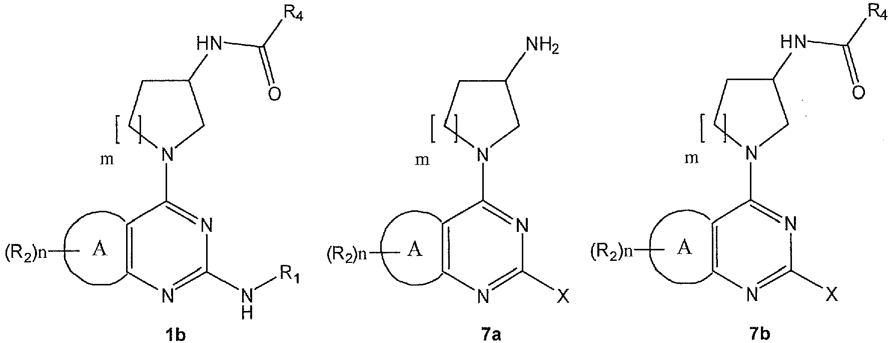 wherein,
R₁, R₂, R₄, ring A, X, m and n are as defined in claim 1.

8. A method for preparing a compound of formula 1c or a pharmaceutically acceptable salt thereof, which comprises performing a reductive amination of a compound of formula 1a as below using an aldehyde or a ketone compound: 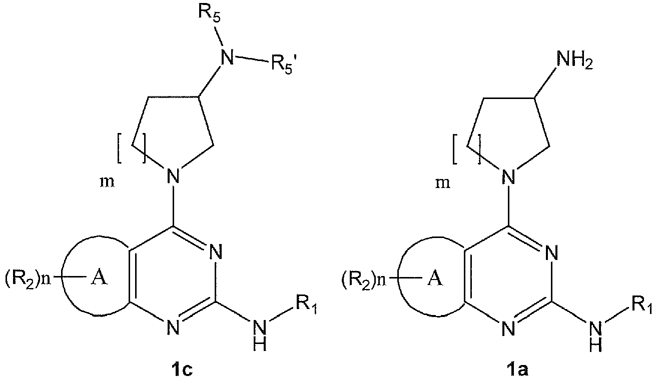 wherein,
R₁, R₂, R₅, R₅', ring A, m and n are as defined in claim 1.

9. A method for preparing a compound of formula 1d or a pharmaceutically acceptable salt thereof, which comprises,
introducing an amine-protecting group into a compound of formula 7a as below to prepare a compound of formula 7c as below;
performing an alkylating of the compound of formula 7c to prepare a compound of formula 7d as below; and
reacting the compound of formula 7d with R₁-NH₂, followed by removing the amine-protecting group: 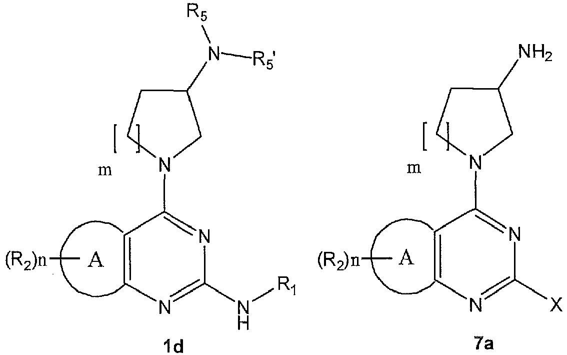 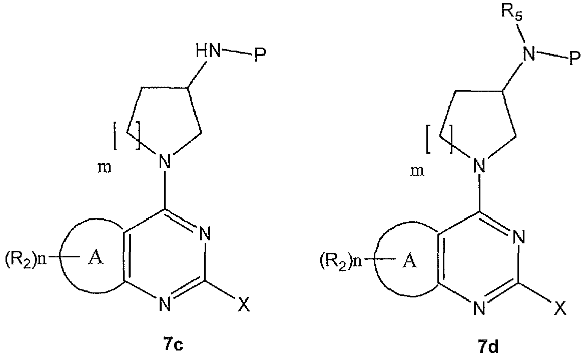 wherein,
R₁, R₂, R₅, ring A, m and n are as defined in claim 1,
R₅' is hydrogen,
X is halogen, and
P is an amine-protecting group.

10. A method for preparing a compound of the formula 1d or a pharmaceutically acceptable salt thereof, which comprises,
performing a reductive amination of a compound of formula 7a as below to prepare a compound of formula 7e as below; and
introducing an amine-protecting group into the compound of formula 7e to prepare a compound of formula 7d as below: 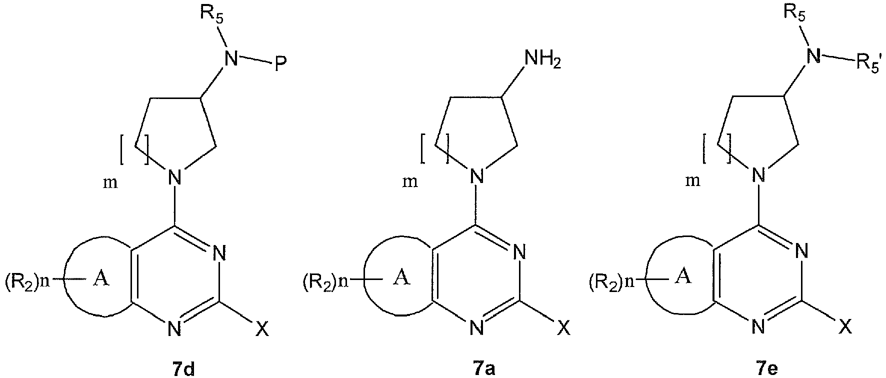 wherein,
R₂, R₅, ring A, m and n are as defined in claim 1,
R₅' is hydrogen,
X is halogen, and
P is an amine protecting group.

11. A method for preparing a compound of formula 1e or a pharmaceutically acceptable salt thereof, which comprises reacting a compound of formula 7f as below with an organic amine to prepare a compound of formula 7g as below; and reacting the compound of formula 7g with R₁-NH₂: 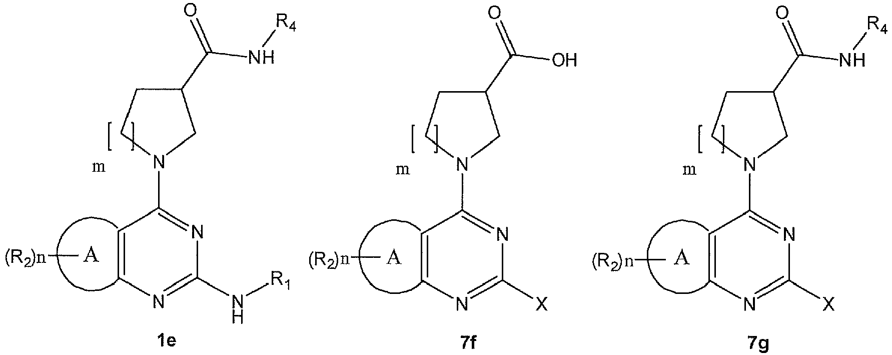 wherein,
R₁, R₂, R₄, ring A, m and n are as defined in claim 1, and
X is halogen.

12. A method for preparing a compound of formula 7 or a pharmaceutically acceptable salt thereof, which comprises reacting a compound of formula 5 as below with a compound of formula 6 as below: 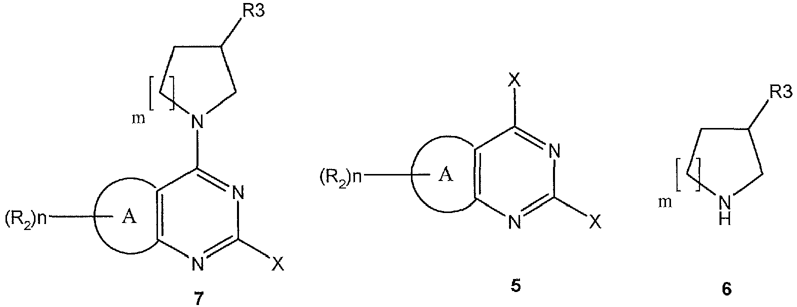 wherein,
R₂, R₃, ring A, m and n are as defined in claim 1, and
X is halogen.

13. A pharmaceutical composition for preventing or treating dysfunction in gastrointestinal motility, which comprises the pharmaceutically effective amount of the compound of formula 1 or the pharmaceutically acceptable salt according to claim 1 and pharmaceutically acceptable carrier thereof.

14. The pharmaceutical composition according to claim 13, wherein the dysfunction in gastrointestinal motility is gastroesophageal reflux disease (GERD), constipation, irritable bowel syndrome (IBS), dyspepsia, post-operative ileus, delayed gastric emptying, gastroparesis, intestinal pseudo-obstruction, drug-induced delayed transit, or diabetic gastric atony.

15. A method for preventing or treating dysfunction in gastrointestinal motility, which comprises administering a composition comprising the compound of formula 1 or the pharmaceutically acceptable salt thereof according to claim 1 as an active ingredient to a mammal including a human, in need of it.

16. Use of a composition comprising the compound of formula 1 or the pharmaceutically acceptable salt thereof according to claim 1 as an active ingredient, for the preparation of a medicament for preventing or treating dysfunction in gastrointestinal motility.
